# EUROPEAN PATENT SPECIFICATION

(11) **EP 1 553 980 B1**
(45) Date of publication and mention of the grant of the patent: **16.11.2011**
(21) Application number: 02728645.9
(22) Date of filing: 01.04.2002
(51) Int. Cl.: A61K 39/395, A61K 38/00, A61K 48/00, C07K 16/00, C12N 5/12, C12P 21/00, C07H 21/04, C12Q 1/68, G01N 33/543

(54) **NUCLEIC ACID AND CORRESPONDING PROTEIN ENTITLED 238P1B2 USEFUL IN TREATMENT AND DETECTION OF CANCER**
NUKLEINSÄURE UND ENTSPRECHENDES PROTEIN MIT DER BEZEICHNUNG 238P1B2, GEEIGNET ZUR BEHANDLUNG UND ZUM NACHWEIS VON KREBS
ACIDE NUCLEIQUE ET PROTEINE CORRESPONDANTE APPELEE 238P1B2 UTILE DANS LE TRAITEMENT ET LA DETECTION DU CANCER

(43) Date of publication of application: 20.07.2005
(73) Proprietor: Agensys, Inc., Santa Monica, CA 90404 (US)
(72) Inventor: Raitano, Arthur B., Los Angeles, CA 90064 (US); Challita-Eid, Pia M., Encino, CA 91436 (US); Faris, Mary, Los Angeles, CA 90077 (US); Hubert, Rene S., Los Angeles, CA 90026 (US); Morrison, Robert Kendall, Santa Monica, CA 90403 (US); Ge, Wangmao, Culver City, CA 90230 (US); Jakobovits, Aya, Beverly Hills, CA 90210 (US)
(74) Representative: Roques, Sarah Elizabeth
(86) International application number: PCT/US2002/010132
(87) International publication number: WO 2003/085081

(56) References cited:
- EP-A- 1 270 724
- WO-A-02/16548
- WO-A2-01/27158
- DATABASE EMBL 1 March 2002 (2002-03-01), XP002377206 retrieved from EBI Database accession no. Q8VH15
- DATABASE NCBI 1 February 2002 (2002-02-01), XP002377207 retrieved from EBI Database accession no. ac090719

## Description

### FIELD OF THE INVENTION

The invention described herein relates to a gene and its encoded protein, termed 238P1B2, expressed in certain cancers, and to diagnostic and therapeutic methods and compositions useful in the management of cancers that express 238P1B2.

### BACKGROUND OF THE INVENTION

Cancer is the second leading cause of human death next to coronary disease. Worldwide, millions of people die from cancer every year. In the United States alone, as reported by the American Cancer Society, cancer causes the death of well over a half-million people annually, with over 1.2 million new cases diagnosed per year. While deaths from heart disease have been declining significantly, those resulting from cancer generally are on the rise. In the early part of the next century, cancer is predicted to become the leading cause of death.

Worldwide, several cancers stand out as the leading killers. In particular, carcinomas of the lung, prostate, breast, colon, pancreas, and ovary represent the primary causes of cancer death. These and virtually all other carcinomas share a common lethal feature. With very few exceptions, metastatic disease from a carcinoma is fatal. Moreover, even for those cancer patients who initially survive their primary cancers, common experience has shown that their lives are dramatically altered. Many cancer patients experience strong anxieties driven by the awareness of the potential for recurrence or treatment failure. Many cancer patients experience physical debilitations following treatment. Furthermore, many cancer patients experience a recurrence.

Worldwide, prostate cancer is the fourth most prevalent cancer in men. In North America and Northern Europe, it is by far the most common cancer in males and is the second leading cause of cancer death in men. In the United States alone, well over 30,000 men die annually of this disease - second only to lung cancer. Despite the magnitude of these figures, there is still no effective treatment for metastatic prostate cancer. Surgical prostatectomy, radiation therapy, hormone ablation therapy, surgical castration and chemotherapy continue to be the main treatment modalities. Unfortunately, these treatments are ineffective for many and are often associated with undesirable consequences.

On the diagnostic front, the lack of a prostate tumor marker that can accurately detect early-stage, localized tumors remains a significant limitation in the diagnosis and management of this disease. Although the serum prostate specific antigen (PSA) assay has been a very useful tool, however its specificity and general utility is widely regarded as lacking in several important respects.

Progress in identifying additional specific markers for prostate cancer has been improved by the generation of prostate cancer xenografts that can recapitulate different stages of the disease in mice. The LAPC (Los Angeles Prostate Cancer) xenografts are prostate cancer xenografts that have survived passage in severe combined immune deficient (SCID) mice and have exhibited the capacity to mimic the transition from androgen dependence to androgen independence (Klein et al., 1997, Nat. Med. 3:402). More recently identified prostate cancer markers include PCTA-1 (Su et al., 1996, Proc. Natl. Acad. Sci. USA 93: 7252), prostate-specific membrane (PSM) antigen (Pinto et al., Clin Cancer Res 1996 Sep 2 (9): 1445-51), STEAP (Hubert, et al., Proc Natl Acad Sci USA. 1999 Dec 7; 96(25): 14523-8) and prostate stem cell antigen (PSCA) (Reiter et al., 1998, Proc. Natl. Acad. Sci. USA 95: 1735).

While previously identified markers such as PSA, PSM, PCTA and PSCA have facilitated efforts to diagnose and treat prostate cancer, there is need for the identification of additional markers and therapeutic targets for prostate and related cancers in order to further improve diagnosis and therapy.

Renal cell carcinoma (RCC) accounts for approximately 3 percent of adult malignancies. Once adenomas reach a diameter of 2 to 3 cm, malignant potential exists. In the adult, the two principal malignant renal tumors are renal cell adenocarcinoma and transitional cell carcinoma of the renal pelvis or ureter. The incidence of renal cell adenocarcinoma is estimated at more than 29,000 cases in the United States, and more than 11,600 patients died of this disease in 1998. Transitional cell carcinoma is less frequent, with an incidence of approximately 500 cases per year in the United States.

Surgery has been the primary therapy for renal cell adenocarcinoma for many decades. Until recently, metastatic disease has been refractory to any systemic therapy. With recent developments in systemic therapies, particularly immunotherapies, metastatic renal cell carcinoma may be approached aggressively in appropriate patients with a possibility of durable responses. Nevertheless, there is a remaining need for effective therapies for these patients.

Of all new cases of cancer in the United States, bladder cancer represents approximately 5 percent in men (fifth most common neoplasm) and 3 percent in women (eighth most common neoplasm). The incidence is increasing slowly, concurrent with an increasing older population. In 1998, there was an estimated 54,500 cases, including 39,500 in men and 15,000 in women. The age-adjusted incidence in the United States is 32 per 100,000 for men and 8 per 100,000 in women. The historic male/female ratio of 3:1 may be decreasing related to smoking patterns in women. There were an estimated 11,000 deaths from bladder cancer in 1998 (7,800 in men and 3,900 in women). Bladder cancer incidence and mortality strongly increase with age and will be an increasing problem as the population becomes more elderly.

Most bladder cancers recur in the bladder. Bladder cancer is managed with a combination of transurethral resection of the bladder (TUR) and intravesical chemotherapy or immunotherapy. The multifocal and recurrent nature of bladder cancer points out the limitations of TUR. Most muscle-invasive cancers are not cured by TUR alone. Radical cystectomy and urinary diversion is the most effective means to eliminate the cancer but carry an undeniable impact on urinary and sexual function. There continues to be a significant need for treatment modalities that are beneficial for bladder cancer patients.

An estimated 130,200 cases of colorectal cancer occurred in 2000 in the United States, including 93,800 cases of colon cancer and 36,400 of rectal cancer. Colorectal cancers are the third most common cancers in men and women. Incidence rates declined significantly during 1992-1996 (-2.1% per year). Research suggests that these declines have been due to increased screening and polyp removal, preventing progression of polyps to invasive cancers. There were an estimated 56,300 deaths (47,700 from colon cancer, 8,600 from rectal cancer) in 2000, accounting for about 11% of all U.S. cancer deaths.

At present, surgery is the most common form of therapy for colorectal cancer, and for cancers that have not spread, it is frequently curative. Chemotherapy, or chemotherapy plus radiation, is given before or after surgery to most patients whose cancer has deeply perforated the bowel wall or has spread to the lymph nodes. A permanent colostomy (creation of an abdominal opening for elimination of body wastes) is occasionally needed for colon cancer and is infrequently required for rectal cancer. There continues to be a need for effective diagnostic and treatment modalities for colorectal cancer.

There were an estimated 164,100 new cases of lung and bronchial cancer in 2000, accounting for 14% of all U.S. cancer diagnoses. The incidence rate of lung and bronchial cancer is declining significantly in men, from a high of 86.5 per 100,000 in 1984 to 70.0 in 1996. In the 1990s, the rate of increase among women began to slow. In 1996, the incidence rate in women was 42.3 per 100,000.

Lung and bronchial cancer caused an estimated 156,900 deaths in 2000, accounting for 28% of all cancer deaths. During 1992-1996, mortality from lung cancer declined significantly among men (-1.7% per year) while rates for women were still significantly increasing (0.9% per year). Since 1987, more women have died each year of lung cancer than breast cancer, which, for over 40 years, was the major cause of cancer death in women. Decreasing lung cancer incidence and mortality rates most likely resulted from decreased smoking rates over the previous 30 years; however, decreasing smoking patterns among women lag behind those of men. Of concern, although the declines in adult tobacco use have slowed, tobacco use in youth is increasing again.

Treatment options for lung and bronchial cancer are determined by the type and stage of the cancer and include surgery, radiation therapy, and chemotherapy. For many localized cancers, surgery is usually the treatment of choice. Because the disease has usually spread by the time it is discovered, radiation therapy and chemotherapy are often needed in combination with surgery. Chemotherapy alone or combined with radiation is the treatment of choice for small cell lung cancer; on this regimen, a large percentage of patients experience remission, which in some cases is long lasting. There is however, an ongoing need for effective treatment and diagnostic approaches for lung and bronchial cancers.

An estimated 182,800 new invasive cases of breast cancer were expected to occur among women in the United States during 2000. Additionally, about 1,400 new cases of breast cancer were expected to be diagnosed in men in 2000. After increasing about 4% per year in the 1980s, breast cancer incidence rates in women have leveled off in the 1990s to about 110.6 cases per 100,000.

In the U.S. alone, there were an estimated 41,200 deaths (40,800 women, 400 men) in 2000 due to breast cancer. Breast cancer ranks second among cancer deaths in women. According to the most recent data, mortality rates declined significantly during 1992-1996 with the largest decreases in younger women, both white and black. These decreases were probably the result of earlier detection and improved treatment.

Taking into account the medical circumstances and the patient's preferences, treatment of breast cancer may involve lumpectomy (local removal of the tumor) and removal of the lymph nodes under the arm; mastectomy (surgical removal of the breast) and removal of the lymph nodes under the arm; radiation therapy; chemotherapy; or hormone therapy. Often, two or more methods are used in combination. Numerous studies have shown that, for early stage disease, long-term survival rates after lumpectomy plus radiotherapy are similar to survival rates after modified radical mastectomy. Significant advances in reconstruction techniques provide several options for breast reconstruction after mastectomy. Recently, such reconstruction has been done at the same time as the mastectomy.

Local excision of ductal carcinoma *in situ* (DCIS) with adequate amounts of surrounding normal breast tissue may prevent the local recurrence of the DCIS. Radiation to the breast and/or tamoxifen may reduce the chance of DCIS occurring in the remaining breast tissue. This is important because DCIS, if left untreated, may develop into invasive breast cancer. Nevertheless, there are serious side effects or sequelae to these treatments. There is, therefore, a need for efficacious breast cancer treatments.

There were an estimated 23,100 new cases of ovarian cancer in the United States in 2000. It accounts for 4% of all cancers among women and ranks second among gynecologic cancers. During 1992-1996, ovarian cancer incidence rates were significantly declining. Consequent to ovarian cancer, there were an estimated 14,000 deaths in 2000. Ovarian cancer causes more deaths than any other cancer of the female reproductive system.

Surgery, radiation therapy, and chemotherapy are treatment options for ovarian cancer. Surgery usually includes the removal of one or both ovaries, the fallopian tubes (salpingo-oophorectomy), and the uterus (hysterectomy). In some very early tumors, only the involved ovary will be removed, especially in young women who wish to have children. In advanced disease, an attempt is made to remove all intra-abdominal disease to enhance the effect of chemotherapy. There continues to be an important need for effective treatment options for ovarian cancer.

There were an estimated 28,300 new cases of pancreatic cancer in the United States in 2000. Over the past 20 years, rates of pancreatic cancer have declined in men. Rates among women have remained approximately constant but may be beginning to decline. Pancreatic cancer caused an estimated 28,200 deaths in 2000 in the United States. Over the past 20 years, there has been a slight but significant decrease in mortality rates among men (about -0.9% per year) while rates have increased slightly among women.

Surgery, radiation therapy, and chemotherapy are treatment options for pancreatic cancer. These treatment options can extend survival and/or relieve symptoms in many patients but are not likely to produce a cure for most. There is a significant need for additional therapeutic and diagnostic options for pancreatic cancer.

WO01/27158 discloses a number of human olfactory receptor polypeptides and polynucleotides encoding them. WO02/16548 and EP-A-1270724, published 2 January 2003, disclose G protein coupled receptor proteins and polynucleotides encoding them. No link is made between any of these proteins and their expression in tumour tissue.

### SUMMARY OF THE INVENTION

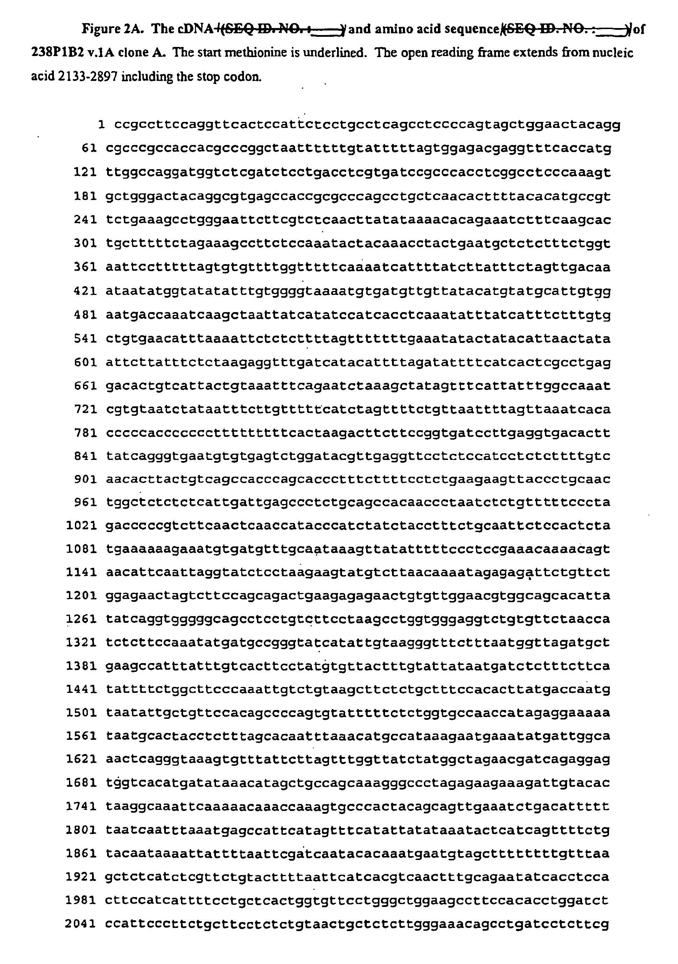

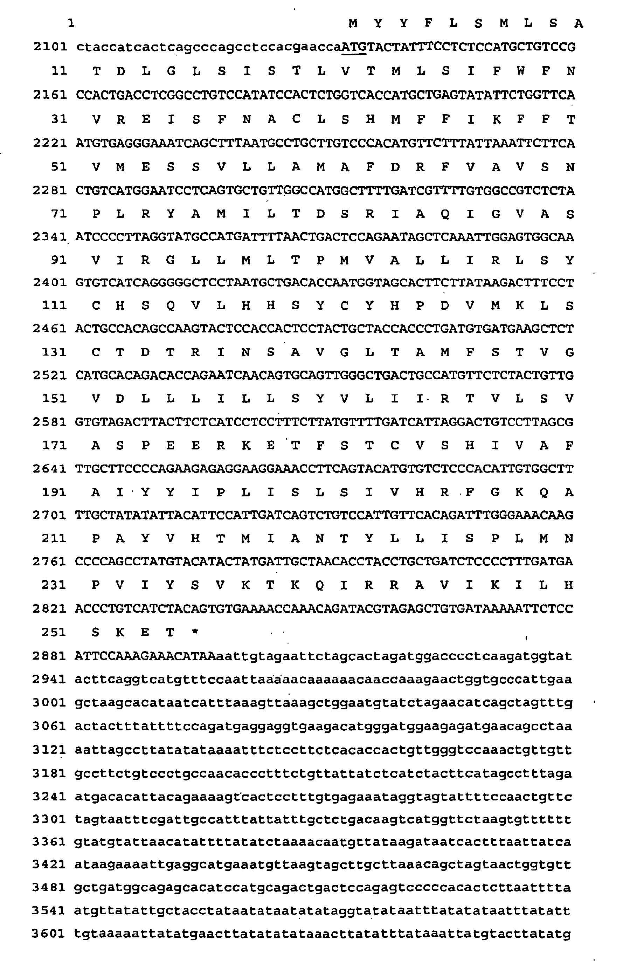

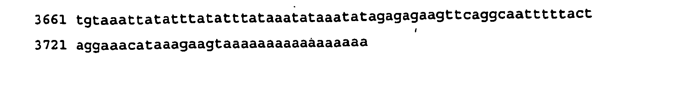

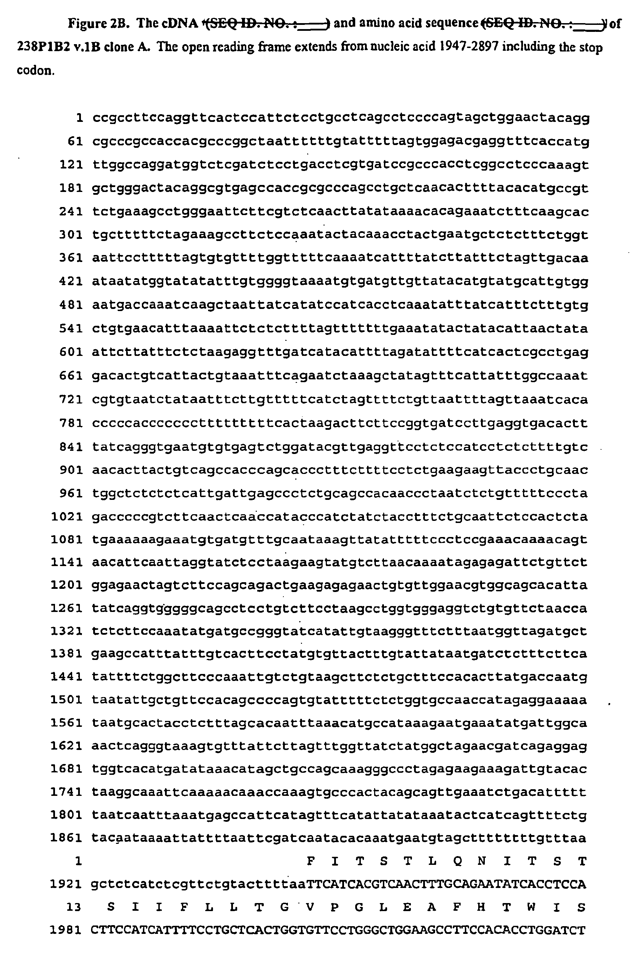

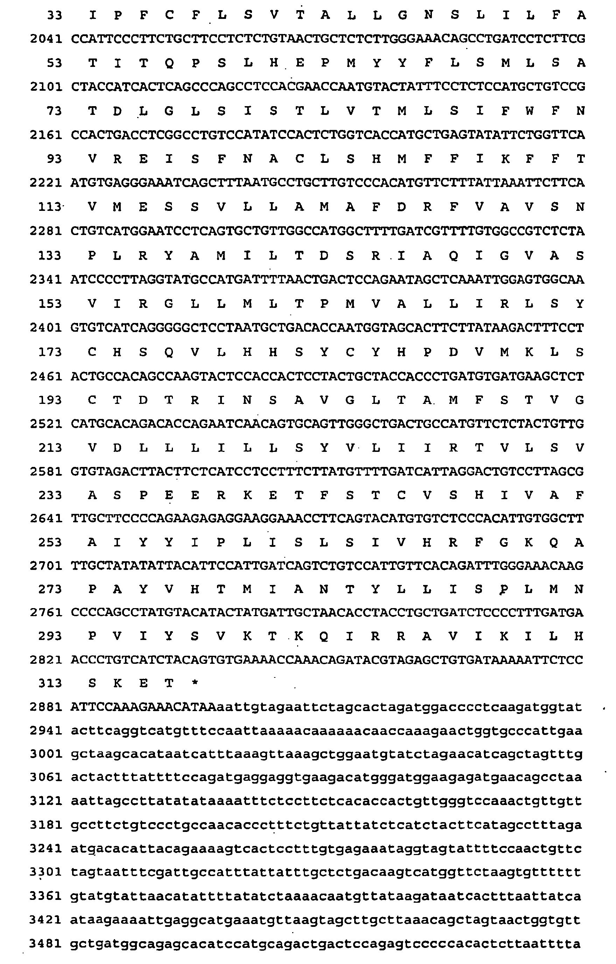

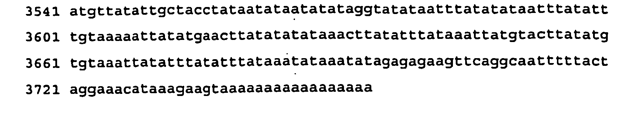

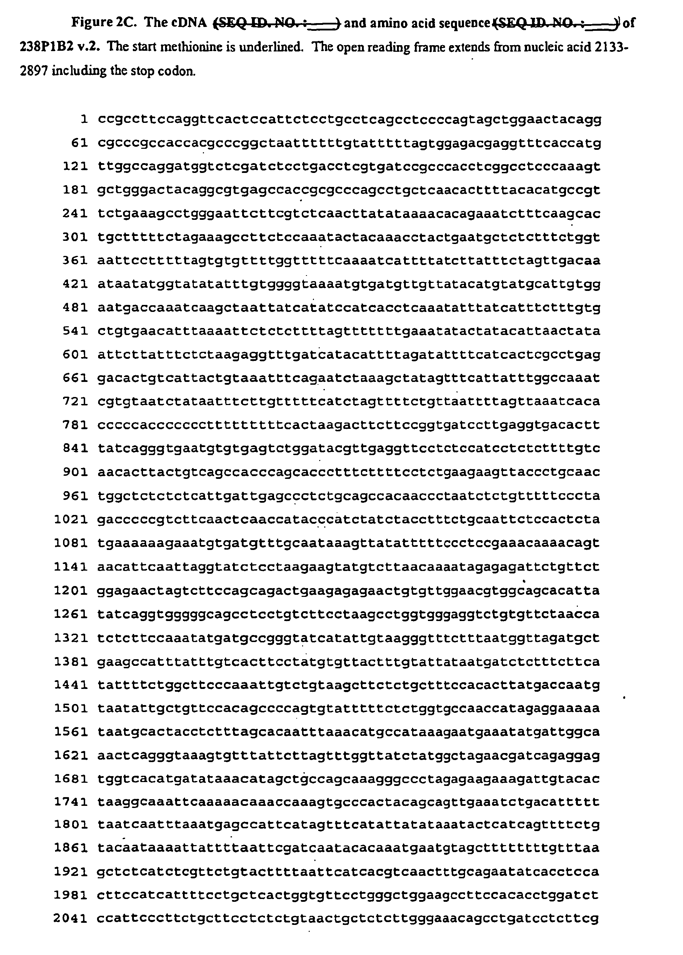

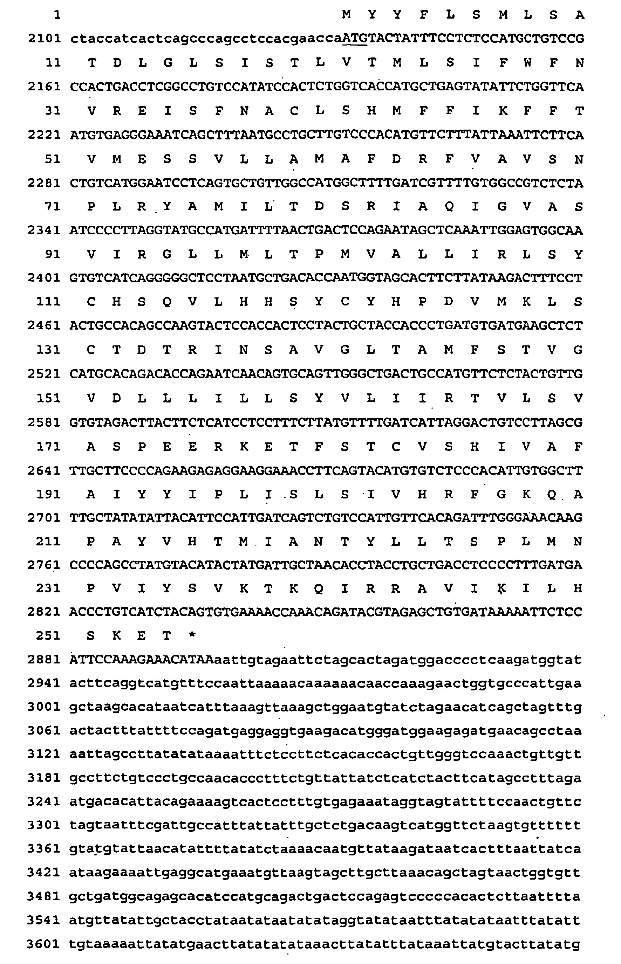

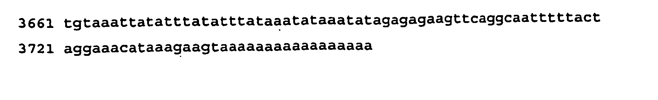

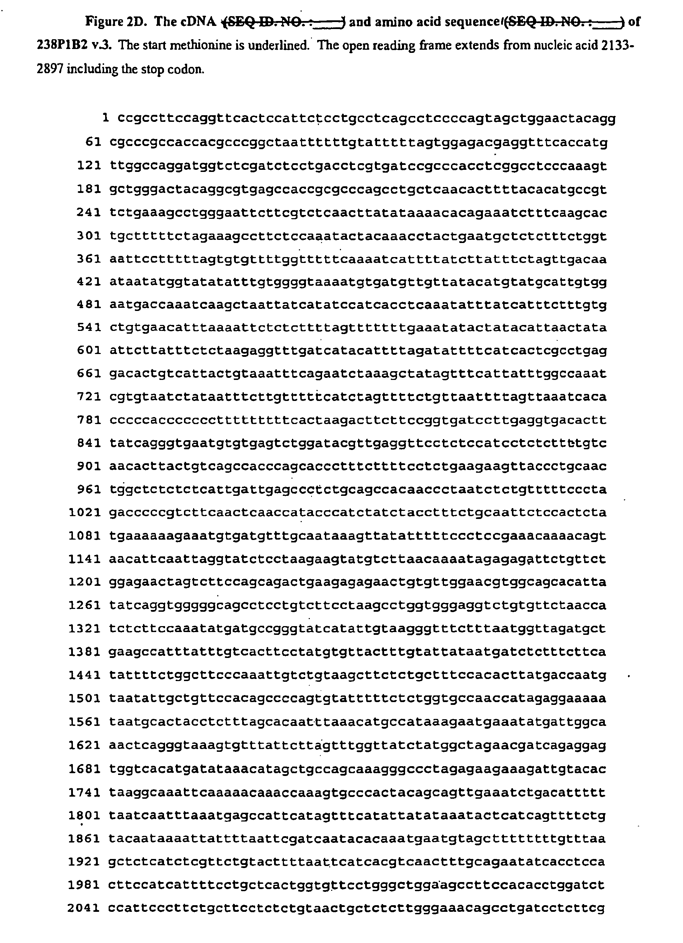

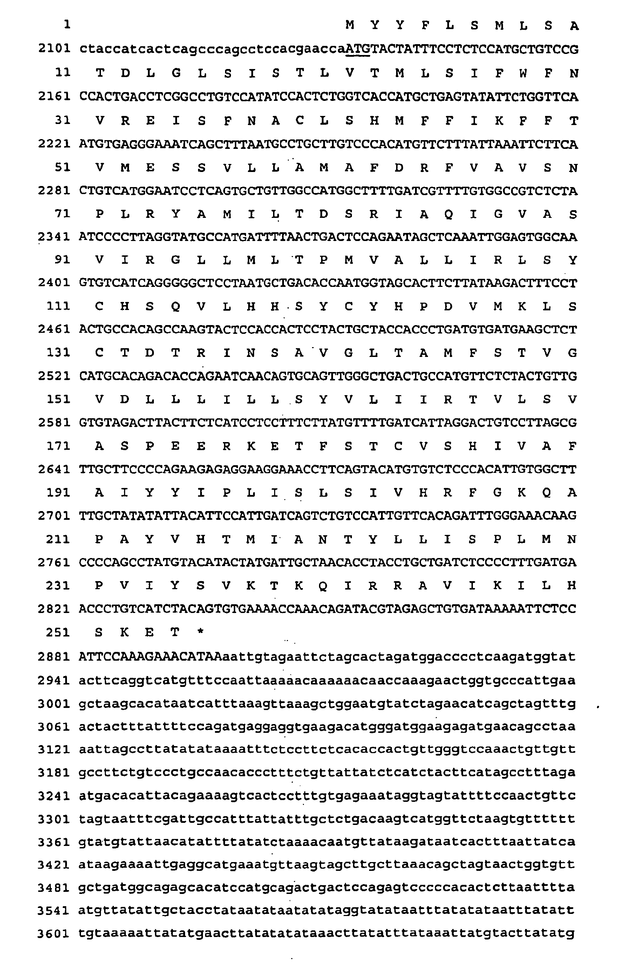

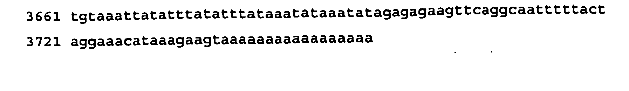

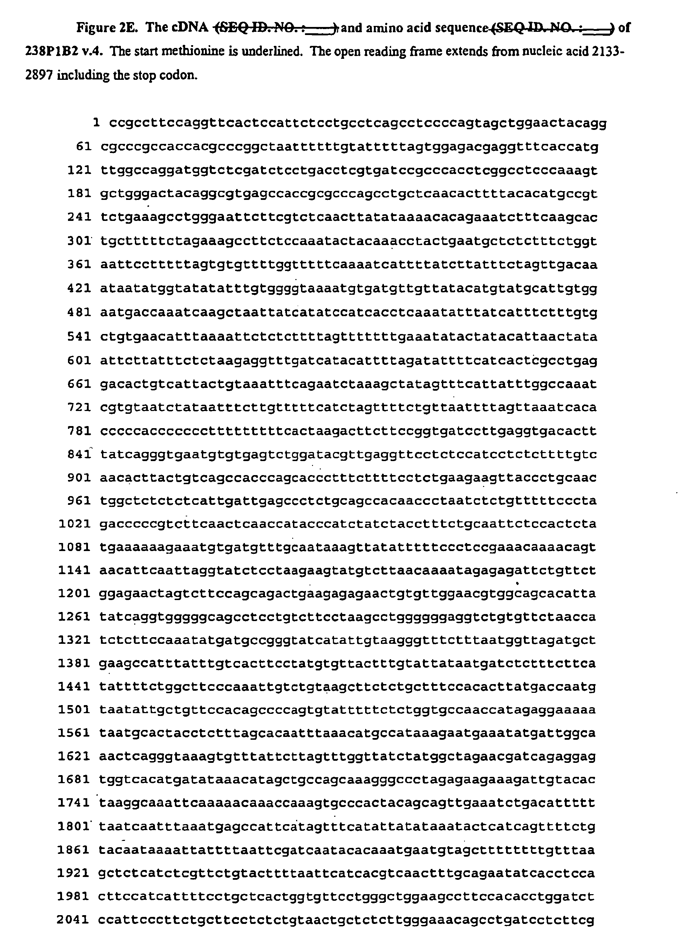

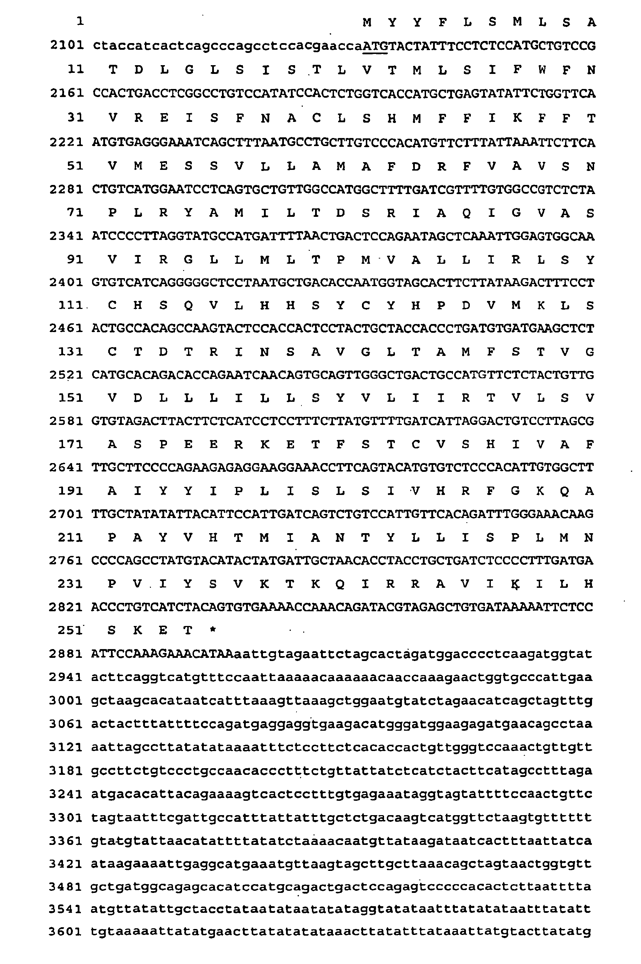

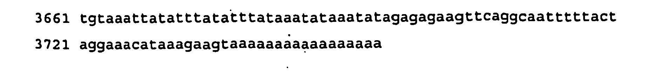

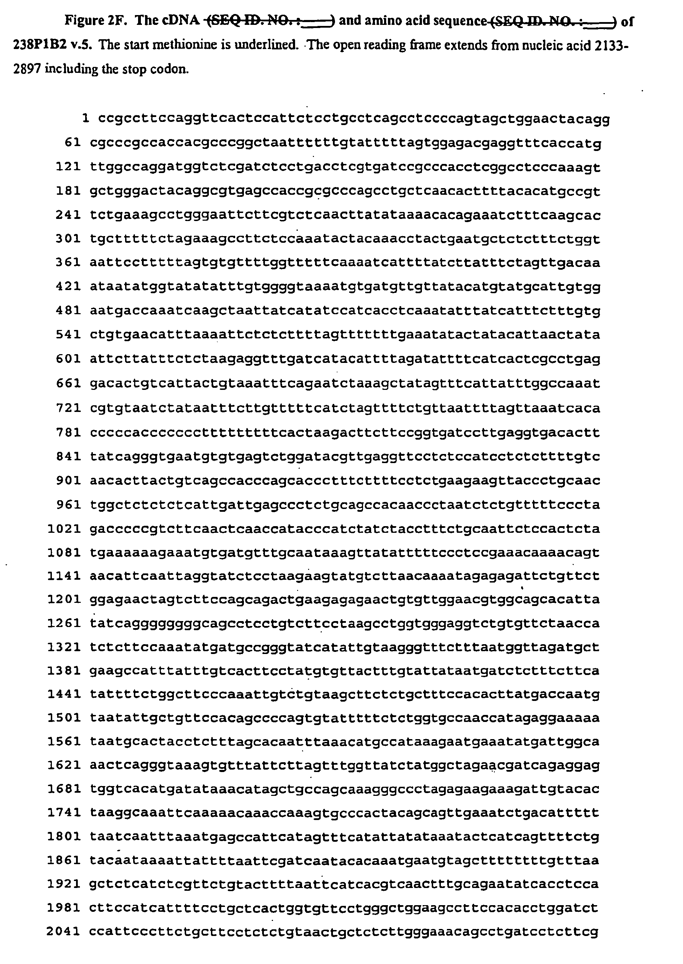

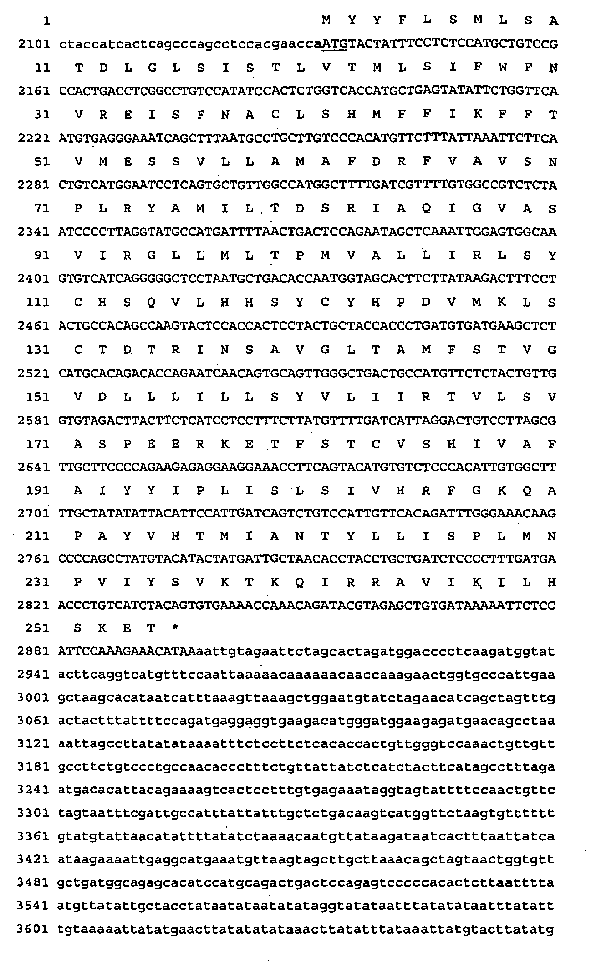

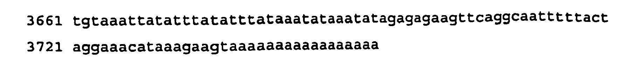

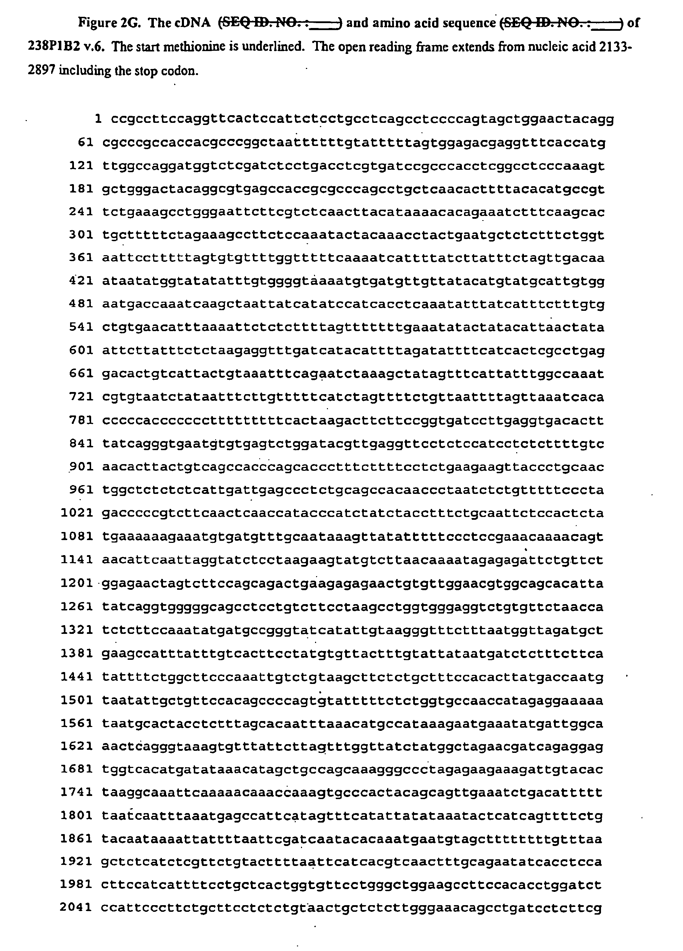

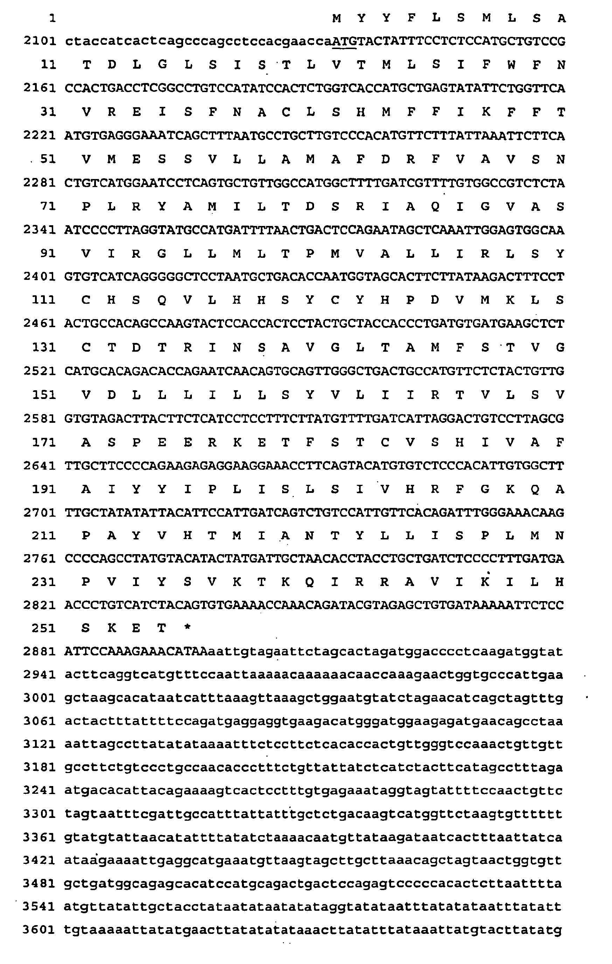

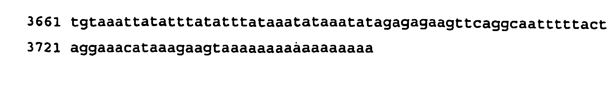

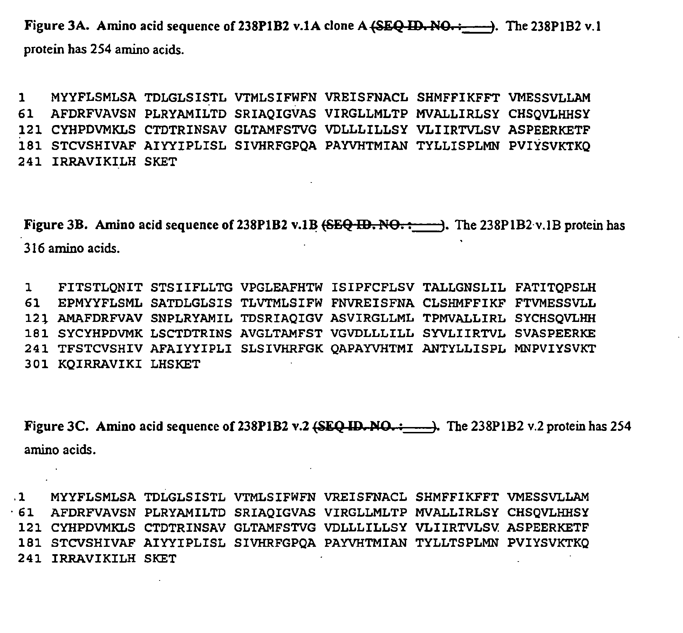

The present invention relates to a gene, designated 238P1B2, that has now been found to be over- expressed in the cancer (s) listed in Table 1. Northern blot expression analysis of 238P1B2 gene expression in normal tissues shows a restricted expression pattern in adult tissues. The nucleotide (Figure 2) and amino acid (Figure 2, and Figure 3) sequences of 238P1B2 are provided. The tissue-related profile of 238P1B2 in normal adult tissues, combined with the over-expression observed in the tumors listed in Table I, shows that 238P1B2 is aberrantly over-expressed in at least some cancers, and thus serves as a useful diagnostic, prophylactic, prognostic, and/or therapeutic target for cancers of the tissue (s) such as those listed in Table I.

In accordance with the present invention, there is provided a method for detecting the presence of prostate cancer in a test sample, the method comprising contacting the sample with a probe that specifically binds to a polynucleotide of Figure 2B (SEQ ID NO: 8872); and detecting binding of the polynucleotide in the sample thereto.

We describe polynucleotides corresponding or complementary to all or part of the 238P1B2 genes, mRNAs, and/or coding sequences, preferably in isolated form, including polynucleotides encoding 238PIB2-related proteins and fragments of 4, 5, 6, 7, 8, 9, 10, 11, 12, 13, 14, 15, 16,17, 18, 19, 20, 21, 22, 23, 24, 25, or more than 25 contiguous amino acids; at least 30, 35, 40, 45, 50, 55, 60, 65, 70, 80, 85, 90, 95, 100 or more than 100 contiguous amino acids of a 238P1B2-related protein, as well as the peptides/proteins themselves; DNA, RNA, DNA/RNA hybrids, and related molecules, polynucleotides or oligonucleotides complementary or having at least a 90% homology to the 238P1B2 genes or mRNA sequences or parts thereof, and polynucleotides or oligonucleotides that hybridize to the 238P1B2 genes, mRNAs, or to 238PlB2-encoding polynucleotides. We also describe means for isolating cDNAs and the genes encoding 238P1B2. Recombinant DNA molecules containing 238P1B2 polynucleotides, cells transformed or transduced with such molecules, and host-vector systems for the expression of 238P1B2 gene products are also described. There may be a proviso that the entire nucleic acid sequence of Figure 2 is not encoded and/or the entire amino acid sequence of Figure 2 is not prepared. The entire nucleic acid sequence of Figure 2 may be encoded and/or the entire amino acid sequence of Figure 2 may be prepared, either of which are in respective human unit dose forms.

The invention provides methods for detecting the presence and status of 238P 1 B2 polynucleotides in various biological samples, as well as methods for identifying cells that express 238P1B2. A typical embodiment of this invention provides methods for monitoring 238P1B2 gene products in a tissue or hematology sample having or suspected of having some form of growth dysregulation such as cancer.

### BRIEF DESCRIPTION OF THE FIGURES

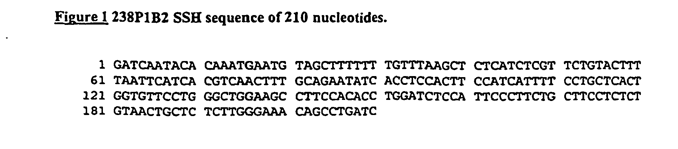

**Figure 1****.** The 238P1B2 SSH sequence of 210 nucleotides.
**Figure 2****.** The cDNA and amino acid sequence of 238P1B2 variant 1A is shown in Figure 2A. The start methionine is underlined. The open reading frame extends from nucleic acids 2133 to 2897 including the stop codon. The nucleic acid and amino acid sequence of 238P1B2 variant 1B is shown in Figure 2B, the codon for the start methionine is underlined. The open reading frame extends from nucleic acid 1947 to 2897 including the stop codon. The nucleic acid and amino acid sequence of 238P1B2 variant 2 is shown in Figure 2C, the codon for the start methionine is underlined. The open reading frame extends from nucleic acid 2133 to 2897 including the stop codon. The nucleic acid and amino acid sequence of 238P1B2 variant 3 is shown in Figure 2D, the codon for the start methionine is underlined. The open reading frame extends from nucleic acid 2133 to 2897 including the stop codon. The nucleic acid and amino acid sequence of 238P1B2 variant 4 is shown in Figure 2E, the codon for the start methionine is underlined. The open reading frame extends from nucleic acid 2133 to 2897 including the stop codon. The nucleic acid and amino acid sequence of 238P1B2 variant 5 is shown in Figure 2F, the codon for the start methionine is underlined. The open reading frame extends from nucleic acid 2133 to 2897 including the stop codon. The nucleic acid and amino acid sequence of 238P1B2 variant 6 is shown in Figure 2G, the codon for the start methionine is underlined. The open reading frame extends from nucleic acid 2133 to 2897 including the stop codon.
**Figure 3****.** Amino acid sequence of 238P1B2 variant 1A is shown in Figure 3A; it has 254 amino acids. The amino acid sequence of 238P1B2 variant 1B is shown in Figure 3B; it has 316 amino acids. The amino acid sequence of 238P1B2 variant 2 is shown in Figure 3C; it has 254 amino acids.

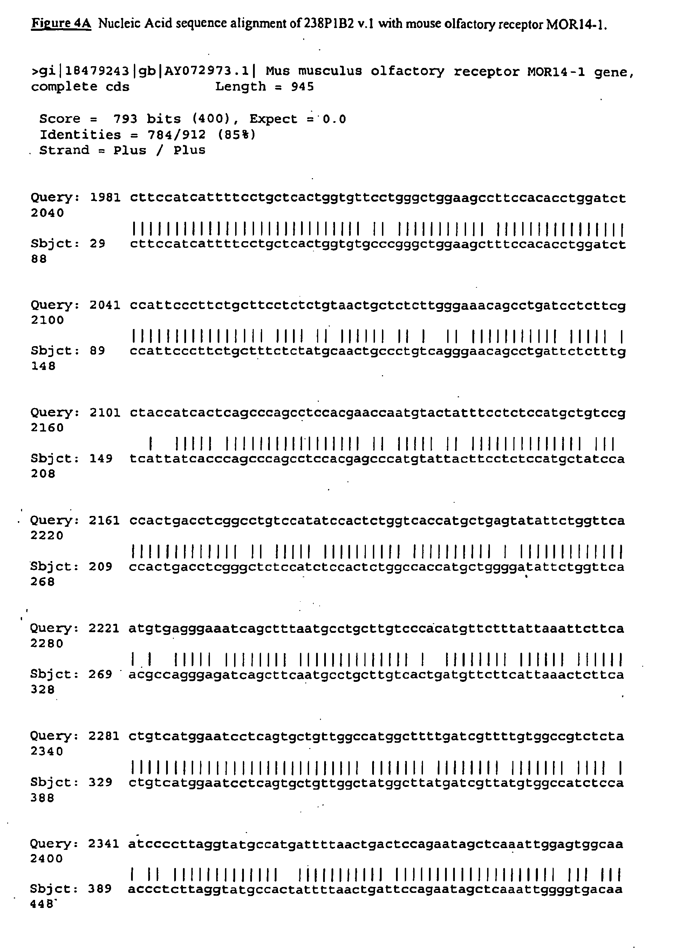

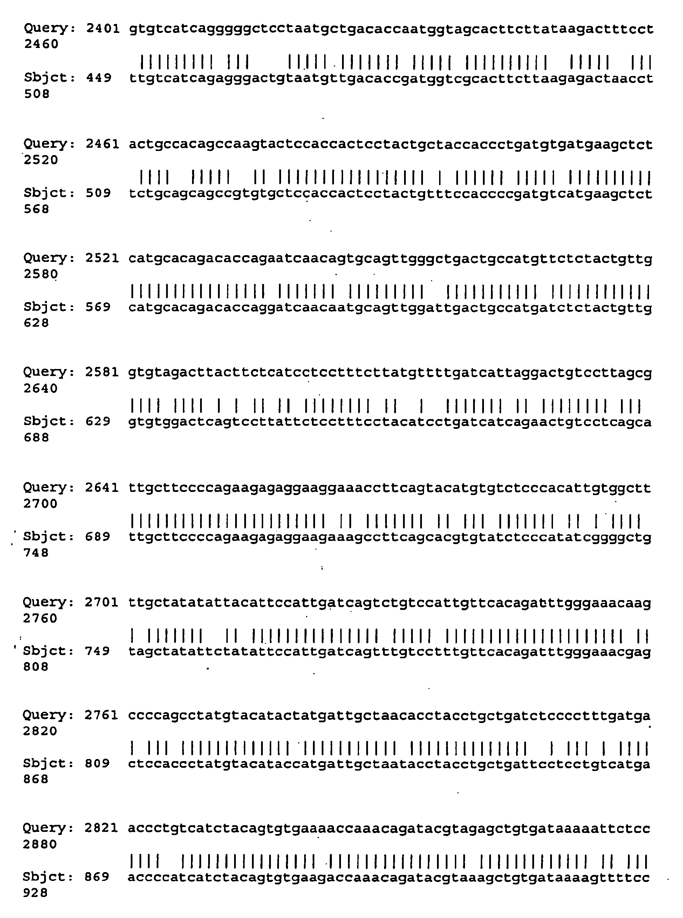

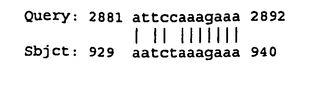

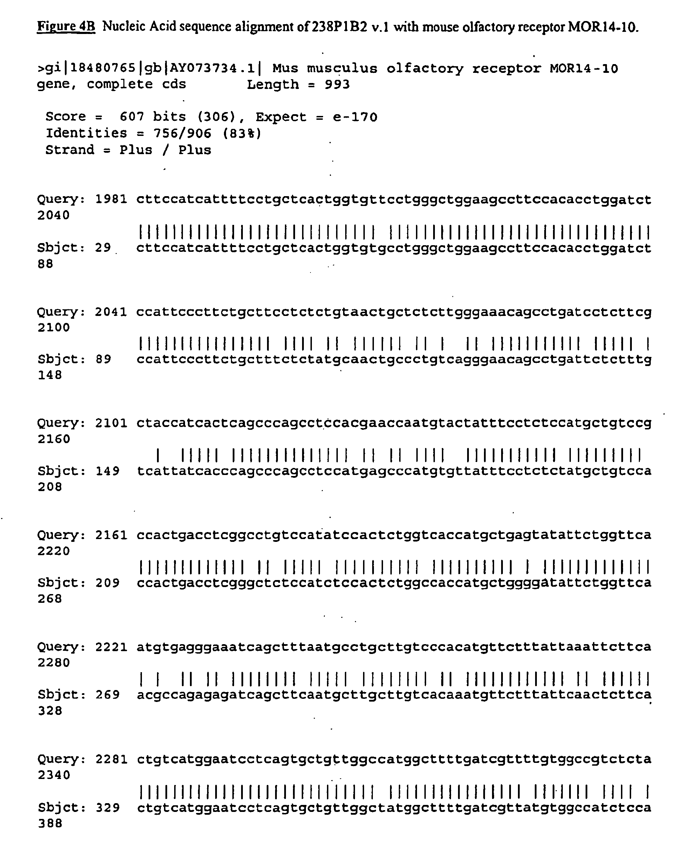

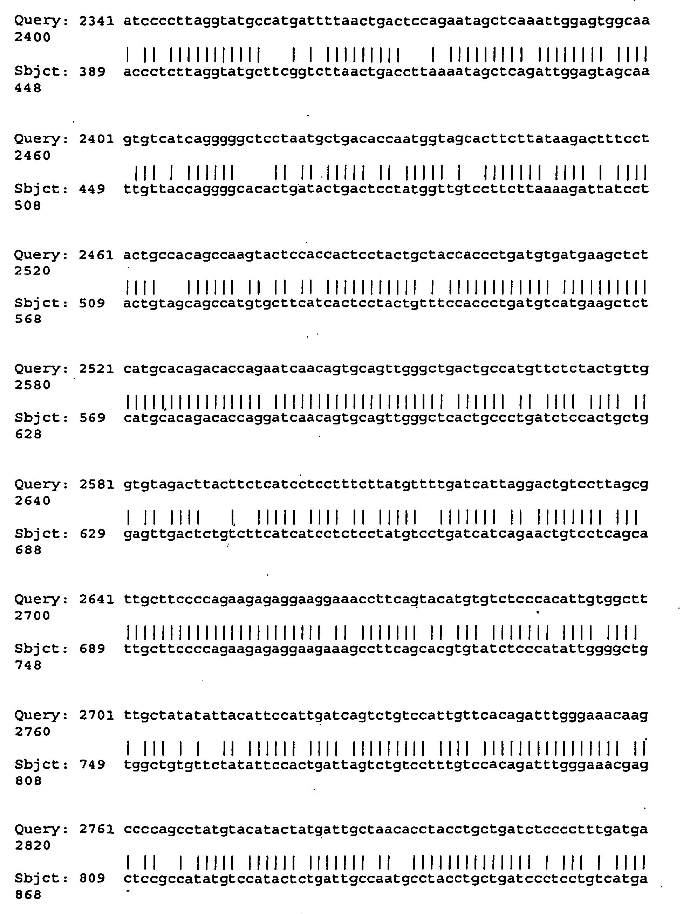

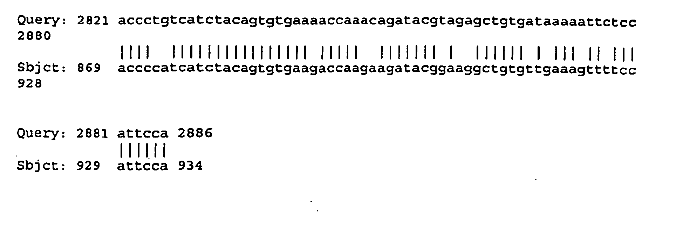

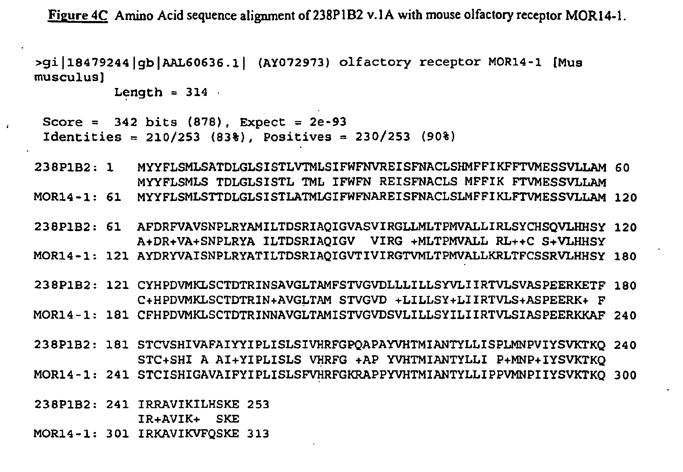

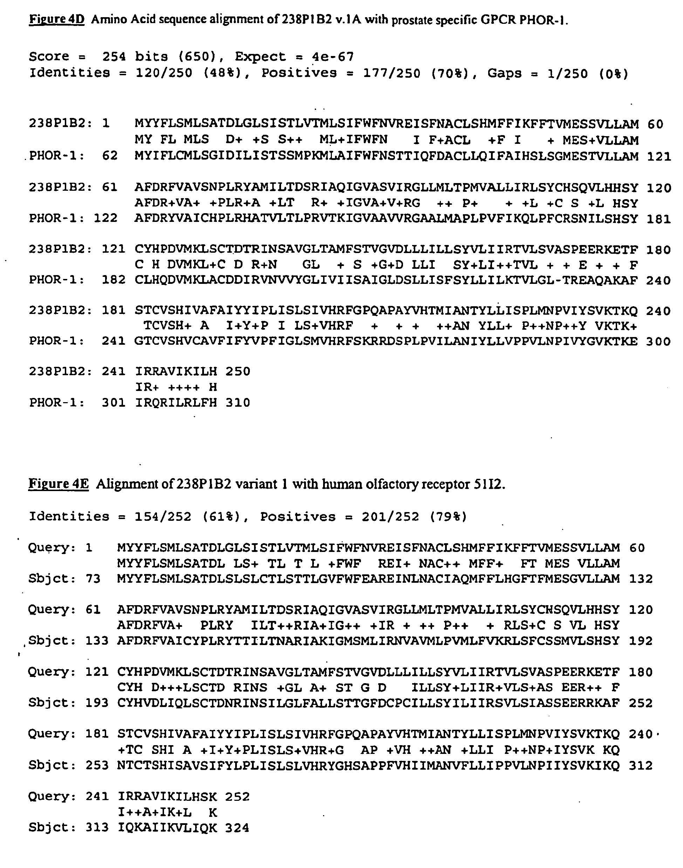

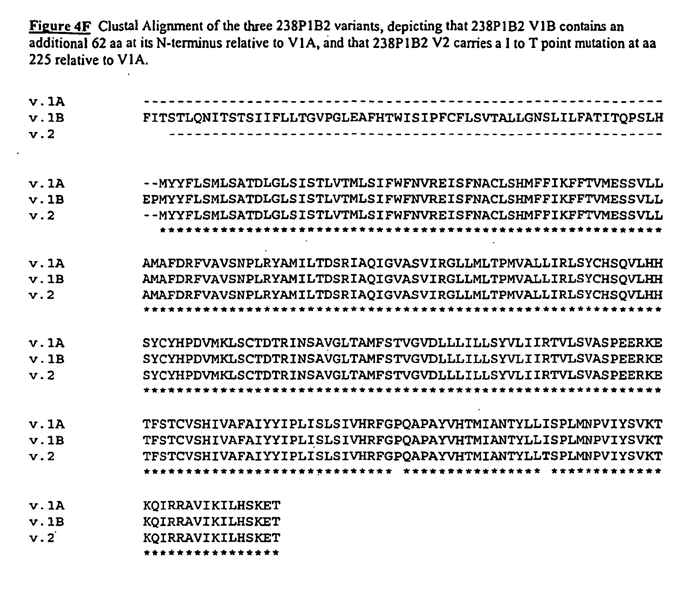

**Figure 4****.** A. Nucleic Acid sequence alignment of 238P1B2 variant 1 with mouse olfactory receptor MOR14-1. B. Nucleic Acid sequence alignment of 238P1B2 variant 1 with mouse olfactory receptor MOR14-10. C. Amino Acid sequence alignment of 238P1B2 v.1A with mouse olfactory receptor MOR14-1. D. Amino Acid sequence alignment of 238P1B2 v.1A with prostate specific OPCRPHOR-1. E. Amino acid sequence alignment of 238P1B2 variant 1 with human olfactory receptor 5112. F. Amino Acid sequence Clustal Alignment of the three 238P1B2 variants, depicting that 238P1B2 V1B contains an additional 62 aa at its N-terminus relative to V1A, and that 238P1B2 V2 carries a I to T point mutation at aa 225 relative to VIA.

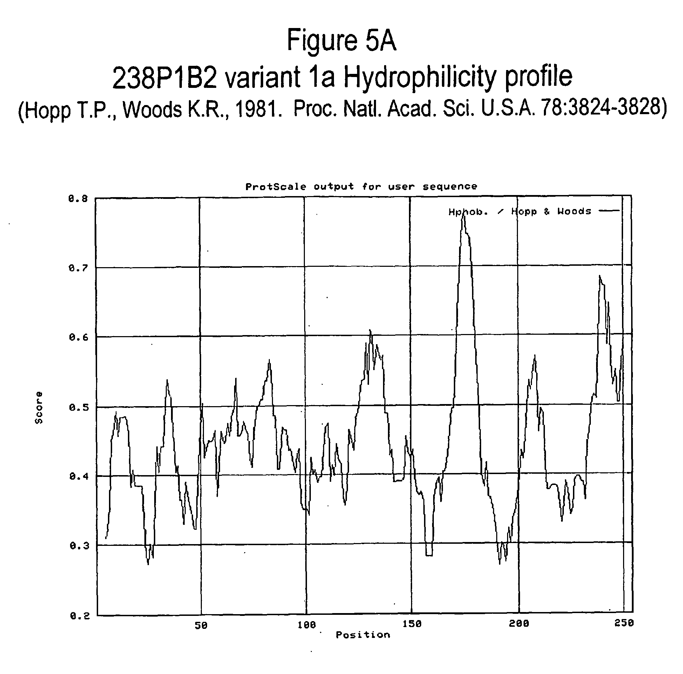

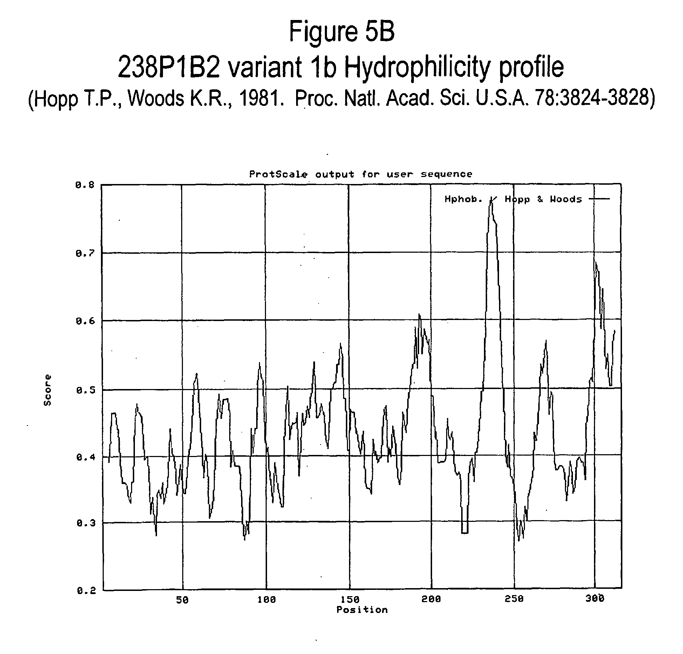

**Figure 5**. Hydrophilicity amino acid profile of A) 238P1B2 and B) 238P1B2 var1A determined by computer algorithm sequence analysis using the method ofHopp and Woods (Hopp T.P., Woods K.R, 1981. Proc. Natl. Acad. Sci. U.S.A. 78:3824-3828) accessed on the Protscale website (www.expasy.ch/cgibin/protscale.pl) through the ExPasy molecular biology server.

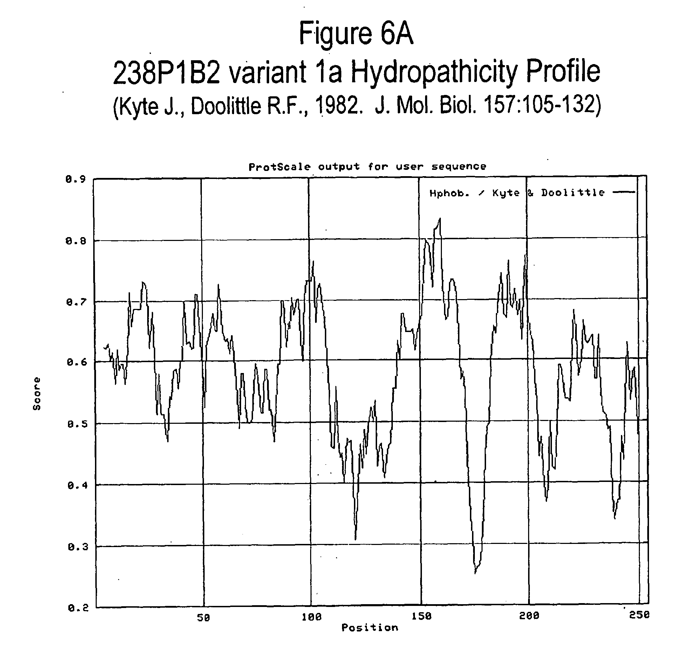

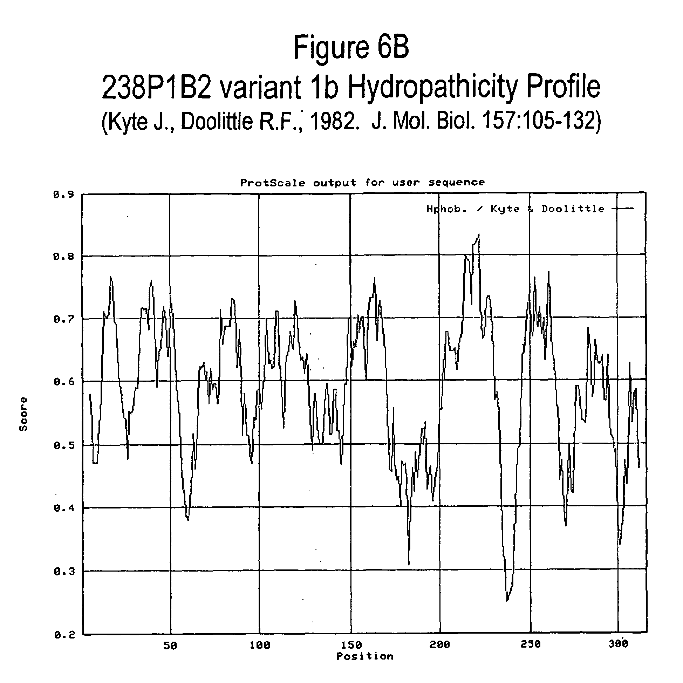

**Figure 6****.** Hydropathicity amino acid profile of A) 238P1B2 and B) 238P1B2 var1A determined by computer algorithm sequence analysis using the method of Kyte and Doolittle (Kyte J., Doolittle R.F., 1982. J. Mol. Biol. 157:105-132) accessed on the ProtScale website (www.expasy.ch/cgi-bin/protscale.pl) through the ExPasy molecular biology server.

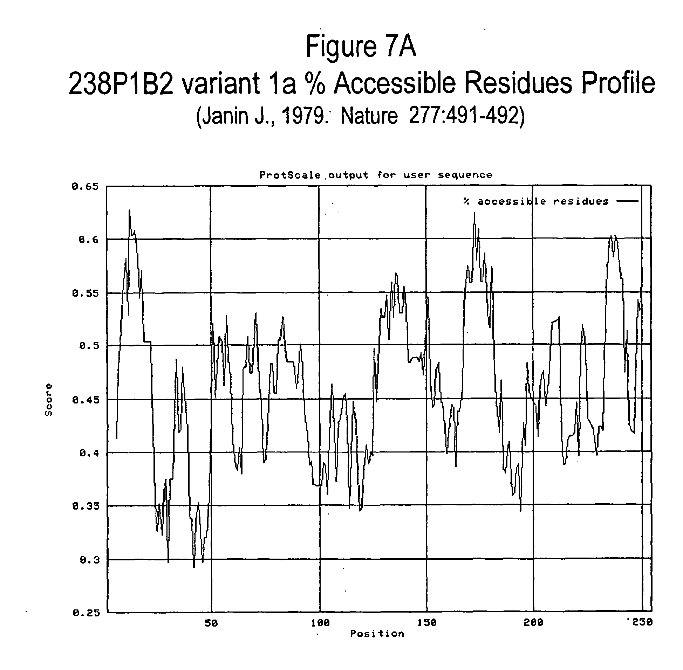

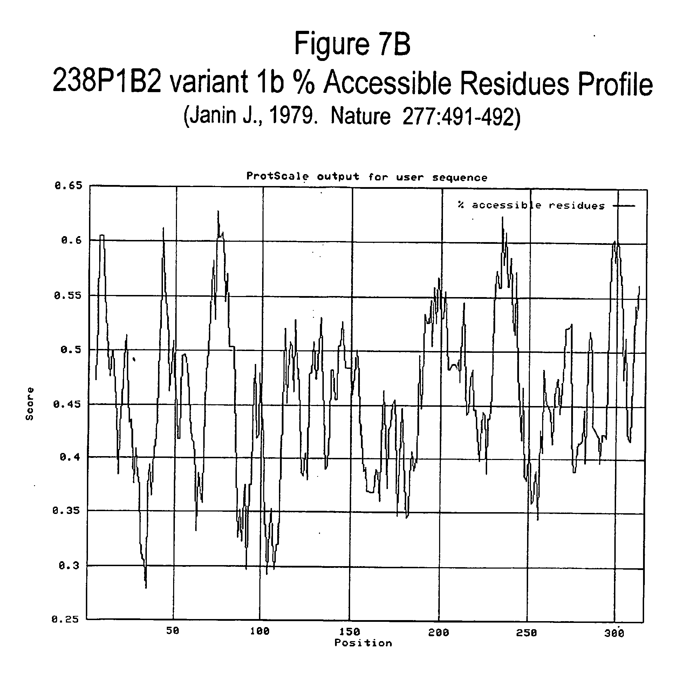

**Figure 7****.** Percent accessible residues amino acid profile of A) 238P1B2 and B) 238P1B2 var1A determined by computer algorithm sequence analysis using the method of Janin (Janin J., 1979 Nature 277:491-492) accessed on the ProtScale website (www.expasy.ch/cgi-bin/protscale.pl) through the ExPasy molecular biology server.

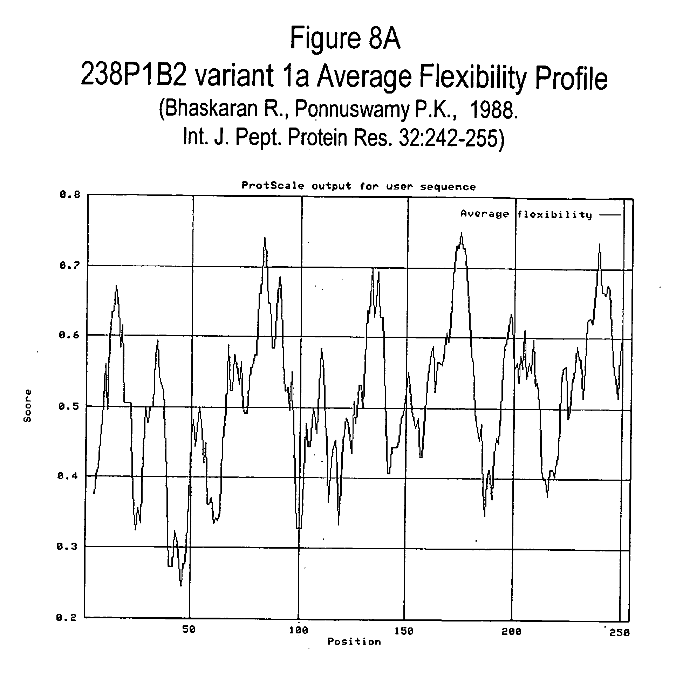

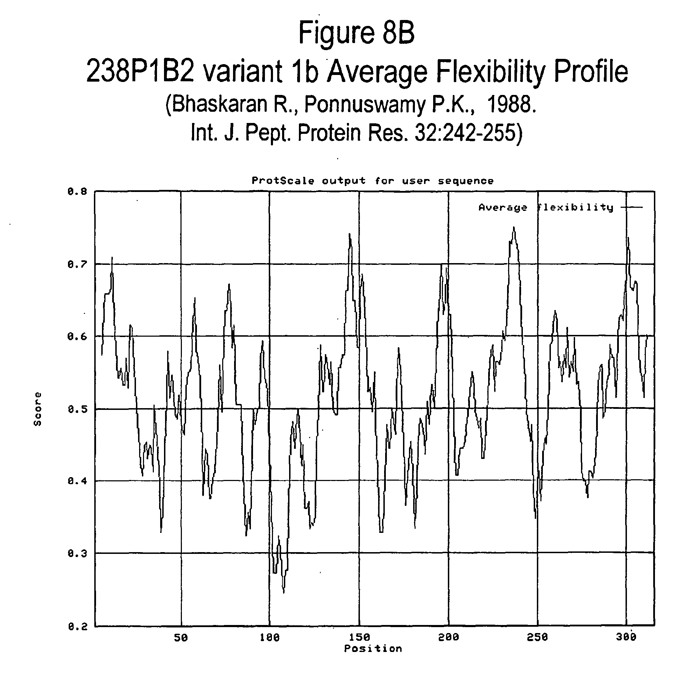

**Figure 8****.** Average flexibility amino acid profile of A) 238P1B2 and B) 238P1B2 var1A determined by computer algorithm sequence analysis using the method of Bhaskaran and Ponnuswamy (Bhaskaran R., and Ponnuswamy P.K., 1988. Int. J. Pept. Protein Res. 32:242-255) accessed on the ProtScale website (www.expasy.ch/cgi-bin/protscale.pl) through the ExPasy molecular biology server.

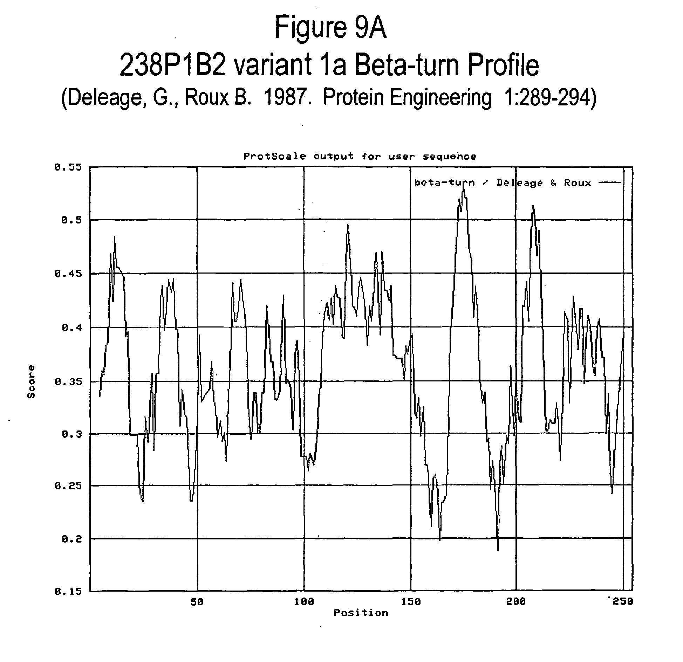

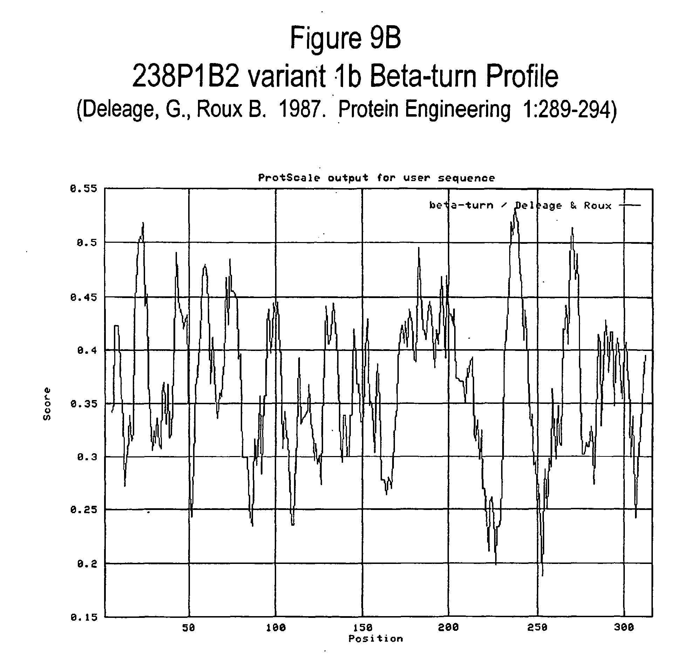

**Figure 9****.** Beta-turn amino acid profile of A) 238P1B2 and B) 238P1B2 var1A determined by computer algorithm sequence analysis using the method of Deleage and Roux (Deleage, G., Roux B. 1987 Protein Engineering 1:289-294) accessed on the ProtScale website (www.expasy.ch/cgi-bin/protscale.pl) through the ExPasy molecular biology server.

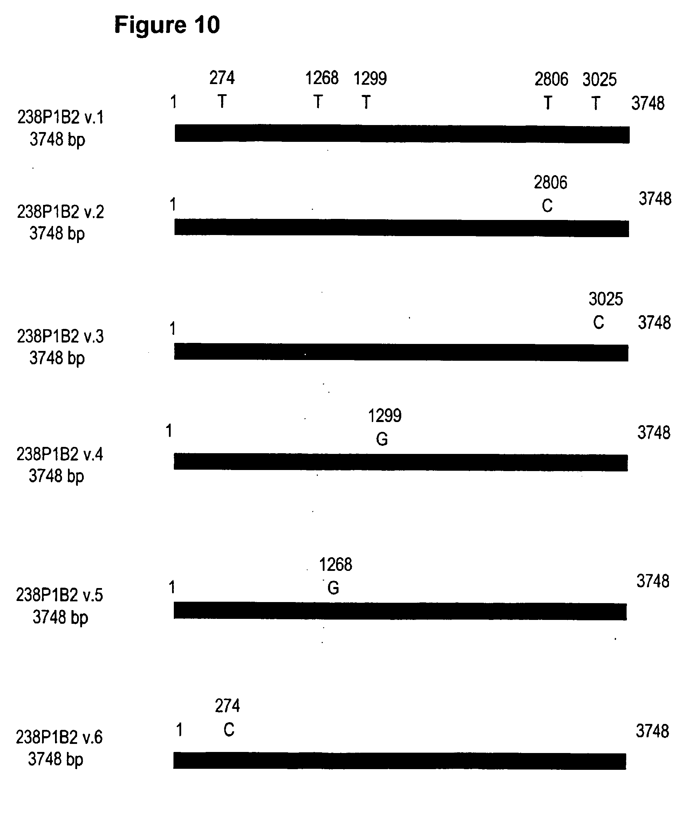

**Figure 10****.** Schematic display of nucleotide variants of 238P1B2. Variant 238P1B2 v.2 through 238P1B2 v.6 are variants with single nucleotide variations. Black box shows the same sequence as 238P1B2 v.1. Numbers correspond to those of 238P1B2 v.1. SNPs are indicated above the box.

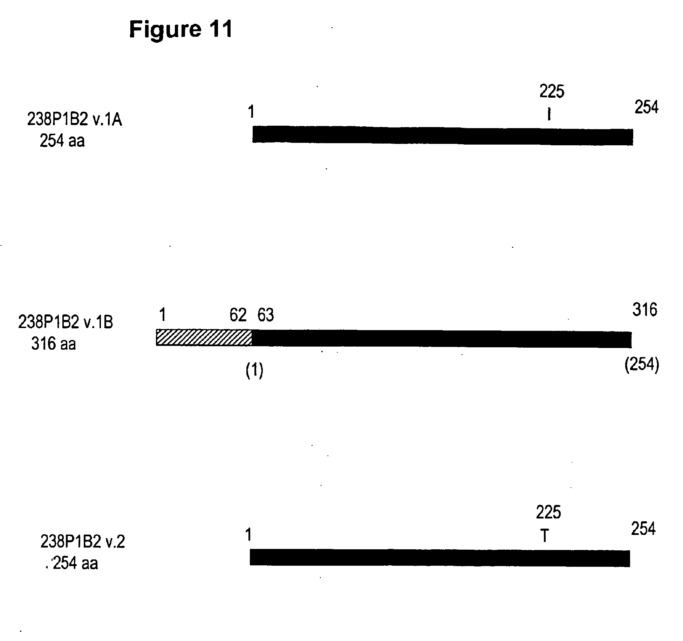

**Figure 11****.** Schematic display of protein variants of 238P1B2. Nucleotide variant 238P1B2 v.1 in Figure 10 codes for protein variants 238P1B2 v.lA and v.1B. Nucleotide variant 238P1B2 v.2 in Figure 10 codes for protein variant 238P1B2 v.2. Black box shows the same sequence as 238P1B2 v.1. Numbers in "()" underneath the box correspond to those of 238P1B2 v.1A. Single amino acid differences are indicated above the box.

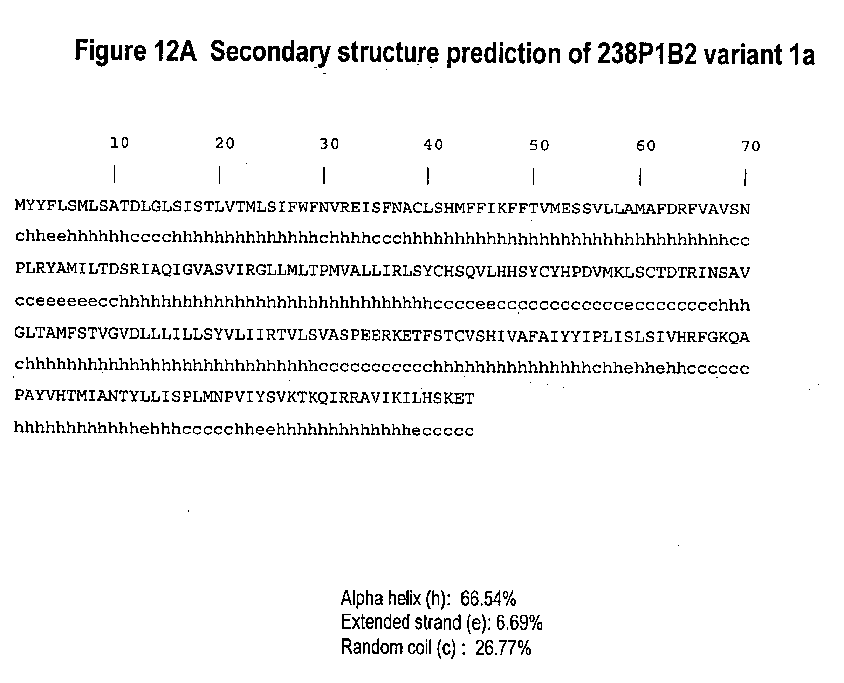

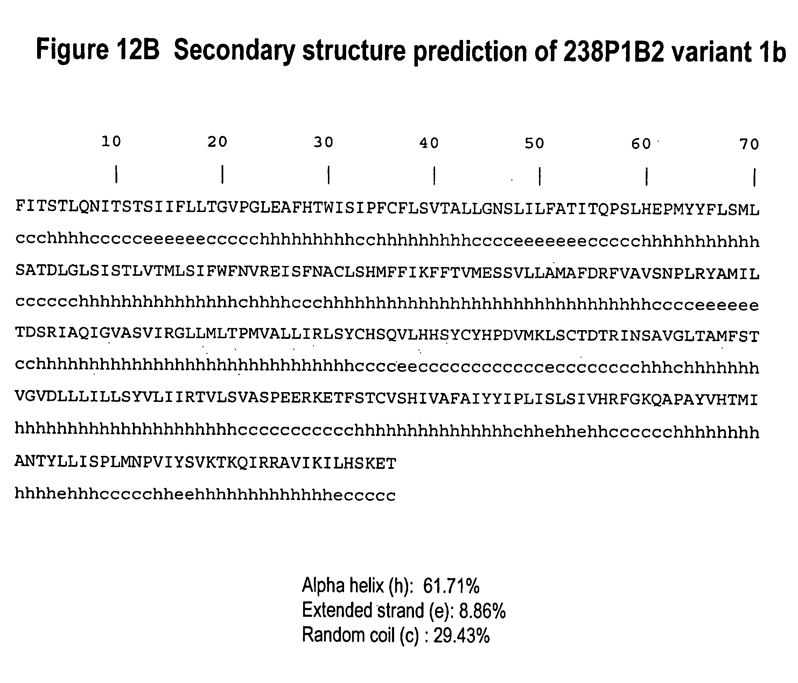

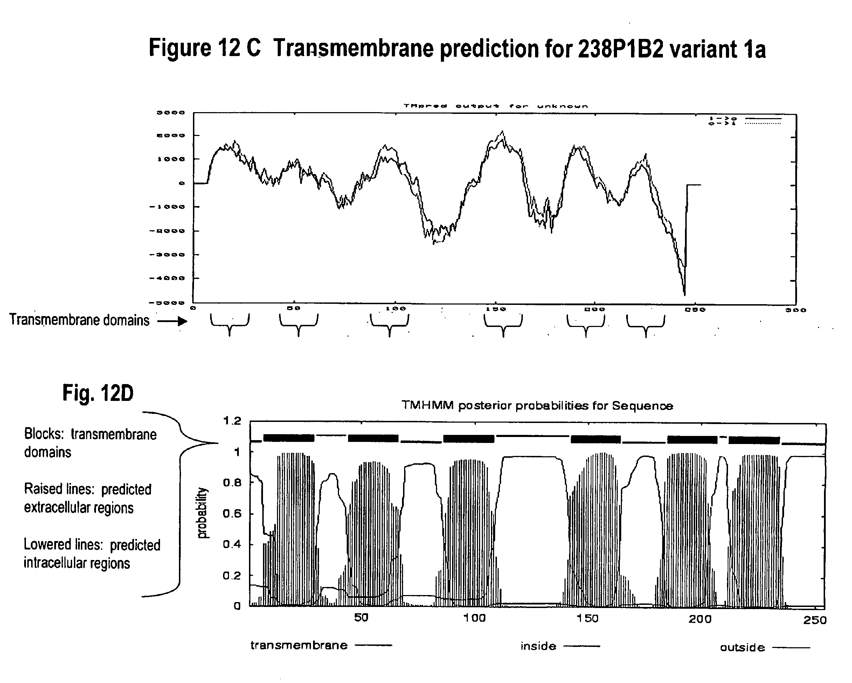

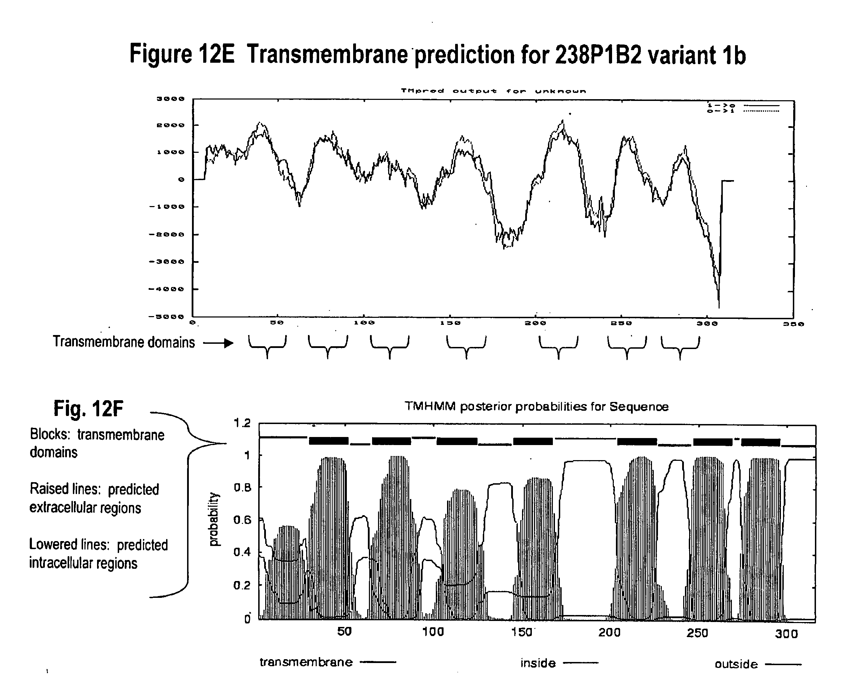

**Figure 12****.** A, B. Secondary structure prediction for 238P1B2 variant 1a and variantlb. The secondary structures of 238P1B2 variant 1a (A) and of variant 1b (B) proteins were predicted using the HNN - Hierarchical Neural Network method (Guermeur, 1997, at World Wide Web URL pbil.ibcp.fr/cgibin/npsa_automat.pl?page=npsa_nn.html), accessed from the ExPasy molecular biology server (at World Wide Web URL www.expasy.ch/tools/). This method predicts the presence and location of alpha helices, extended strands, and random coils from the primary protein sequence. The percent of the protein in a given secondary structure is also listed for each variant.
   C, D, E, F. Transmembrane prediction for 238P1B2 variant 1a and 1b. C, E. Schematic representations of the probability of existence of transmembrane regions and orientation of 238P1B2 variant 1a (C) and variant 1b (E) based on the TMpred algorithm of Hofmann and Stoffel which utilizes TMBASE (K. Hofmann, W. Stoffel. TMBASE - A database of membrane spanning protein segments Biol. Chem. Hoppe-Seyler 374:166, 1993). D, F. Schematic representation of the probability of the existence of transmembrane regions and the extracellular and intracellular orientation of 238P1B2 variant 1a (D) and variant 1b (F) based on the TMHMM algorithm of Sonnhammer, von Heijne, and Krogh (Erik L.L. Sonnhammer, Gunnar von Heijne, and Anders Krogh: A hidden Markov model for predicting transmembrane helices in protein sequences. In Proc. of Sixth Int. Conf. on Intelligent Systems for Molecular Biology, p 175-182 Ed J. Glasgow, T. Littlejohn, F. Major, R. Lathrop, D. Sankoff, and C. Sensen Menlo Park, CA: AAAI Press, 1998). The TMpred and TMHMM algorithms are accessed from the ExPasy molecular biology server (at World Wide Web URL www.expasy.ch/tools/). The results of the transmembrane prediction programs presented depict 238P1B2 variant 1a as most likely containing 6 transmembrane domains and variant 1 b 7 transmembrane domains.

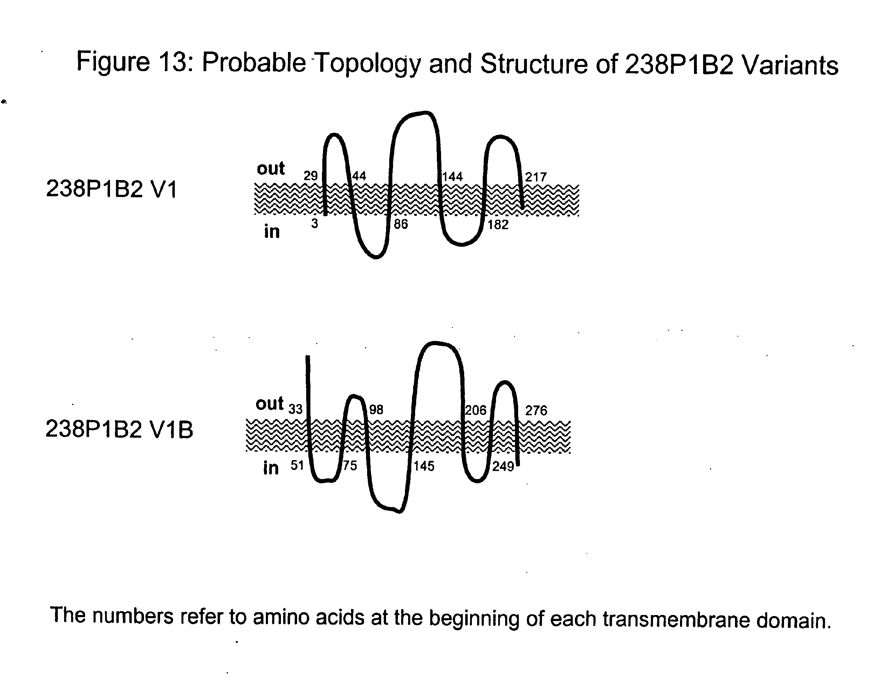

**Figure 13****:** Probable topology and structure of 238P1B2 variants I and 1B.

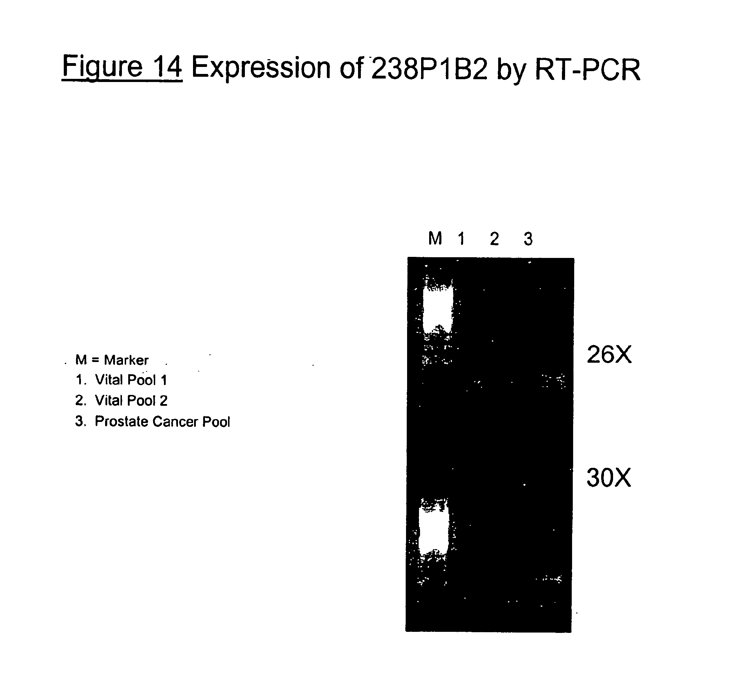

**Figure 14****.** Expression of 238P1B2 by RT-PCR. First strand cDNA was prepared from vital pool 1 (liver, lung and kidney), vital pool 2 (pancreas, colon and stomach), and prostate cancer pool. Normalization was performed by PCR using primers to actin and GAPDH. Semi-quantitative PCR, using primers to 238P1B2, was performed at 26 and 30 cycles of amplification. Results show strong expression of 238P1B2 in prostate cancer pool but not in vital pool I and vital pool 2.

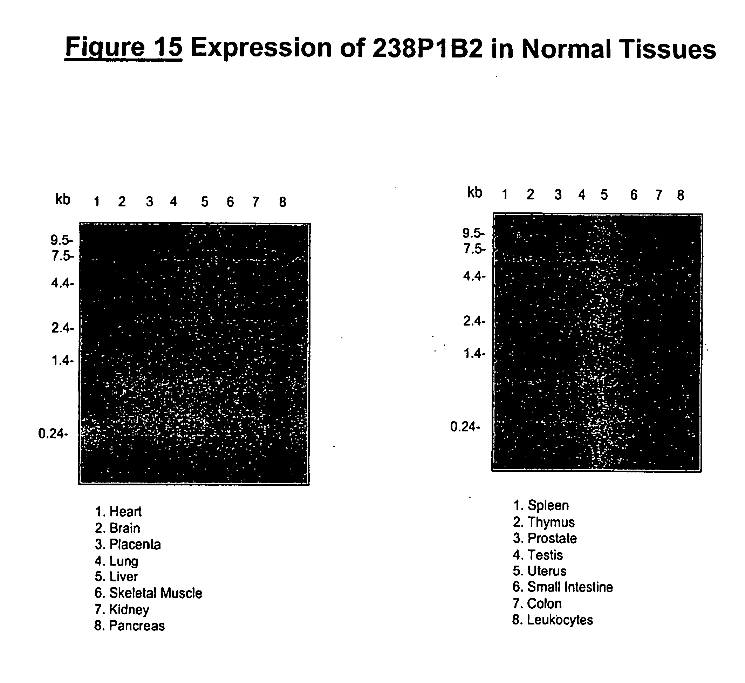

**Figure 15****.** Expression of 238P1B2 in normal tissues. Two multiple tissue northern blots (Clontech) both with 2 ug of mRNA/lane were probed with the 238P1B2 SSH fragment. Size standards in kilobases (kb) are indicated on the side. Results show absence of 238P1B2 expression in all 16 normal tissues tested.

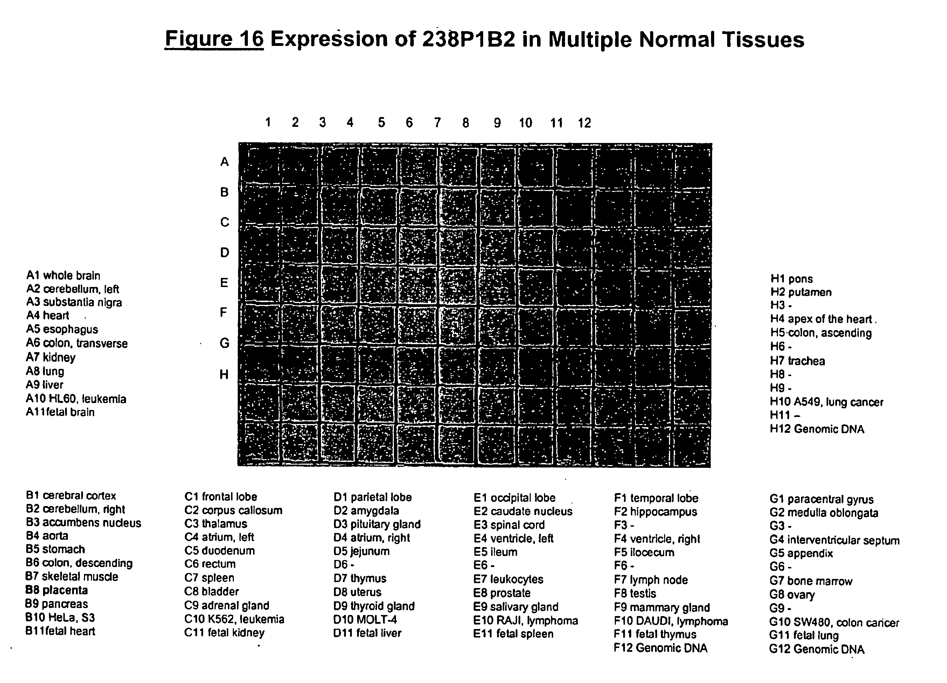

**Figure 16****.** Expression of 238P1B2 in Multiple Normal Tissues. An mRNA dot blot containing 76 different samples from human tissues was analyzed using a 238P1B2 SSH probe. Expression was only detected in placenta.

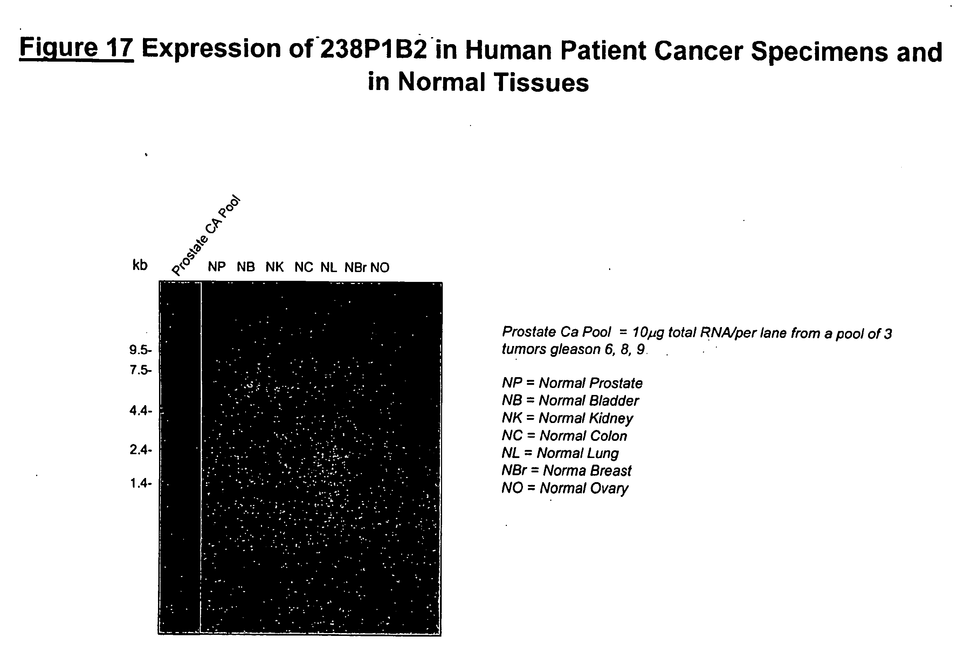

**Figure 17****.** Expression of 238P1B2 in Human Patient Cancer Specimens and Normal Tissues. RNA was extracted from a pool of three prostate cancer patient specimens, as well as from normal prostate (NP), normal bladder (NB), normal kidney (NK), normal colon (NC), normal lung (NL), normal breast (NBr), and normal ovary (NO. Northern blot with 10 µg of total RNA/lane was probed with 238P1B2 SSH sequence. Size standards in kilobases (kb) are indicated on the side. The results show expression of 238P1B2 in the prostate cancer pool and normal ovary.

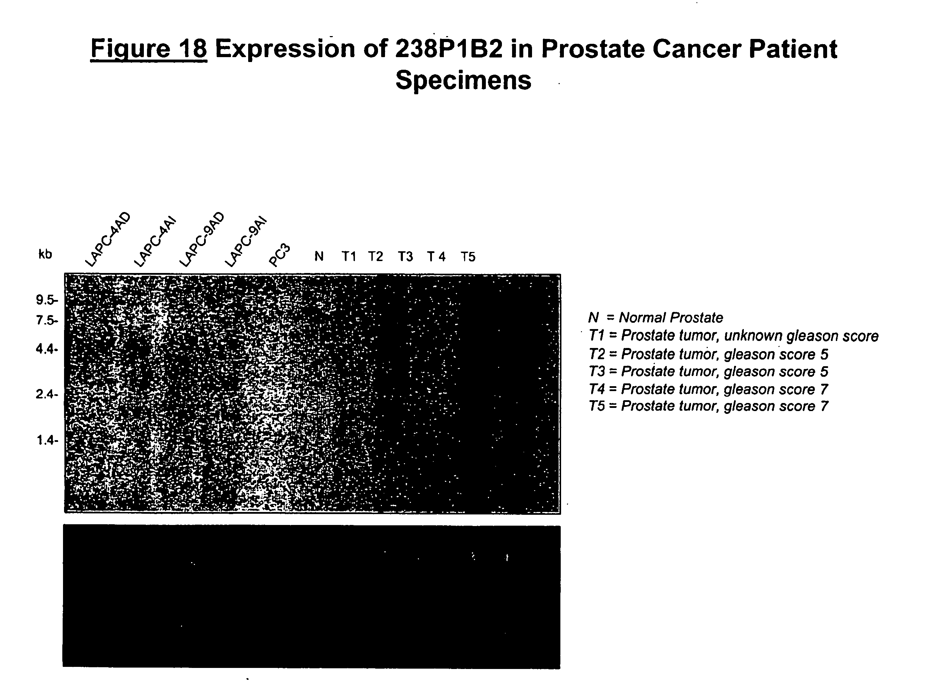

**Figure 18****.** Expression of 238P1B2 in prostate cancer patient tissues. RNA was extracted from normal prostate (N), prostate cancer xenografts (LAPC-4AD, LAPC-4AI, LAPC-9AD, LAPC-9A1), prostate cancer cell lines (LNCaP and PC3), and prostate cancer patient tumors (T). Northern blots with 10 ug of total RNA were probed with the 238P1B2 SSH fragment. Size standards in kilobases are on the side. Results show strong expression of 238P1B2 in prostate tumor tissues. The lower panel represents the ethidium bromide staining of the gel.

### DETAILED DESCRIPTION OF THE INVENTION

### Outline of Sections

### I.) Definitions

### II) 238P1B2 Polynucleotides

### II.A.) Uses of 238P1B2 Polynucleotides

### II.A.1.) Monitoring of Genetic Abnormalities

### II. A.2.) Antisense

### II. A.3.) Primers and Primer Pairs

### II. A.4.) Isolation of 238P1B2-Encoding Nucleic Acid Molecules

### II. A.5.) Recombinant Nucleic Acid Molecules and Host-Vector Systems

### III.) 238P1B2-related Proteins

### III.A.) Motif-bearing Proteins

### III.B.) Expression of 238P1B2-related Proteins

### III.C.) Modifications of 238P1B2-related Proteins

### III.D.) Uses of 238P1B2-related Proteins

### IV.) Methods for the Detection of 238P1B2

### V.) Methods for Monitoring the Status of 238PIB2-related Genes and Their Products

### VI.) Identification of Molecules That Interact With 238P1B2

### VII.) Diagnostic and Prognostic Methods.

### VIII.) KITS

### I.) Definitions:

Unless otherwise defined, all terms of art, notations and other scientific terms or terminology used herein are intended to have the meanings commonly understood by those of skill in the art to which this invention pertains. In some cases, terms with commonly understood meanings are defined herein for clarity and/or for ready reference, and the inclusion of such definitions herein should not necessarily be construed to represent a substantial difference over what is generally understood in the art. Many of the techniques and procedures described or referenced herein are well understood and commonly employed using conventional methodology by those skilled in the art, such as, for example, the widely utilized molecular cloning methodologies described in Sambrook et al., Molecular Cloning: A Laboratory Manual 2nd. edition (1989) Cold Spring Harbor Laboratory Press, Cold Spring Harbor, N. Y. As appropriate, procedures involving the use of commercially available kits and reagents are generally carried out in accordance with manufacturer defined protocols and/or parameters unless otherwise noted.

The terms "advanced prostate cancer", "locally advanced prostate cancer", "advanced disease" and "locally advanced disease" mean prostate cancers that have extended through the prostate capsule, and are meant to include stage C disease under the American Urological Association (AUA) system, stage C1-C2 disease under the Whitmore-Jewett system, and stage T3-T4 and N+ disease under the TNM (tumor, node, metastasis) system. In general, surgery is not recommended for patients with locally advanced disease, and these patients have substantially less favorable outcomes compared to patients having clinically localized (organ-confined) prostate cancer. Locally advanced disease is clinically identified by palpable evidence of induration beyond the lateral border of the prostate, or asymmetry or induration above the prostate base.

Locally advanced prostate cancer is presently diagnosed pathologically following radical prostatectomy if the tumor invades or penetrates the prostatic capsule, extends into the surgical margin, or invades the seminal vesicles.

"Altering the native glycosylation pattern" is intended for purposes herein to mean deleting one or more carbohydrate moieties found in native sequence 238P1B2 (either by removing the underlying glycosylation site or by deleting the glycosylation by chemical and/or enzymatic means), and/or adding one or more glycosylation sites that are not present in the native sequence 238P1B2. In addition, the phrase includes qualitative changes in the glycosylation of the native proteins, involving a change in the nature and proportions of the various carbohydrate moieties present.

The term "analog" refers to a molecule which is structurally similar or shares similar or corresponding attributes with another molecule (e.g. a 238P1B2-related protein). For example an analog of a 238P1B2 protein can be specifically bound by an antibody or T cell that specifically binds to 238P1B2.

The term "antibody" is used in the broadest sense. Therefore an "antibody" can be naturally occurring or man-made such as monoclonal antibodies produced by conventional hybridoma technology. Anti-238P1B2 antibodies comprise monoclonal and polyclonal antibodies as well as fragments containing the antigen-binding domain and/or one or more complementarity determining regions of these antibodies.

An "antibody fragment" is defined as at least a portion of the variable region of the immunoglobulin molecule that binds to its target, i.e., the antigen-binding region. It may specifically cover single anti-238P1B2 antibodies and clones thereof (including agonist, antagonist and neutralizing antibodies) and anti-238P1B2 antibody compositions with polyepitopic specificity.

The term "codon optimized sequences" refers to nucleotide sequences that have been optimized for a particular host species by replacing any codons having a usage frequency of less than about 20%. Nucleotide sequences that have been optimized for expression in a given host species by elimination of spurious polyadenylation sequences, elimination of exon/intron splicing signals, elimination of transposon-like repeats and/or optimization of GC content in addition to codon optimization are referred to herein as an "expression enhanced sequences."

The term "cytotoxic agent" refers to a substance that inhibits or prevents the expression activity of cells, function of cells and/or causes destruction of cells. The term is intended to include radioactive isotopes chemotherapeutic agents, and toxins such as small molecule toxins or enzymatically active toxins of bacterial, fungal, plant or animal origin, including fragments and/or variants thereof. Examples of cytotoxic agents include, but are not limited to maytansinoids, yttrium, bismuth, ricin, ricin A-chain, doxorubicin, daunorubicin, taxol, ethidium bromide, mitomycin, etoposide, tenoposide, vincristine, vinblastine, colchicine, dihydroxy anthracin dione, actinomycin, diphtheria toxin, Pseudomonas exotoxin (PE) A, PE40, abrin, abrin A chain, modeccin A chain, alpha-sarcin, gelonin, mitogellin, retstrictocin, phenomycin, enomycin, curicin, crotin, calicheamicin, sapaonaria officinalis inhibitor, and glucocorticoid and other chemotherapeutic agents, as well as radioisotopes such as At²¹¹, I¹³¹, I¹²⁵, Y⁹⁰, Re¹⁸⁶, R¹⁸⁸, S¹⁵³, Bi²¹², P³² and radioactive isotopes of Lu. Antibodies may also be conjugated to an anti-cancer pro-drug activating enzyme capable of converting the pro-drug to its active form.

The term "homolog" refers to a molecule which exhibits homology to another molecule, by for example, having sequences of chemical residues that are the same or similar at corresponding positions.

"Human Leukocyte Antigen" or "HLA" is a human class I or class II Major Histocompatibility Complex (MHC) protein (see, e.g., Stites, et al., IMMUNOLOGY, 8TH ED., Lange Publishing, Los Altos, CA (1994).

The terms "hybridize", "hybridizing", "hybridizes" and the like, used in the context of polynucleotides, are meant to refer to conventional hybridization conditions, preferably such as hybridization in 50% formamide/6XSSC/0.1% SDS/100 µg/ml ssDNA, in which temperatures for hybridization are above 37 degrees C and temperatures for washing in 0.1XSSC/0.1% SDS are above 55 degrees C.

The phrases "isolated" or "biologically pure" refer to material which is substantially or essentially free from components which normally accompany the material as it is found in its native state. Thus, isolated peptides described herein preferably do not contain materials normally associated with the peptides in their in situ environment. For example, a polynucleotide is said to be "isolated" when it is substantially separated from contaminant polynucleotides that correspond or are complementary to genes other than the 238P1B2 genes or that encode polypeptides other than 238P1B2 gene product or fragments thereof. A skilled artisan can readily employ nucleic acid isolation procedures to obtain an isolated 238P1B2 polynucleotide.

A protein is said to be "isolated" for example, when physical, mechanical or chemical methods are employed to remove the 238P1B2 proteins from cellular constituents that are normally associated with the protein. A skilled artisan can readily employ standard purification methods to obtain an isolated 238P1B2 protein. Alternatively, an isolated protein can be prepared by chemical means.

The term "mammal" refers to any organism classified as a mammal, including mice, rats, rabbits, dogs, cats, cows, horses and humans. The mammal may be a mouse. The mammal may be a human.

The terms "metastatic prostate cancer" and "metastatic disease" mean prostate cancers that have spread to regional lymph nodes or to distant sites, and are meant to include stage D disease under the AUA system and stage TxNxM+ under the TNM system. As is the case with locally advanced prostate cancer, surgery is generally not indicated for patients with metastatic disease, and hormonal (androgen ablation) therapy is a preferred treatment modality. Patients with metastatic prostate cancer eventually develop an androgen-refractory state within 12 to 18 months of treatment initiation. Approximately half of these androgen-refractory patients die within 6 months after developing that status. The most common site for prostate cancer metastasis is bone. Prostate cancer bone metastases are often osteoblastic rather than osteolytic (i.e., resulting in net bone formation). Bone metastases are found most frequently in the spine, followed by the femur, pelvis, rib cage, skull and humerus. Other common sites for metastasis include lymph nodes, lung, liver and brain. Metastatic prostate cancer is typically diagnosed by open or laparoscopic pelvis lymphadenectomy, whole body radionuclide scans, skeletal radiography, and/or bone lesion biopsy.

The term "monoclonal antibody" refers to an antibody obtained from a population of substantially homogeneous antibodies, i.e., the antibodies comprising the population are identical except for possible naturally occurring mutations that are present in minor amounts.

A "motif', as in biological motif of an 238P 1B2-related protein, refers to any pattern of amino acids forming part of the primary sequence of a protein, that is associated with a particular function (e.g. protein-protein interaction, protein-DNA interaction, etc) or modification (e.g. that is phosphorylated, glycosylated or amidated), or localization (e.g. secretory sequence, nuclear localization sequence, etc.) or a sequence that is correlated with being immunogenic, either humorally or cellularly. A motif can be either contiguous or capable of being aligned to certain positions that are generally correlated with a certain function or property. In the context of HLA motifs, "motif" refers to the pattern of residues in a peptide of defined length, usually a peptide of from about 8 to about 13 amino acids for a class I HLA motif and from about 6 to about 25 amino acids for a class II HLA motif, which is recognized by a particular HLA molecule. Peptide motifs for HLA binding are typically different for each protein encoded by each human HLA allele and differ in the pattern of the primary and secondary anchor residues.

A "pharmaceutical excipient" comprises a material such as an adjuvant, a carrier, pH-adjusting and buffering agents, tonicity adjusting agents, wetting agents, preservative, and the like.

"Pharmaceutically acceptable" refers to a non-toxic, inert, and/or composition that is physiologically compatible with humans or other mammals.

The term "polynucleotide" means a polymeric form of nucleotides of at least 10 bases or base pairs in length, either ribonucleotides or deoxynucleotides or a modified form of either type of nucleotide, and is meant to include single and double stranded forms of DNA and/or RNA. In the art, this term if often used interchangeably with "oligonucleotide". A polynucleotide can comprise a nucleotide sequence disclosed herein wherein thymine (T), as shown for example in Figure 2, can also be uracil (U); this definition pertains to the differences between the chemical structures of DNA and RNA, in particular the observation that one of the four major bases in RNA is uracil (U) instead of thymine (T).

The term "polypeptide" means a polymer of at least about 4, 5, 6, 7, or 8 amino acids. Throughout the specification, standard three letter or single letter designations for amino acids are used. In the art, this term is often used interchangeably with "peptide" or "protein".

An HLA "primary anchor residue" is an amino acid at a specific position along a peptide sequence which is understood to provide a contact point between the immunogenic peptide and the HLA molecule. One to three, usually two, primary anchor residues within a peptide of defined length generally defines a "motif" for an immunogenic peptide. These residues are understood to fit in close contact with peptide binding groove of an HLA molecule, with their side chains buried in specific pockets of the binding groove. For example, the primary anchor residues for an HLA class I molecule may be located at position 2 (from the amino terminal position) and at the carboxyl terminal position of a 8, 9, 10, 11, or 12 residue peptide epitope described herein. The primary anchor residues of a peptide that will bind an HLA class II molecule may be spaced relative to each other, rather than to the termini of a peptide, where the peptide is generally of at least 9 amino acids in length.

A "recombinant" DNA or RNA molecule is a DNA or RNA molecule that has been subjected to molecular manipulation *in vitro.*

Non-limiting examples of small molecules include compounds that bind or interact with 238P1B2, ligands including hormones, neuropeptides, chemokines, odorants, phospholipids, and functional equivalents thereof that bind and preferably inhibit 238P1B2 protein function. Such non-limiting small molecules preferably have a molecular weight of less than about 10 kDa, more preferably below about 9, about 8, about 7, about 6, about 5 or about 4 kDa. Small molecules may physically associate with, or bind, 238P1B2 protein; are not found in naturally occurring metabolic pathways; and/or are more soluble in aqueous than non-aqueous solutions.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured nucleic acid sequences to reanneal when complementary strands are present in an environment below their melting temperature. The higher the degree of desired homology between the probe and hybridizable sequence, the higher the relative temperature that can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).

"Stringent conditions" or "high stringency conditions", as defined herein, are identified by, but not limited to, those that: (1) employ low ionic strength and high temperature for washing, for example 0.015 M sodium chloride/0.0015 M sodium citrate/0.1% sodium dodecyl sulfate at 50°C; (2) employ during hybridization a denaturing agent, such as formamide, for example, 50% (v/v) formamide with 0.1% bovine serum albumin/0.1% Ficoll/0.1% polyvinylpyrrolidone/50 mM sodium phosphate buffer at pH 6.5 with 750 mM sodium chloride, 75 mM sodium citrate at 42°C; or (3) employ 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 µg/ml), 0.1% SDS, and 10% dextran sulfate at 42°C, with washes at 42°C in 0.2 x SSC (sodium chloride/sodium. citrate) and 50% formamide at 55°C, followed by a high-stringency wash consisting of 0.1 x SSC containing EDTA at 55°C. "Moderately stringent conditions" are described by, but not limited to, those in Sambrook et al., Molecular Cloning: A Laboratory Manual, New York: Cold Spring Harbor Press, 1989, and include the use of washing solution and hybridization conditions (e.g., temperature, ionic strength and %SDS) less stringent than those described above. An example of moderately stringent conditions is overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/mL denatured sheared salmon sperm DNA, followed by washing the filters in 1 x SSC at about 37-50°C. The skilled artisan will recognize how to adjust the temperature, ionic strength, etc. as necessary to accommodate factors such as probe length and the like.

An HLA "supermotif" is a peptide binding specificity shared by HLA molecules encoded by two or more HLA alleles.

As used herein "to treat" or "therapeutic" and grammatically related terms, refer to any improvement of any consequence of disease, such as prolonged survival, less morbidity, and/or a lessening of side effects which are the byproducts of an alternative therapeutic modality; full eradication of disease is not required.

A "transgenic animal" (e.g., a mouse or rat) is an animal having cells that contain a transgene, which transgene was introduced into the animal or an ancestor of the animal at a prenatal, e.g., an embryonic stage. A "transgene" is a DNA that is integrated into the genome of a cell from which a transgenic animal develops.

As used herein, an HLA or cellular immune response "vaccine" is a composition that contains or encodes one or more peptides described herein. There are numerous such vaccines, such as a cocktail of one or more individual peptides; one or more peptides described herein comprised by a polyepitopic peptide; or nucleic acids that encode such individual peptides or polypeptides, e.g., a minigene that encodes a polyepitopic peptide. The "one or more peptides" can include any whole unit integer from 1- 150 or more, e.g., at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 55, 60, 65, 70, 75, 80, 85, 90, 95, 100, 105, 110, 115, 120, 125, 130, 135, 140, 145, or 150 or more peptides described herein. The peptides or polypeptides can optionally be modified, such as by lipidation, addition of targeting or other sequences. HLA class I peptides described herein can be admixed with, or linked to, HLA class II peptides, to facilitate activation of both cytotoxic T lymphocytes and helper T lymphocytes. HLA vaccines can also comprise peptide-pulsed antigen presenting cells, e.g., dendritic cells.

The term "variant" refers to a molecule that exhibits a variation from a described type or norm, such as a protein that has one or more different amino acid residues in the corresponding position(s) of a specifically described protein (e.g. the 238P1B2 protein shown in Figure 2 or Figure 3. An analog is an example of a variant protein. Splice isoforms and single nucleotides polymorphisms (SNPs) are further examples of variants.

"238P1B2-related proteins" include those specifically identified herein, as well as allelic variants, conservative substitution variants, analogs and homologs that can be isolated/generated and characterized without undue experimentation following the methods outlined herein or readily available in the art. Fusion proteins that combine parts of different 238P1B2 proteins or fragments thereof, as well as fusion proteins of a 238P1B2 protein and a heterologous polypeptide are also included. Such 238P1B2 proteins are collectively referred to as the 238P1B2-related proteins, the proteins described herein, or 238P1B2. The term "238P1B2-related protein" refers to a polypeptide fragment or an 238P1B2 protein sequence of 4, 5, 6, 7, 8, 9, 10, 11, 12, 13, 14, 15, 16, 17, 18, 19, 20, 21, 22, 23, 24, 25, or more than 25 amino acids; or, at least 30, 35, 40, 45, 50, 55, 60, 65, 70, 80, 85, 90, 95, 100 or more than 100 amino acids.

### II) 238P1B2 Polynucleotides

We describe polynucleotides corresponding or complementary to all or part of an 238P1B2 gene, mRNA, and/or coding sequence, preferably in isolated form, including polynucleotides encoding an 238P1B2-related protein and fragments thereof, DNA, RNA, DNA/RNA hybrid, and related molecules, polynucleotides or oligonucleotides complementary to an 238P1B2 gene or mRNA sequence or a part thereof, and polynucleotides or oligonucleotides that hybridize to an 238P1B2 gene, mRNA, or to an 238P1B2 encoding polynucleotide (collectively, "238P1B2 polynucleotides"). In all instances when referred to in this section, T can also be U in Figure 2.

238P1B2 polynucleotides include: a 238P1B2 polynucleotide having the sequence shown in Figure 2, the nucleotide sequence of 238P1B2 as shown in Figure 2 wherein T is U; at least 10 contiguous nucleotides of a polynucleotide having the sequence as shown in Figure 2; or, at least 10 contiguous nucleotides of a polynucleotide having the sequence as shown in Figure 2 where T is U. For example, 238P1B2 nucleotides comprise, without limitation:
(I) a polynucleotide comprising, consisting essentially of, or consisting of a sequence as shown in Figure 2, wherein T can also be U;
(II) a polynucleotide comprising, consisting essentially of, or consisting of the sequence as shown in Figure 2A, from nucleotide residue number 2133 through nucleotide residue number 2894, followed by a stop codon, wherein T can also be U;
(III) a polynucleotide comprising, consisting essentially of, or consisting of the sequence as shown in Figure 2B, from nucleotide residue number 1947 through nucleotide residue number 2894, followed by a stop codon, wherein T can also be U;
(IV) a polynucleotide comprising, consisting essentially of, or consisting of the sequence as shown in Figure 2C, from nucleotide residue number 2133 through nucleotide residue number 2894, followed by a stop codon, wherein T can also be U;
(V) a polynucleotide comprising, consisting essentially of, or consisting of the sequence as shown in Figure 2D, from nucleotide residue number 2133 through nucleotide residue number 2894, followed by a stop codon, wherein T can also be U;
(VI) a polynucleotide comprising, consisting essentially of, or consisting of the sequence as shown in Figure 2E from nucleotide residue number 2133 through nucleotide residue number 2894, followed by a stop codon, wherein T can also be U;
(VII) a polynucleotide comprising, consisting essentially of, or consisting of the sequence as shown in Figure 2F from nucleotide residue number 2133 through nucleotide residue number 2894, followed by a stop codon, wherein T can also be U;
(VIII) a polynucleotide comprising, consisting essentially of, or consisting of the sequence as shown in Figure 2G, from nucleotide residue number 2133 through nucleotide residue number 2894, followed by a stop codon, wherein T can also be U;
(IX) a polynucleotide that encodes an 238PIB2-related protein that is at least 90% homologous to an entire amino acid sequence shown in Figure 2A-G;
(X) a polynucleotide that encodes an 238P1B2-related protein that is at least 90% identical to an entire amino acid sequence shown in Figure 2A-G;
(XI) a polynucleotide that encodes at least one peptide set forth in Tables V-XVIII or Table XIX ;
(XII) a polynucleotide that encodes a peptide region of at least 5 amino acids of a peptide of Figure 3A in any whole number increment up to 254 that includes an amino acid position having a value greater than 0.5 in the Hydrophilicity profile of Figure 5A, or of Figure 3B in any whole number increment up to 316 that includes an amino acid position having a value greater than 0.5 in the Hydrophilicity profile of Figure 5B;
(XIII) a polynucleotide that encodes a peptide region of at least 5 amino acids of a peptide of Figure 3A in any whole number increment up to 254 that includes an amino acid position having a value less than 0.5 in the Hydropathicity profile of Figure 6A, or of Figure 3B in any whole number increment up to 316, that includes an amino acid position having a value less than 0.5 in the Hydropathicity profile of Figure 6B;
(XIV) a polynucleotide that encodes a peptide region of at least 5 amino acids of a peptide of Figure 3A in any whole number increment up to 254 that includes an amino acid position having a value greater than 0.5 in the Percent Accessible Residues profile of Figure 7A, or of Figure 3B in any whole number increment up to 316, that includes an amino acid position having a value greater than 0.5 in the Percent Accessible Residues profile of Figure 7B;
(XV) a polynucleotide that encodes a peptide region of at least 5 amino acids of a peptide of Figure 3A in any whole number increment up to 254 that includes an amino acid position having a value greater than 0.5 in the Average Flexibility profile on Figure 8A, or of Figure 3B in any whole number increment up to 316, that includes an amino acid position having a value greater than 0.5 in the Average Flexibility profile on Figure 8B;
(XVI) a polynucleotide that encodes a peptide region of at least 5 amino acids of a peptide of Figure 3A in any whole number increment up to 254 that includes an amino acid position having a value greater than 0.5 in the Beta-turn profile of Figure 9A, or of Figure 3B in any whole number increment up to 316, that includes an amino acid position having a value greater than 0.5 in the Beta-turn profile of Figure 9B;
(XVII) a polynucleotide that encodes a 238P1B2-related protein whose sequence is encoded by the cDNAs contained in the plasmid deposited with American Type Culture Collection as Accession No. PTA-4124 on March 7, 2002;
(XVIII) a polynucleotide that is fully complementary to a polynucleotide of any one of (I)-(XVII);
(XIX) a polynucleotide that selectively hybridizes under stringent conditions to a polynucleotide of (I)-(XVIII);
(XX) a peptide that is encoded by any of (I)-(XIX); and,
(XXI) a polynucleotide of any of (I)-(XIX)or peptide of (XX) together with a pharmaceutical excipient and/or in a human unit dose form

As used herein, a range is understood to specifically disclose all whole unit positions thereof. Typical 238P1B2 polynucleotides encode specific portions of 238P1B2 mRNA sequences (and those which are complementary to such sequences) such as those that encode the proteins and/or fragments thereof, for example:
(a) 4, 5, 6, 7, 8, 9, 10, 11, 12, 13, 14, 15, 16, 17, 18, 19, 20, 21, 22, 23, 24, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, or 254 contiguous amino acids of 238P1B2;
(b) 4, 5, 6, 7, 8, 9, 10, 11, 12, 13, 14, 15, 16, 17, 18, 19, 20, 21, 22, 23, 24, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, or 254 contiguous amino acids of variant 1A;
(c) 4, 5, 6, 7, 8, 9, 10, 11, 12, 13, 14, 15, 16, 17, 18, 19, 20, 21, 22, 23, 24, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 265, 270, 275, 280, 285, 290, 295, 300, 305, 310, 315, or 316 contiguous amino acids of variant 1B; or
(d) 4, 5, 6, 7, 8, 9, 10, 11, 12, 13, 14, 15, 16, 17, 18, 19, 20, 21, 22, 23, 24, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, or 254 contiguous amino acids of variant 2.

For example, polynucleotides and their encoded peptides are described herein, representative polynucleotides may encode about amino acid 1 to about amino acid 10 of the 238P1B2 protein or variants shown in Figure 2 or Figure 3, polynucleotides encoding about amino acid 10 to about amino acid 20 of the 238P1B2 protein or variants shown in Figure 2 or Figure 3, polynucleotides encoding about amino acid 20 to about amino acid 30 of the 238P1B2 protein or variants shown in Figure 2 or Figure 3, polynucleotides encoding about amino acid 30 to about amino acid 40 of the 238P1B2 protein or variants shown in Figure 2 or Figure 3, polynucleotides encoding about amino acid 40 to about amino acid 50 of the 238P1B2 protein or variants shown in Figure 2 or Figure 3, polynucleotides encoding about amino acid 50 to about amino acid 60 of the 238P1B2 protein or variants shown in Figure 2 or Figure 3, polynucleotides encoding about amino acid 60 to about amino acid 70 of the 238P1B2 protein or variants shown in Figure 2 or Figure 3, polynucleotides encoding about amino acid 70 to about amino acid 80 of the 238P1B2 protein or variants shown in Figure 2 or Figure 3, polynucleotides encoding about amino acid 80 to about amino acid 90 of the 238P1B2 protein or variants shown in Figure 2 or Figure 3, polynucleotides encoding about amino acid 90 to about amino acid 100 of the 238P1B2 protein or variants shown in Figure 2 or Figure 3, or encoding regions from about amino acid 100 to amino acids later in the sequence, in increments of about 10 amino acids, ending at the carboxyl terminal amino acid set forth in Figure 2 or Figure 3. Accordingly polynucleotides encoding portions of the amino acid sequence (of about 10 amino acids), of amino acids 100 through the carboxyl terminal amino acid of the 238P1B2 protein are described herein. Wherein it is understood that each particular amino acid position discloses that position plus or minus five amino acid residues.

Polynucleotides encoding relatively long portions of a 238P1B2 protein are also described herein. For example, polynucleotides encoding from about amino acid 1 (or 20 or 30 or 40 etc.) to about amino acid 20, (or 30, or 40 or 50 etc.) of the 238P1B2 protein or variants shown in Figure 2 or Figure 3 can be generated by a variety of techniques well known in the art. These polynucleotide fragments can include any portion of the 238P1B2 sequence or variants as shown in Figure 2.

Additional illustrative polynucleotides include 238P1B2 polynucleotide fragments encoding one or more of the biological motifs contained within a 238P1B2 protein sequence or variant sequence.

We describe antibody epitopes which comprise a peptide region, or an oligonucleotide encoding the peptide region, that has one two, three, four, or five of the following characteristics:
i) a peptide region of at least 5 amino acids of a particular peptide of Figure 3, in any whole number increment up to the full length of that protein in Figure 3, that includes an amino acid position having a value equal to or greater than 0.5, 0.6, 0.7, 0.8, 0.9, or having a value equal to 1.0, in the Hydrophilicity profile of Figure 5;
ii) a peptide region of at least 5 amino acids of a particular peptide of Figure 3, in any whole number increment up to the full length of that protein in Figure 3, that includes an amino acid position having a value equal to or less than 0.5, 0.4, 0.3, 0.2, 0.1, or having a value equal to 0.0, in the Hydropathicity profile of Figure 6;
iii) a peptide region of at least 5 amino acids of a particular peptide of Figure 3, in any whole number increment up to the full length of that protein in Figure 3, that includes an amino acid position having a value equal to or greater than 0.5, 0.6, 0.7, 0.8, 0.9, or having a value equal to 1.0, in the Percent Accessible Residues profile of Figure 7;
iv) a peptide region of at least 5 amino acids of a particular peptide of Figure 3, in any whole number increment up to the full length of that protein in Figure 3, that includes an amino acid position having a value equal to or greater than 0.5, 0.6, 0.7, 0.8, 0.9, or having a value equal to 1.0, in the Average Flexibility profile of Figure 8; or
v) a peptide region of at least 5 amino acids of a particular peptide of Figure 3, in any whole number increment up to the full length of that protein in Figure 3, that includes an amino acid position having a value equal to or greater than 0.5, 0.6, 0.7, 0.8, 0.9, or having a value equal to 1.0, in the Beta-turn profile of Figure 9.

Typical polynucleotide fragments encode one or more of the regions of 238P1B2 protein or variant that exhibit homology to a known molecule. Typical polynucleotide fragments can encode one or more of the 238P1B2 protein or variant N-glycosylation sites, cAMP and cGMP-dependent protein kinase phosphorylation sites, casein kinase II phosphorylation sites or N-myristoylation site and amidation sites.

### II.A.) Uses of 238P1B2 Polynucleotides

### II.A.1.) Monitoring of Genetic Abnormalities

The polynucleotides of the preceding paragraphs have a number of different specific uses. The human 238P1B2 gene maps to the chromosomal location set forth in Example 3. For example, because the 238P1B2 gene maps to this chromosome, polynucleotides that encode different regions of the 238P1B2 proteins are used to characterize cytogenetic abnormalities of this chromosomal locale, such as abnormalities that are identified as being associated with various cancers. In certain genes, a variety of chromosomal abnormalities including rearrangements have been identified as frequent cytogenetic abnormalities in a number of different cancers (see e.g. Krajinovic et al., Mutat. Res. 382(3-4): 81-83 (1998); Johansson et al., Blood 86(10): 3905-3914 (1995) and Finger et al., P.N.A.S. 85(23): 9158-9162 (1988)). Thus, polynucleotides encoding specific regions of the 238P1B2 proteins provide new tools that can be used to delineate, with greater precision than previously possible, cytogenetic abnormalities in the chromosomal region that encodes 238P1B2 that may contribute to the malignant phenotype. In this context, these polynucleotides satisfy a need in the art for expanding the sensitivity of chromosomal screening in order to identify more subtle and less common chromosomal abnormalities (see e. g. Evans et al., Am. J. Obstet. Gynecol 171(4): 1055-1057 (1994)).

Furthermore, as 238P1B2 was shown to be highly expressed in bladder and other cancers, 238P1B2 polynucleotides are used in methods assessing the status of 238P1B2 gene products in normal versus cancerous tissues. Typically, polynucleotides that encode specific regions of the 238P1B2 proteins are used to assess the presence of perturbations (such as deletions, insertions, point mutations, or alterations resulting in a loss of an antigen etc.) in specific regions of the 238P1B2 gene, such as regions containing one or more motifs. Exemplary assays include both RT-PCR assays as well as single-strand conformation polymorphism (SSCP) analysis (see, e. g., Marrogi et al., J. Cutan. Pathol. 26(8): 369-378 (1999), both of which utilize polynucleotides encoding specific regions of a protein to examine these regions within the protein.

### II.A.2.) Antisense

We describe genomic DNA, cDNAs, ribozymes, and antisense molecules, as well as nucleic acid molecules based on an alternative backbone, or including alternative bases, whether derived from natural sources or synthesized, and include molecules capable of inhibiting the RNA or protein expression of 238P1B2. For example, antisense molecules can be RNAs or other molecules, including peptide nucleic acids (PNAs) or non-nucleic acid molecules such as phosphorothioate derivatives, that specifically bind DNA or RNA in a base pair-dependent manner. A skilled artisan can readily obtain these classes of nucleic acid molecules using the 238P1B2 polynucleotides and polynucleotide sequences disclosed herein.

Antisense technology entails the administration of exogenous oligonucleotides that bind to a target polynucleotide located within the cells. The term "antisense" refers to the fact that such oligonucleotides are complementary to their intracellular targets, e.g., 238P1B2. See for example, Jack Cohen, Oligodeoxynucleotides, Antisense Inhibitors of Gene Expression, CRC Press, 1989; and Synthesis 1:1-5 (1988). The 238P1B2 antisense oligonucleotides described herein include derivatives such as S-oligonucleotides (phosphorothioate derivatives or S-oligos, see, Jack Cohen, supra), which exhibit enhanced cancer cell growth inhibitory action. S-oligos (nucleoside phosphorothioates) are isoelectronic analogs of an oligonucleotide (O-oligo) in which a nonbridging oxygen atom of the phosphate group is replaced by a sulfur atom. The S-oligos described herein can be prepared by treatment of the corresponding O-oligos with 3H-1,2-benzodithiol-3-one-1,1-dioxide, which is a sulfur transfer reagent. See, e.g., Iyer, R. P. et al., J. Org. Chem. 55: 4693-4698 (1990); and Iyer, R. P. et al., J. Am. Chem. Soc. 112: 1253-1254 (1990). Additional 238P1B2 antisense oligonucleotides include morpholino antisense oligonucleotides known in the art (see, e.g., Partridge et al., 1996, Antisense & Nucleic Acid Drug Development 6: 169-175).

The 238P1B2 antisense oligonucleotides described herein typically can be RNA or DNA that is complementary to and stably hybridizes with the first 100 5' codons or last 100 3' codons of a 238P1B2 genomic sequence or the corresponding mRNA. Absolute complementarity is not required, although high degrees of complementarity are preferred. Use of an oligonucleotide complementary to this region allows for the selective hybridization to 238P1B2 mRNA and not to mRNA specifying other regulatory subunits of protein kinase. 238P1B2 antisense oligonucleotides may be 15 to 30-mer fragments of the antisense DNA molecule that have a sequence that hybridizes to 238P1B2 mRNA. Optionally, 238P1B2 antisense oligonucleotide is a 30-mer oligonucleotide that is complementary to a region in the first 10 5' codons or last 10 3' codons of 238P1B2. Alternatively, the antisense molecules are modified to employ ribozymes in the inhibition of 238P1B2 expression, see, e.g., L. A. Couture & D. T. Stinchcomb; Trends Genet 12: 510-515 (1996).

### II.A.3.) Primers and Primer Pairs

We describe primers and primer pairs, which allow the specific amplification of polynucleotides described herein or of any specific parts thereof, and probes that selectively or specifically hybridize to nucleic acid molecules described herein or to any part thereof. Probes can be labeled with a detectable marker, such as, for example, a radioisotope, fluorescent compound, bioluminescent compound, a chemiluminescent compound, metal chelator or enzyme. Such probes and primers are used to detect the presence of a 238P1B2 polynucleotide in a sample and as a means for detecting a cell expressing a 238P1B2 protein.

Examples of such probes include polypeptides comprising all or part of the human 238P1B2 cDNA sequence shown in Figure 2. Examples of primer pairs capable of specifically amplifying 238P1B2 mRNAs are also described in the Examples. As will be understood by the skilled artisan, a great many different primers and probes can be prepared based on the sequences provided herein and used effectively to amplify and/or detect a 238P1B2 mRNA.

The 238P1B2 polynucleotides described herein are useful for a variety of purposes, including but not limited to their use as probes and primers for the amplification and/or detection of the 238P1B2 gene (s), mRNA (s), or fragments thereof; as reagents for the diagnosis and/or prognosis of prostate cancer and other cancers; as coding sequences capable of directing the expression of 238P1B2 polypeptides; as tools for modulating or inhibiting the expression of the 238P1B2 gene (s) and/or translation of the 238P1B2 transcript (s); and as therapeutic agents.

Any probe as described herein may be used to identify and isolate a 238P1B2 or 238P1B2 related nucleic acid sequence from a naturally occurring source, such as humans or other mammals, as well as the isolated nucleic acid sequence per se, which would comprise all or most of the sequences found in the probe used.

### II.A.4) Isolation of 238P1B2-Encodine Nucleic Acid Molecules

The 238P1B2 cDNA sequences described herein enable the isolation of other polynucleotides encoding 238P1B2 gene product(s), as well as the isolation of polynucleotides encoding 238P1B2 gene product homologs, alternatively spliced isoforms, allelic variants, and mutant forms of a 238P1B2 gene product as well as polynucleotides that encode analogs of 238P1B2-related proteins. Various molecular cloning methods that can be employed to isolate full length cDNAs encoding an 238P1B2 gene are well known (see, for example, Sambrook, J. et al, Molecular Cloning: A Laboratory Manual, 2nd edition, Cold Spring Harbor Press, New York, 1989; Current Protocols in Molecular Biology. Ausubel et al, Eds., Wiley and Sons, 1995). For example, lambda phage cloning methodologies can be conveniently employed, using commercially available cloning systems (e.g., Lambda ZAP Express, Stratagene). Phage clones containing 238P1B2 gene cDNAs can be identified by probing with a labeled 238P1B2 cDNA or a fragment thereof. For example, a 238P1B2 cDNA (e.g., Figure 2) or a portion thereof can be synthesized and used as a probe to retrieve overlapping and full-length cDNAs corresponding to a 238P1B2 gene. A238P1B2 gene itself can be isolated by screening genomic DNA libraries, bacterial artificial chromosome libraries (BACs), yeast artificial chromosome libraries (YACs), and the like, with 238P1B2 DNA probes or primers.

### II.A.5.) Recombinant Nucleic Acid Molecules and Host-Vector Systems

Recombinant DNA or RNA molecules may containan 238P1B2 polynucleotide, a fragment, analog or homologue thereof, including but not limited to phages, plasmids, phagemids, cosmids, YACs, BACs, as well as various viral and non-viral vectors well known in the art, and cells transformed or transfected with such recombinant DNA or RNA molecules. Methods for generating such molecules are well known (see, for example, Sambrook *et al.,* 1989, supra).

A host-vector system may comprise a recombinant DNA molecule containing a 238P1B2 polynucleotide, fragment, analog or homologue thereof within a suitable prokaryotic or eukaryotic host cell. Examples of suitable eukaryotic host cells include a yeast cell, a plant cell, or an animal cell, such as a mammalian cell or an insect cell (e.g., a baculovirus-infectible cell such as an Sf9 or HighFive cell). Examples of suitable mammalian cells include various prostate cancer cell lines such as DU145 and TsuPr1, other transfectable or transducible prostate cancer cell lines, primary cells (PrEC), as well as a number of mammalian cells routinely used for the expression of recombinant proteins (e.g., COS, CHO, 293, 293T cells). More particularly, a polynucleotide comprising the coding sequence of 238P1B2 or a fragment, analog or homolog thereof can be used to generate 238P1B2 proteins or fragments thereof using any number of host-vector systems routinely used and widely known in the art.

A wide range of host-vector systems suitable for the expression of 238P1B2 proteins or fragments thereof are available, see for example, Sambrook *et al,* 1989, supra; Current Protocols in Molecular Biology, 1995, supra). Preferred vectors for mammalian expression include but are not limited to pcDNA 3.1 myc-His-tag (Invitrogen) and the retroviral vector pSRatkneo (Muller et al, 1991, MCB 11: 1785). Using these expression vectors, 238P1B2 can be expressed in several prostate cancer and non-prostate cell lines, including for example 293,293T, rat-1, NIH 3T3 and TsuPrl. These host-vector systems are useful for the production of a 238P1B2 protein or fragment thereof. Such host-vector systems can be employed to study the functional properties of 238P1B2 and 238P1B2 mutations or analogs.

Recombinant human 238P1B2 protein or an analog or homolog or fragment thereof can be produced by mammalian cells transfected with a construct encoding a 238P1B2-related nucleotide. For example, 293T cells can be transfected with an expression plasmid encoding 238P1B2 or fragment, analog or homolog thereof, a 238P1B2-related protein is expressed in the 293T cells, and the recombinant 238P1B2 protein is isolated using standard purification methods (e.g., affinity purification using anti-238P1B2 antibodies). A 238P1B2 coding sequence may be subcloned into the retroviral vector pSRaMSVtkneo and used to infect various mammalian cell lines, such as NIH 3T3, TsuPr1, 293 and rat-1 in order to establish 238P1B2 expressing cell lines. Various other expression systems well known in the art can also be employed. Expression constructs encoding a leader peptide joined in frame to a 238P1B2 coding sequence can be used for the generation of a secreted form of recombinant 238P1B2 protein.

As discussed herein, redundancy in the genetic code permits variation in 238P1B2 gene sequences. In particular, it is known in the art that specific host species often have specific codon preferences, and thus one can adapt the disclosed sequence as preferred for a desired host. For example, preferred analog codon sequences typically have rare codons (i.e., codons having a usage frequency of less than about 20% in known sequences of the desired host) replaced with higher frequency codons. Codon preferences for a specific species are calculated, for example, by utilizing codon usage tables available on the INTERNET such as at URL www.dna.affrc.gojp/- nakamura/codon.html.

Additional sequence modifications are known to enhance protein expression in a cellular host. These include elimination of sequences encoding spurious polyadenylation signals, exon/intron splice site signals, transposon-like repeats, and/or other such well-characterized sequences that are deleterious to gene expression. The GC content of the sequence is adjusted to levels average for a given cellular host, as calculated by reference to known genes expressed in the host cell. Where possible, the sequence is modified to avoid predicted hairpin secondary mRNA structures. Other useful modifications include the addition of a translational initiation consensus sequence at the start of the open reading frame, as described in Kozak, Mol. Cell Biol., 9: 5073-5080 (1989). Skilled artisans understand that the general rule that eukaryotic ribosomes initiate translation exclusively at the 5' proximal AUG codon is abrogated only under rare conditions (see, e.g., Kozak PNAS 92(7): 2662-2666, (1995) and Kozak NAR 15(20): 8125-8148 (1987)).

### III.) 238PIB2-related Proteins

We describe 238P1B2-related proteins. Specific 238P1B2 proteins comprise a polypeptide having all or part of the amino acid sequence of human 238P1B2 as shown in Figure 2 or Figure 3. Alternatively, 238P1B2 proteins comprise variant, homolog or analog polypeptides that have alterations in the amino acid sequence of 238P1B2 shown in Figure 2 or Figure 3.

In general, naturally occurring allelic variants of human 238P1B2 share a high degree of structural identity and homology (e.g., 90% or more homology). Typically, allelic variants of a 238P1B2 protein contain conservative amino acid substitutions within the 238P1B2 sequences described herein or contain a substitution of an amino acid from a corresponding position in a homologue of 238P1B2. One class of 238P1B2 allelic variants are proteins that share a high degree of homology with at least a small region of a particular 238P1B2 amino acid sequence, but further contain a radical departure from the sequence, such as a non-conservative substitution, truncation, insertion or frame shift. In comparisons of protein sequences, the terms, similarity, identity, and homology each have a distinct meaning as appreciated in the field of genetics. Moreover, orthology and paralogy can be important concepts describing the relationship of members of a given protein family in one organism to the members of the same family in other organisms.

Amino acid abbreviations are provided in Table II. Conservative amino acid substitutions can frequently be made in a protein without altering either the conformation or the function of the protein. Proteins described herein can comprise 1, 2, 3, 4, 5, 6, 7, 8, 9, 10, 11, 12, 13, 14, 15 conservative substitutions. Such changes include substituting any of isoleucine (I), valine (V), and leucine (L) for any other of these hydrophobic amino acids; aspartic acid (D) for glutamic acid (E) and vice versa; glutamine (Q) for asparagine (N) and vice versa; and serine (S) for threonine (T) and vice versa. Other substitutions can also be considered conservative, depending on the environment of the particular amino acid and its role in the three-dimensional structure of the protein. For example, glycine (G) and alanine (A) can frequently be interchangeable, as can alanine (A) and valine (V). Methionine (M), which is relatively hydrophobic, can frequently be interchanged with leucine and isoleucine, and sometimes with valine. Lysine (K) and arginine (R) are frequently interchangeable in locations in which the significant feature of the amino acid residue is its charge and the differing pK's of these two amino acid residues are not significant. Still other changes can be considered "conservative" in particular environments (see, e.g. Table III herein; pages 13-15 "Biochemistry" 2ndd ED. Lubert Stryer ed (Stanford University); Henikoffet et al., PNAS 1992 Vol 89 10915-10919; Lei et al., J Biol Chem 1995 May 19; 270 (20): 11882-6).

We describe a wide variety of art-accepted variants or analogs of 238P1B2 proteins such as polypeptides having amino acid insertions, deletions and substitutions. 238P1B2 variants can be made using methods known in the art such as site-directed mutagenesis, alanine scanning, and PCR mutagenesis. Site-directed mutagenesis (Carter et al., Nucl. Acids Res., 13: 4331 (1986); Zoller et al., Nucl. Acids Res., 10: 6487 (1987)), cassette mutagenesis (Wells et al., Gene, 34: 315 (1985)), restriction selection mutagenesis (Wells et al., Philos. Trans. R. Soc. London SerA, 317: 415 (1986)) or other known techniques can be performed on the cloned DNA to produce the 238P1B2 variant DNA.

Scanning amino acid analysis can also be employed to identify one or more amino acids along a contiguous sequence that is involved in a specific biological activity such as a protein-protein interaction. Among the preferred scanning amino acids are relatively small, neutral amino acids. Such amino acids include alanine, glycine, serine, and cysteine. Alanine is typically a preferred scanning amino acid among this group because it eliminates the side-chain beyond the beta-carbon and is less likely to alter the main- chain conformation of the variant. Alanine is also typically preferred because it is the most common amino acid. Further, it is frequently found in both buried and exposed positions (Creighton, The Proteins, (W. H. Freeman & Co., N. Y.); Chothia, J. Mol. Biol., 150:1 (1976)). If alanine substitution does not yield adequate amounts of variant, an isosteric amino acid can be used.

As defined herein, 238P1B2 variants, analogs or homologs, have the distinguishing attribute of having at least one epitope that is "cross reactive" with a 238P1B2 protein having an amino acid sequence of Figure 3. As used in this sentence,"cross reactive"means that an antibody or T cell that specifically binds to an 238P1B2 variant also specifically binds to a 238P1B2 protein having an amino acid sequence set forth in Figure 3. A polypeptide ceases to be a variant of a protein shown in Figure 3, when it no longer contains any epitope capable of being recognized by an antibody or T cell that specifically binds to the starting 238P1B2 protein. Those skilled in the art understand that antibodies that recognize proteins bind to epitopes of varying size, and a grouping of the order of about four or five amino acids, contiguous or not, is regarded as a typical number of amino acids in a minimal epitope. See, e. g. , Nair et al., J. Immunol 2000 165 (12): 6949-6955; Hebbes et al., Mol Immunol (1989) 26 (9): 865-73; Schwartz et aL, J Immunol (1985) 135 (4): 2598-608.

Other classes of 238P1B2-related protein variants share 70%, 75%, 80%, 85% or 90% or more similarity with an amino acid sequence of Figure 3, or a fragment thereof. Another specific class of 238P1B2 protein variants or analogs comprise one or more of the 238P1B2 biological motifs described herein or presently known in the art. Thus, analogs of 238P1B2 fragments (nucleic or amino acid) may have altered functional (e.g. immunogenic) properties relative to the starting fragment. It is to be appreciated that motifs now or which become part of the art are to be applied to the nucleic or amino acid sequences of Figure 2 or Figure 3.

As discussed herein, polypeptides may contain less than the full amino acid sequence of a 238P1B2 protein shown in Figure 2 or Figure 3. For example, representative peptides/proteins may have any 4, 5, 6, 7, 8, 9, 10, 11, 12, 13, 14, 15 or more contiguous amino acids of a 238P1B2 protein shown in Figure 2 or Figure 3.

Moreover, representative polypeptides may consist of about amino acid 1 to about amino acid 10 of a 238P1B2 protein shown in Figure 2 or Figure 3, polypeptides consisting of about amino acid 10 to about amino acid 20 of a 238P1B2 protein shown in Figure 2 or Figure 3, polypeptides consisting of about amino acid 20 to about amino acid 30 of a 238P1B2 protein shown in Figure 2 or Figure 3, polypeptides consisting of about amino acid 30 to about amino acid 40 of a 238P1B2 protein shown in Figure 2 or Figure 3, polypeptides consisting of about amino acid 40 to about amino acid 50 of a 238P1B2 protein shown in Figure 2 or Figure 3, polypeptides consisting of about amino acid 50 to about amino acid 60 of a 238P1B2 protein shown in Figure 2 or Figure 3, polypeptides consisting of about amino acid 60 to about amino acid 70 of a 238P1B2 protein shown in Figure 2 or Figure 3, polypeptides consisting of about amino acid 70 to about amino acid 80 of a 238P1B2 protein shown in Figure 2 or Figure 3, polypeptides consisting of about amino acid 80 to about amino acid 90 of a 238P1B2 protein shown in Figure 2 or Figure 3, polypeptides consisting of about amino acid 90 to about amino acid 100 of a 238P1B2 protein shown in Figure 2 or Figure 3, etc. throughout the entirety of a 238P1B2 amino acid sequence. Moreover, polypeptides consisting of about amino acid 1 (or 20 or 30 or 40 etc.) to about amino acid 20, (or 130, or 140 or 150 etc.) of a 238P1B2 protein shown in Figure 2 or Figure 3. It is to be appreciated that the starting and stopping positions in this paragraph refer to the specified position as well as that position plus or minus 5 residues.

238P1B2-related proteins are generated using standard peptide synthesis technology or using chemical cleavage methods well known in the art. Alternatively, recombinant methods can be used to generate nucleic acid molecules that encode a 238P1B2-related protein. Nucleic acid molecules may provide a means to generate defined fragments of a 238P1B2 protein (or variants, homologs or analogs thereof).

### III.A.) Motif-bearine Proteins

Additional 238P1B2 polypeptides comprise the amino acid residues of one or more of the biological motifs contained within a 238P1B2 polypeptide sequence set forth in Figure 2 or Figure 3. Various motifs are known in the art, and a protein can be evaluated for the presence of such motifs by a number of publicly available Internet sites (see, e. g., World Wide Web URL addresses: pfam.wustl.edu/; searchlauncher.bcm.tmc.edu/seq-search/struc-predict.html; psort.ims.u-tokyo.ac. jp/; www.cbs.dtu.dk/; www.ebi.ac.uk/interpro/scan.html; www.expasy.ch/tools/scnpsiti.html; Epimatrix™ and Epimer™, Brown University, www.brown.edu/Research/TB-HIVLab/epimatrix-/epimatrix.html; and BIMAS, bimas.dcrt.nih.gov/.).

A variety of references reflect the art regarding the identification and generation of epitopes in a protein of interest as well as analogs thereof. See, for example, WO 9733602 to Chesnut et al; Sette, Immunogenetics 1999 50(3-4): 201-212; Sette et al., J. Immunol. 2001 166 (2): 1389-1397; Sidney et al., Hum. Immunol. 1997 58(1): 12-20; Kondo et al., Immunogenetics 1997 45(4): 249-258; Sidney et al., J. Immunol. 1996 157(8): 3480-90; and Falk et al., Nature 351: 290-6 (1991); Hunt et al., Science 255: 1261-3 (1992); Parker et al, J. Immunol. 149: 3580-7 (1992); Parker et al., J. Immunol. 152: 163-75 (1994)); Kast *et al.,* 1994 152 (8): 3904-12; Borras-Cuesta et al., Hum. Immunol. 2000 61(3): 266-278; Alexander et al., J. Immunol. 2000 164(3); 164 (3): 1625-1633; Alexander *et al.*, PMID: 7895164, UI: 95202582; O'Sullivan et al., J. Immunol. 1991 147(8): 2663-2669; Alexander et al., Immunity 1994 1(9): 751-761 and Alexander et al., Immunol. Res. 1998 18(2): 79-92.

238P1B2-related proteins may be in many forms, preferably in isolated form. A purified 238P1B2 protein molecule will be substantially free of other proteins or molecules that impair the binding of 238P1B2 to antibody, T cell or other ligand. The nature and degree of isolation and purification will depend on the intended use. 238P1B2-related proteins include purified 238P1B2- related proteins and functional, soluble 238P1B2-related proteins. A functional, soluble 238P1B2 protein or fragment thereof may retain the ability to be bound by antibody, T cell or other ligand.

238P1B2 proteins may comprise biologically active fragments of a 238P1B2 amino acid sequence shown in Figure 2 or Figure 3. Such proteins exhibit properties of the starting 238P1B2 protein, such as the ability to elicit the generation of antibodies that specifically bind an epitope associated with the starting 238P1B2 protein; to be bound by such antibodies; to elicit the activation of HTL or CTL; and/or, to be recognized by HTL or CTL that also specifically bind to the starting protein.

238P1B2-related polypeptides that contain particularly interesting structures can be predicted and/or identified using various analytical techniques well known in the art, including, for example, the methods of Chou- Fasman, Gamier-Robson, Kyte-Doolittle, Eisenberg, Karplus-Schultz or Jameson-Wolf analysis, or on the basis of immunogenicity. Fragments that contain such structures are particularly useful in generating subunit-specific anti-238P1B2 antibodies, or T cells or in identifying cellular factors that bind to 238P1B2. For example, hydrophilicity profiles can be generated, and immunogenic peptide fragments identified, using the method of Hopp, T. P. and Woods, K. R., 1981, Proc. Natl. Acad. Sci. U.S.A. 78: 3824-3828. Hydropathicity profiles can be generated, and immunogenic peptide fragments identified, using the method of Kyte, J, and Doolittle, R. F., 1982, J. Mol. Biol. 157: 105-132. Percent (%) Accessible Residues profiles can be generated, and immunogenic peptide fragments identified, using the method of Janin J., 1979, Nature 277: 491-492. Average Flexibility profiles can be generated, and immunogenic peptide fragments identified, using the method of Bhaskaran R., Ponnuswamy P. K., 1988, Int. J. Pept. Protein Res. 32: 242-255. Beta-turn profiles can be generated, and immunogenic peptide fragments identified, using the method of Deleage, G. , Roux B. , 1987, Protein Engineering 1: 289-294.

CTL epitopes can be determined using specific algorithms to identify peptides within an 238P1B2 protein that are capable of optimally binding to specified HLA alleles (e.g., by using the SYFPEITHI site at World Wide Web URL syfpeithi.bmi-heidelberg.com/; Epimatrix™ and Eimer™, Brown University, URL (www.brown.edu/Research/TB-HIV Lab/epirnatrix/epirnatrix.htrnl); and BIMAS, URL bimas.dcrt.nih.gov/).

The HLA peptide motif search algorithm was developed by Dr. Ken Parker based on binding of specific peptide sequences in the groove of HLA Class I molecules, in particular HLA-A2 (see, e.g., Falk et al., Nature 351: 290-6(1991); Hunt et al., Science 255: 1261-3 (1992); Parker et al., J. Immunol. 149: 3580-7 (1992); Parker et al., J. Immunol. 152: 163-75 (1994)). This algorithm allows location and ranking of 8-mer, 9-mer, and 10-mer peptides from a complete protein sequence for predicted binding to HLA-A2 as well as numerous other HLA Class I molecules. Many HLA class I binding peptides are 8-, 9-, 10 or 11-mers. For example, for class I HLA-A2, the epitopes preferably contain a leucine (L) or methionine (M) at position 2 and a valine (V) or leucine (L) at the C-terminus (see, e.g., Parker et al., J. Immunol. 149: 3580-7 (1992)).
The binding score corresponds to the estimated half time of dissociation of complexes containing the peptide at 37°C at pH 6.5. Peptides with the highest binding score are predicted to be the most tightly bound to HLA Class I on the cell surface for the greatest period of time and thus represent the best immunogenic targets for T-cell recognition.

Actual binding of peptides to an HLA allele can be evaluated by stabilization of HLA expression on the antigen-processing defective cell line T2 (see, e.g., Xue et al., Prostate 30: 73-8 (1997) and Peshwa et al., Prostate 36: 129-38 (1998)). Immunogenicity of specific peptides can be evaluated in vitro by stimulation of CD8+ cytotoxic T lymphocytes (CTL) in the presence of antigen presenting cells such as dendritic cells.

It is to be appreciated that every epitope predicted by the BIMAS site, Epimer™ and Epimatrix™ sites, or specified by the HLA class I or class II motifs available in the art or which become part of the art such as set forth in Table IV (or determined using World Wide Web site URL syfpeithi.bmi-heidelberg.com/, or BIMAS, bimas.dcrt.nih.gov/) are to be "applied" to a 238P1B2 protein described herein. As used in this context "applied" means that a 238P1B2 protein is evaluated, e.g., visually or by computer- based patterns finding methods, as appreciated by those of skill in the relevant art. Every subsequence of a 238P1B2 protein of 8, 9, 10 or 11 amino acid residues that bears an HLA Class I motif, or a subsequence of 9 or more amino acid residues that bear an HLA Class II motif are described herein.

### III. B.) Expression of 238P1B2-related Proteins

As described in the examples that follow, 238P1B2 can be conveniently expressed in cells (such as 293T cells) transfected with a commercially available expression vector such as a CMV- driven expression vector encoding 238P1B2 with a C-terminal 6XHis and MYC tag (pcDNA3.1/mycHIS, Invitrogen or Tag5, GenHunter Corporation, Nashville TN). The Tag5 vector provides an IgGK secretion signal that can be used to facilitate the production of a secreted 238P 1 B2 protein in transfected cells. The secreted HIS- tagged 238P1B2 in the culture media can be purified, e.g., using a nickel column using standard techniques.

### III.C.) Modifications of 238P1B2-related Proteins

Modifications of 238P1B2-related proteins such as covalent modifications are described herein. One type of covalent modification includes reacting targeted amino acid residues of a 238P1B2 polypeptide with an organic derivatizing agent that is capable of reacting with selected side chains or the N-or C-terminal residues of a 238P1B2 protein. Another type of covalent modification of a 238P1B2 polypeptide described herein comprises altering the native glycosylation pattern of a protein described herein. Another type of covalent modification of 238P1B2 comprises linking a 238P1B2 polypeptide to one of a variety of nonproteinaceous polymers, e.g., polyethylene glycol (PEG), polypropylene glycol, or polyoxyalkylenes, in the manner set forth in U.S. Patent Nos. 4,640,835; 4,496,689; 4,301,144; 4,670,417; 4,791,192 or 4,179,337.

The 238P1B2-related proteins described herein can also be modified to form a chimeric molecule comprising 238P1B2 fused to another, heterologous polypeptide or amino acid sequence. Such a chimeric molecule can be synthesized chemically or recombinantly. A chimeric molecule can have a protein described herein fused to another tumor-associated antigen or fragment thereof. Alternatively, a protein described herein can comprise a fusion of fragments of a 238P1B2 sequence (amino or nucleic acid) such that a molecule is created that is not, through its length, directly homologous to the amino or nucleic acid sequences shown in Figure 2 or Figure 3. Such a chimeric molecule can comprise multiples of the same subsequence of 238P1B2. A chimeric molecule can comprise a fusion of a 238P1B2-related protein with a polyhistidine epitope tag, which provides an epitope to which immobilized nickel can selectively bind, with cytokines or with growth factors. The epitope tag is generally placed at the amino-or carboxyl- terminus of a 238P1B2 protein. Alternatively, the chimeric molecule can comprise a fusion of a 238P1B2-related protein with an immunoglobulin or a particular region of an immunoglobulin. For a bivalent form of the chimeric molecule (also referred to as an "immunoadhesin"), such a fusion could be to the Fc region of an IgG molecule. The Ig fusions preferably include the substitution of a soluble (transmembrane domain deleted or inactivated) form of a 238P1B2 polypeptide in place of at least one variable region within an Ig molecule. The immunoglobulin fusion may includs the hinge, CH2 and CH3, or the hinge, CHI, CH2 and CH3 regions of an IgGI molecule. For the production of immunoglobulin fusions see, e. g., U.S. Patent No. 5,428,130 issued June 27,1995.

### IILD.) Uses of 238P1B2-related Proteins

The proteins described herein have a number of different specific uses. As 238P1B2 is highly expressed in prostate and other cancers, 238P1B2-related proteins are used in methods that assess the status of 238P1B2 gene products in normal versus cancerous tissues, thereby elucidating the malignant phenotype. Typically, polypeptides from specific regions of a 238P1B2 protein are used to assess the presence of perturbations (such as deletions, insertions, point mutations etc.) in those regions (such as regions containing one or more motifs). Exemplary assays utilize antibodies or T cells targeting 238P1B2-related proteins comprising the amino acid residues of one or more of the biological motifs contained within a 238P1B2 polypeptide sequence in order to evaluate the characteristics of this region in normal versus cancerous tissues or to elicit an immune response to the epitope. Alternatively, 238P1B2-related proteins that contain the amino acid residues of one or more of the biological motifs in a 238P1B2 protein are used to screen for factors that interact with that region of 238P1B2.

238P1B2 protein fragments/subsequences are particularly useful in generating and characterizing domain-specific antibodies (e.g. , antibodies recognizing an extracellular or intracellular epitope of an 238P1B2 protein), for identifying agents or cellular factors that bind to 238P1B2 or a particular structural domain thereof, and in various therapeutic and diagnostic contexts, including but not limited to diagnostic assays, cancer vaccines and methods of preparing such vaccines.

Proteins encoded by the 238P1B2 genes, or by analogs, homologs or fragments thereof, have a variety of uses, including but not limited to generating antibodies and in methods for identifying ligands and other agents and cellular constituents that bind to an 238P1B2 gene product. Antibodies raised against an 238P1B2 protein or fragment thereof are useful in diagnostic and prognostic assays, and imaging methodologies in the management of human cancers characterized by expression of 238P1 B2 protein, such as those listed in Table I. Such antibodies can be expressed intracellularly and used in methods of treating patients with such cancers. 238P1B2-related nucleic acids or proteins are also used in generating HTL or CTL responses.

Various immunological assays useful for the detection of 238P1B2 proteins are used, including but not limited to various types of radioimmunoassays, enzyme-linked immunosorbent assays (ELISA), enzyme-linked immunofluorescent assays (ELISA), immunocytochemical methods, and the like. Antibodies can be labeled and used as immunological imaging reagents capable of detecting 238P1B2-expressing cells (e. g. , in radioscintigraphic imaging methods). 238P1B2 proteins are also particularly useful in generating cancer vaccines, as further described herein.

### IV.) Methods for the Detection of 238P1B2

We describe methods for detecting 238P1B2 polynucleotides and 238P1B2-related proteins, as well as methods for identifying a cell that expresses 238P1B2. The expression profile of 238P1B2 makes it a diagnostic marker for metastasized disease. Accordingly, the status of 238P1B2 gene products provides information useful for predicting a variety of factors including susceptibility to advanced stage disease, rate of progression, and/or tumor aggressiveness. As discussed in detail herein, the status of 238P1B2 gene products in patient samples can be analyzed by a variety protocols that are well known in the art including immunohistochemical analysis, the variety of Northern blotting techniques including in situ hybridization, RT-PCR analysis (for example on laser capture micro-dissected samples), Western blot analysis and tissue array analysis.

More particularly, we describe assays for the detection of 238P1B2 polynucleotides in a biological sample, such as serum, bone, prostate, and other tissues, urine, semen, cell preparations, and the like. Detectable 238P1B2 polynucleotides include, for example, a 238P1B2 gene or fragment thereof, 238P1B2 mRNA, alternative splice variant 238P1B2 mRNAs, and recombinant DNA or RNA molecules that contain a 238P1B2 polynucleotide. A number of methods for amplifying and/or detecting the presence of 238P1B2 polynucleotides are well known in the art and can be employed in the practice of these assays.

A method for detecting an 238P1B2 mRNA in a biological sample may comprise producing cDNA from the sample by reverse transcription using at least one primer; amplifying the cDNA so produced using an 238P1B2 polynucleotides as sense and antisense primers to amplify 238P1B2 cDNAs therein; and detecting the presence of the amplified 238P1B2 cDNA. Optionally, the sequence of the amplified 238P1B2 cDNA can be determined.

A method of detecting a 238P1B2 gene in a biological sample may comprise first isolating genomic DNA from the sample; amplifying the isolated genomic DNA using 238P1B2 polynucleotides as sense and antisense primers; and detecting the presence of the amplified 238P1B2 gene. Any number of appropriate sense and antisense probe combinations can be designed from a 238P1B2 nucleotide sequence (see, e.g., Figure 2) and used for this purpose.

We also describe assays for detecting the presence of an 238P1B2 protein in a tissue or other biological sample such as serum, semen, bone, prostate, urine, cell preparations, and the like. Methods for detecting a 238P1B2-related protein are also well known and include, for example, immunoprecipitation, immunohistochemical analysis, Western blot analysis, molecular binding assays, ELISA, ELIFA and the like. For example, a method of detecting the presence of a 238P1B2- related protein in a biological sample comprises first contacting the sample with a 238P1B2 antibody, a 238P1 B2-reactive fragment thereof, or a recombinant protein containing an antigen binding region of a 238P1B2 antibody; and then detecting the binding of 238P1B2-related protein in the sample.

Methods for identifying a cell that expresses 238P1B2 are also described. An assay for identifying a cell that expresses a 238P1B2 gene may comprise detecting the presence of 238P1B2 mRNA in the cell. Methods for the detection of particular mRNAs in cells are well known and include, for example, hybridization assays using complementary DNA probes (such as in situ hybridization using labeled 238P1B2 riboprobes, Northern blot and related techniques) and various nucleic acid amplification assays (such as RT-PCR using complementary primers specific for 238P1B2, and other amplification type detection methods, such as, for example, branched DNA, SISBA, TMA and the like). Alternatively, an assay for identifying a cell that expresses a 238P1B2 gene comprises detecting the presence of 238P1B2-related protein in the cell or secreted by the cell. Various methods for the detection of proteins are well known in the art and are employed for the detection of 238P1B2-related proteins and cells that express 238P1B2-related proteins.

238P1B2 expression analysis is also useful as a tool for identifying and evaluating agents that modulate 238P1B2 gene expression. For example, 238P1B2 expression is significantly upregulated in prostate cancer, and is expressed in cancers of the tissues listed in Table 1. Identification of a molecule or biological agent that inhibits 238P1B2 expression or over-expression in cancer cells is of therapeutic value. For example, such an agent can be identified by using a screen that quantifies 238P1B2 expression by RT-PCR, nucleic acid hybridization or antibody binding.

### V.) Methods for Monitoring the Status of 238P1B2-related Genes and Their Products

Oncogenesis is known to be a multistep process where cellular growth becomes progressively dysregulated and cells progress from a normal physiological state to precancerous and then cancerous states (see, e.g., Alers et al., Lab Invest. 77(5):437-438 (1997) and Isaacs et al., Cancer Surv. 23:19-32 (1995)). In this context, examining a biological sample for evidence of dysregulated cell growth (such as aberrant 238P1B2 expression in cancers) allows for early detection of such aberrant physiology, before a pathologic state such as cancer has progressed to a stage that therapeutic options are more limited and or the prognosis is worse. In such examinations, the status of 238P1B2 in a biological sample of interest can be compared, for example, to the status of 238P1B2 in a corresponding normal sample (e. g. a sample from that individual or alternatively another individual that is not affected by a pathology). An alteration in the status of 238P1B2 in the biological sample (as compared to the normal sample) provides evidence of dysregulated cellular growth. In addition to using a biological sample that is not affected by a pathology as a normal sample, one can also use a predetermined normative value such as a predetermined normal level of mRNA expression (see, e.g., Grever et al., J. Comp. Neurol. 1996 Dec 9; 376(2):306-14 and U.S. Patent No. 5,837,501) to compare 238P1B2 status in a sample.

The term "status" in this context is used according to its art accepted meaning and refers to the condition or state of a gene and its products. Typically, skilled artisans use a number of parameters to evaluate the condition or state of a gene and its products. These include, but are not limited to the location of expressed gene products (including the location of 238P1B2 expressing cells) as well as the level, and biological activity of expressed gene products (such as 238P1B2 mRNA, polynucleotides and polypeptides). Typically, an alteration in the status of 238P1B2 comprises a change in the location of 238P1B2 and/or 238PIB2 expressing cells and/or an increase in 238P1B2 mRNA and/or protein expression.

238P1B2 status in a sample can be analyzed by a number of means well known in the art, including without limitation, immunohistochemical analysis, in situ hybridization, RT-PCR analysis on laser capture micro- dissected samples, Western blot analysis, and tissue array analysis. Typical protocols for evaluating the status of a 238P1B2 gene and gene products are found, for example in Ausubel et al. eds., 1995, Current Protocols In Molecular Biology, Units 2 (Northern Blotting), 4 (Southern Blotting), 15 (Immunoblotting) and 18 (PCR Analysis). Thus, the status of 238PIB2 in a biological sample is evaluated by various methods utilized by skilled artisans including, but not limited to genomic Southern analysis (to examine, for example perturbations in a 238P1B2 gene), Northern analysis and/or PCR analysis of 238P1B2 mRNA (to examine, for example alterations in the polynucleotide sequences or expression levels of 238P1B2 mRNAs), and, Western and/or immunohistochemical analysis (to examine, for example alterations in polypeptide sequences, alterations in polypeptide localization within a sample, alterations in expression levels of 238P1B2 proteins and/or associations of 238P1B2 proteins with polypeptide binding partners). Detectable 238P1B2 polynucleotides include, for example, a 238P1B2 gene or fragment thereof, 238P1B2 mRNA, alternative splice variants, 238P1B2 mRNAs, and recombinant DNA or RNA molecules containing a 238P1B2 polynucleotide.

The expression profile of 238P1B2 makes it a diagnostic marker for local and/or metastasized disease, and provides information on the growth or oncogenic potential of a biological sample. In particular, the status of 238P1B2 provides information useful for predicting susceptibility to particular disease stages, progression, and/or tumor aggressiveness. We describe methods and assays for determining 238P1B2 status and diagnosing cancers that express 238P1B2, such as cancers of the tissues listed in Table I. For example, because 238P1B2 mRNA is so highly expressed in prostate and other cancers relative to normal prostate tissue, assays that evaluate the levels of 238P1B2 mRNA transcripts or proteins in a biological sample can be used to diagnose a disease associated with 238P1B2 dysregulation, and can provide prognostic information useful in defining appropriate therapeutic options.

The expression status of 238P1B2 provides information including the presence, stage and location of dysplastic, precancerous and cancerous cells, predicting susceptibility to various stages of disease, and/or for gauging tumor aggressiveness. Moreover, the expression profile makes it useful as an imaging reagent for metastasized disease. Consequently, we describe various molecular prognostic and diagnostic methods for examining the status of 238PIB2 in biological samples such as those from individuals suffering from, or suspected of suffering from a pathology characterized by dysregulated cellular growth, such as cancer.

As described above, the status of 238P1B2 in a biological sample can be examined by a number of well-known procedures in the art. For example, the status of 238P1B2 in a biological sample taken from a specific location in the body can be examined by evaluating the sample for the presence or absence of 238P1B2 expressing cells (e.g. those that express 238P1B2 mRNAs or proteins). This examination can provide evidence of dysregulated cellular growth, for example, when 238P1B2-expressing cells are found in a biological sample that does not normally contain such cells (such as a lymph node), because such alterations in the status of 238P1B2 in a biological sample are often associated with dysregulated cellular growth. Specifically, one indicator of dysregulated cellular growth is the metastases of cancer cells from an organ of origin (such as the prostate) to a different area of the body (such as a lymph node). In this context, evidence of dysregulated cellular growth is important for example because occult lymph node metastases can be detected in a substantial proportion of patients with prostate cancer, and such metastases are associated with known predictors of disease progression (see, e.g., Murphy et al., Prostate 42(4):315-317 (2000); Su et al., Semin. Surg. Oncol. 18(1):17-28 (2000) and Freeman et al., J Urol 1995 Aug 154 (2 Pt 1):474-8).

We describe methods for monitoring 238P1B2 gene products by determining the status of 238P1B2 gene products expressed by cells from an individual suspected of having a disease associated with dysregulated cell growth (such as hyperplasia or cancer) and then comparing the status so determined to the status of 238P1B2 gene products in a corresponding normal sample. The presence of aberrant 238P1B2 gene products in the test sample relative to the normal sample provides an indication of the presence of dysregulated cell growth within the cells of the individual.

We describe assays useful in determining the presence of cancer in an individual, comprising detecting a significant increase in 238P1B2 mRNA or protein expression in a test cell or tissue sample relative to expression levels in the corresponding normal cell or tissue. The presence of 238P1B2 mRNA can, for example, be evaluated in tissue samples including but not limited to those listed in Table I. The presence of significant 238P1B2 expression in any of these tissues is useful to indicate the emergence, presence and/or severity of a cancer, since the corresponding normal tissues do not express 238P1B2 mRNA or express it at lower levels.

238P1B2 status may be determined at the protein level rather than at the nucleic acid level. For example, such a method comprises determining the level of 238P1B2 protein expressed by cells in a test tissue sample and comparing the level so determined to the level of 238P1B2 expressed in a corresponding normal sample. The presence of 238P1B2 protein may be evaluated, for example, using immunohistochemical methods. 238P1B2 antibodies or binding partners capable of detecting 238P1B2 protein expression are used in a variety of assay formats well known in the art for this purpose.

One can evaluate the status of 238P1B2 nucleotide and amino acid sequences in a biological sample in order to identify perturbations in the structure of these molecules. These perturbations can include insertions, deletions, substitutions and the like. Such evaluations are useful because perturbations in the nucleotide and amino acid sequences are observed in a large number of proteins associated with a growth dysregulated phenotype (see, e.g., Marrogi et al., 1999, J. Cutan. Pathol. 26(8):369-378). For example, a mutation in the sequence of 238P1B2 may be indicative of the presence or promotion of a tumor. Such assays therefore have diagnostic and predictive value where a mutation in 238P1B2 indicates a potential loss of function or increase in tumor growth.

A wide variety of assays for observing perturbations in nucleotide and amino acid sequences are well known in the art. For example, the size and structure of nucleic acid or amino acid sequences of 238P1B2 gene products are observed by the Northern, Southern, Western, PCR and DNA sequencing protocols discussed herein. In addition, other methods for observing perturbations in nucleotide and amino acid sequences such as single strand conformation polymorphism analysis are well known in the art (see, e.g., U.S. Patent Nos. 5,382,510 issued 7 September 1999, and 5,952,170 issued 17 January 1995).

Additionally, one can examine the methylation status of a 238P1B2 gene in a biological sample. Aberrant demethylation and/or hypermethylation of CpG islands in gene 5' regulatory regions frequently occurs in immortalized and transformed cells, and can result in altered expression of various genes. For example, promoter hypermethylation of the pi-class glutathione S-transferase (a protein expressed in normal prostate but not expressed in >90% of prostate carcinomas) appears to permanently silence transcription of this gene and is the most frequently detected genomic alteration in prostate carcinomas (De Marzo et al., Am. J. Pathol. 155(6):1985-1992 (1999)). In addition, this alteration is present in at least 70% of cases of high-grade prostatic intraepithelial neoplasia (PIN) (Brooks et al., Cancer Epidemiol. Biomarkers Prev., 1998,7:531-536). In another example, expression of the LAGE-I tumor specific gene (which is not expressed in normal prostate but is expressed in 25-50% of prostate cancers) is induced by deoxy-azacytidine in lymphoblastoid cells, suggesting that tumoral expression is due to demethylation (Lethe et al., Int J. Cancer 76 (6):903-908 (1998)). A variety of assays for examining methylation status of a gene are well known in the art. For example, one can utilize, in Southern hybridization approaches, methylation-sensitive restriction enzymes that cannot cleave sequences that contain methylated CpG sites to assess the methylation status of CpG islands. In addition, MSP (methylation specific PCR) can rapidly profile the methylation status of all the CpG sites present in a CpG island of a given gene. This procedure involves initial modification of DNA by sodium bisulfite (which will convert all unmethylated cytosines to uracil) followed by amplification using primers specific for methylated versus unmethylated DNA. Protocols involving methylation interference can also be found for example in Current Protocols In Molecular Biology, Unit 12, Frederick M. Ausubel *et al.* eds., 1995.

Gene amplification is an additional method for assessing the status of 238P1B2. Gene amplification is measured in a sample directly, for example, by conventional Southern blotting or Northern blotting to quantitate the transcription ofmRNA (Thomas, 1980, Proc. Natl. Acad. Sci. USA, 77:5201-5205), dot blotting (DNA analysis), or *in situ* hybridization, using an appropriately labeled probe, based on the sequences provided herein. Alternatively, antibodies are employed that recognize specific duplexes, including DNA duplexes, RNA duplexes, and DNA-RNA hybrid duplexes or DNA-protein duplexes. The antibodies in turn are labeled and the assay carried out where the duplex is bound to a surface, so that upon the formation of duplex on the surface, the presence of antibody bound to the duplex can be detected.

Biopsied tissue or peripheral blood can be conveniently assayed for the presence of cancer cells using for example, Northern, dot blot or RT-PCR analysis to detect 238P1B2 expression. The presence of RT-PCR amplifiable 238P1B2 mRNA provides an indication of the presence of cancer. RT-PCR assays are well known in the art. RT-PCR detection assays for tumor cells in peripheral blood are currently being evaluated for use in the diagnosis and management of a number of human solid tumors. In the prostate cancer field, these include RT- PCR assays for the detection of cells expressing PSA and PSM (Verkaik et al, 1997, Urol. Res. 25:373-384; Ghossein et al., 1995, J. Clin. Oncol. 13:1195-2000; Heston et al., 1995, Clin. Chem. 41:1687-1688).

We describe an assessment of the susceptibility that an individual has for developing cancer. We describe a method for predicting susceptibility to cancer comprising detecting 238P1B2 mRNA or 238P1B2 protein in a tissue sample, its presence indicating susceptibility to cancer, wherein the degree of 238P1B2 mRNA expression correlates to the degree of susceptibility. The presence of 238P1B2 in prostate or other tissue may be examined, with the presence of 238P1B2 in the sample providing an indication of prostate cancer susceptibility (or the emergence or existence of a prostate tumor). Similarly, one can evaluate the integrity 238P1B2 nucleotide and amino acid sequences in a biological sample, in order to identify perturbations in the structure of these molecules such as insertions, deletions, substitutions and the like. The presence of one or more perturbations in 238P1B2 gene products in the sample is an indication of cancer susceptibility (or the emergence or existence of a tumor).

We describe methods for gauging tumor aggressiveness. A method for gauging aggressiveness of a tumor may comprise determining the level of 238P1B2 mRNA or 238P1B2 protein expressed by tumor cells, comparing the level so determined to the level of 238P1B2 mRNA or 238P1B2 protein expressed in a corresponding normal tissue taken from the same individual or a normal tissue reference sample, wherein the degree of 238P1B2 mRNA or 238P1B2 protein expression in the tumor sample relative to the normal sample indicates the degree of aggressiveness. Aggressiveness of a tumor may be evaluated by determining the extent to which 238P1B2 is expressed in the tumor cells, with higher expression levels indicating more aggressive tumors. We describe the evaluation of the integrity of 238P1B2 nucleotide and amino acid sequences in a biological sample, in order to identify perturbations in the structure of these molecules such as insertions, deletions, substitutions and the like. The presence of one or more perturbations indicates more aggressive tumors.

We describe methods for observing the progression of a malignancy in an individual over time. Methods for observing the progression of a malignancy in an individual over time may comprise determining the level of 238P1B2 mRNA or 238P1B2 protein expressed by cells in a sample of the tumor, comparing the level so determined to the level of 238P1B2 mRNA or 238P1B2 protein expressed in an equivalent tissue sample taken from the same individual at a different time, wherein the degree of 238P1B2 mRNA or 238P1B2 protein expression in the tumor sample over time provides information on the progression of the cancer. The progression of a cancer may be evaluated by determining 238P1B2 expression in the tumor cells over time, where increased expression over time indicates a progression of the cancer. Also, one can evaluate the integrity 238P1B2 nucleotide and amino acid sequences in a biological sample in order to identify perturbations in the structure of these molecules such as insertions, deletions, substitutions and the like, where the presence of one or more perturbations indicates a progression of the cancer.

The above diagnostic approaches can be combined with any one of a wide variety of prognostic and diagnostic protocols known in the art. For example, we describe methods for observing a coincidence between the expression of 238P1B2 gene and 238P1B2 gene products (or perturbations in 238P1B2 gene and 238P1B2 gene products) and a factor that is associated with malignancy, as a means for diagnosing and prognosticating the status of a tissue sample. A wide variety of factors associated with malignancy can be utilized, such as the expression of genes associated with malignancy (e.g. PSA, PSCA and PSM expression for prostate cancer etc.) as well as gross cytological observations (see, e.g., Bocking et al., 1984, Anal. Quant. Cytol. 6(2):74-88; Epstein, 1995, Hum. Pathol. 26(2):223-9; Thorson et al., 1998, Mod. Pathol. 11(6):543-51; Baisden et al., 1999, Am. J. Surg. Pathol. 23(8):918-24). Methods for observing a coincidence between the expression of 238P1B2 gene and 238P1B2 gene products (or perturbations in 238P1B2 gene and 238P1B2 gene products) and another factor that is associated with malignancy are useful, for example, because the presence of a set of specific factors that coincide with disease provides information crucial for diagnosing and prognosticating the status of a tissue sample.

Methods for observing a coincidence between the expression of 238P1B2 gene and 238P1B2 gene products (or perturbations in 238P1B2 gene and 238P1B2 gene products) and another factor associated with malignancy may entail detecting the overexpression of 238P1B2 mRNA or protein in a tissue sample, detecting the overexpression of PSA mRNA or protein in a tissue sample (or PSCA or PSM expression), and observing a coincidence of 238P1B2 mRNA or protein and PSA mRNA or protein overexpression (or PSCA or PSM expression). The expression of 238P1B2 and PSA mRNA in prostate tissue may be examined, where the coincidence of 238P1B2 and PSA mRNA overexpression in the sample indicates the existence of prostate cancer, prostate cancer susceptibility or the emergence or status of a prostate tumor.

Methods for detecting and quantifying the expression of 238P1B2 mRNA or protein are described herein, and standard nucleic acid and protein detection and quantification technologies are well known in the art. Standard methods for the detection and quantification of 238P1B2 mRNA include in situ hybridization using labeled 238P1B2 riboprobes, Northern blot and related techniques using 238P1B2 polynucleotide probes, RT- PCR analysis using primers specific for 238P1B2, and other amplification type detection methods, such as, for example, branched DNA, SISBA, TMA and the like. Semi-quantitative RT-PCR may be used to detect and quantify 238P1B2 mRNA expression. Any number of primers capable of amplifying 238PIB2 can be used for this purpose, including but not limited to the various primer sets specifically described herein. Polyclonal or monoclonal antibodies may be specifically reactive with the wild-type 238P1B2 protein can be used in an immunohistochemical assay of biopsied tissue.

### VI.) Identification of Molecules That Interact With 238P1B2

The 238P1B2 protein and nucleic acid sequences disclosed herein allow a skilled artisan to identify proteins, small molecules and other agents that interact with 238P1B2, as well as pathways activated by 238P1B2 via any one of a variety of art accepted protocols. For example, one can utilize one of the so-called interaction trap systems (also referred to as the "two-hybrid assay"). In such systems, molecules interact and reconstitute a transcription factor which directs expression of a reporter gene, whereupon the expression of the reporter gene is assayed. Other systems identify protein-protein interactions in vivo through reconstitution of a eukaryotic transcriptional activator, see, e.g., U.S. Patent Nos. 5,955,280 issued 21 September 1999, 5,925,523 issued 20 July 1999, 5,846,722 issued 8 December 1998 and 6,004,746 issued 21 December 1999. Algorithms are also available in the art for genome-based predictions of protein function (see, e.g., Marcotte, et al., Nature 402:4 November 1999, 83-86).

Alternatively one can screen peptide libraries to identify molecules that interact with 238P1B2 protein sequences. In such methods, peptides that bind to 238P1B2 are identified by screening libraries that encode a random or controlled collection of amino acids. Peptides encoded by the libraries are expressed as fusion proteins of bacteriophage coat proteins, the bacteriophage particles are then screened against the 238P1B2 protein(s).

Accordingly, peptides having a wide variety of uses, such as therapeutic, prognostic or diagnostic reagents, are thus identified without any prior information on the structure of the expected ligand or receptor molecule. Typical peptide libraries and screening methods that can be used to identify molecules that interact with 238P1B2 protein sequences are disclosed for example in U.S. Patent Nos. 5,723,286 issued 3 March 1998 and 5,733,731 issued 31 March 1998.

Alternatively, cell lines that express 238P1B2 are used to identify protein-protein interactions mediated by 238P1B2. Such interactions can be examined using immunoprecipitation techniques (see, e.g., Hamilton B.J., et al. Biochem. Biophys. Res. Commun. 1999, 261:646-51). 238P1B2 protein can be immunoprecipitated from 238P1B2-expressing cell lines using anti-238P1B2 antibodies. Alternatively, antibodies against His-tag can be used in a cell line engineered to express fusions of 238P1B2 and a His-tag (vectors mentioned above). The immunoprecipitated complex can be examined for protein association by procedures such as Western blotting, ³⁵S-methionine labeling of proteins, protein microsequencing, silver staining and two-dimensional gel electrophoresis.

Small molecules and ligands that interact with 238P1B2 can be identified through related screening assays. For example, small molecules can be identified that interfere with protein function, including molecules that interfere with 238P1B2's ability to mediate phosphorylation and dephosphorylation, interaction with DNA or RNA molecules as an indication of regulation of cell cycles, second messenger signaling or tumorigenesis. Similarly, small molecules that modulate 238P1B2-related ion channel, protein pump, or cell communication functions are identified and used to treat patients that have a cancer that expresses 238P1B2 (see, e.g., Hille, B., Ionic Channels of Excitable Membranes 2nd Ed., Sinauer Assoc., Sunderland, MA, 1992). Moreover, ligands that regulate 238P1B2 function can be identified based on their ability to bind 238P1B2 and activate a reporter construct. Typical methods are discussed for example in U.S. Patent No. 5,928,868 issued 27 July 1999, and include methods for forming hybrid ligands in which at least one ligand is a small molecule. In an illustrative method, cells engineered to express a fusion protein of 238P1B2 and a DNA-binding protein are used to co-express a fusion protein of a hybrid ligand/small molecule and a cDNA library transcriptional activator protein. The cells further contain a reporter gene, the expression of which is conditioned on the proximity of the first and second fusion proteins to each other, an event that occurs only if the hybrid ligand binds to target sites on both hybrid proteins. Those cells that express the reporter gene are selected and the unknown small molecule or the unknown ligand is identified. This method provides a means of identifying modulators which activate or inhibit 238P1B2.

We describe a method of screening for a molecule that interacts with an 238P1B2 amino acid sequence shown in Figure 2 or Figure 3, comprising the steps of contacting a population of molecules with a 238P1B2 amino acid sequence, allowing the population of molecules and the 238P1B2 amino acid sequence to interact under conditions that facilitate an interaction, determining the presence of a molecule that interacts with the 238P1B2 amino acid sequence, and then separating molecules that do not interact with the 238P1B2 amino acid sequence from molecules that do. The method may further comprise purifying, characterizing and identifying a molecule that interacts with the 238P1B2 amino acid sequence. The identified molecule can be used to modulate a function performed by 238P1B2. The 238P1B2 amino acid sequence may be contacted with a library of peptides.

### VII.) Diagnostic and Prognostic Methods.

As disclosed herein, 238P1B2 polynucleotides, polypeptides, reactive cytotoxic T cells (CTL), reactive helper T cells (HTL) and anti-polypeptide antibodies may be used in well known diagnostic; prognostic and therapeutic assays that examine conditions associated with dysregulated cell growth such as cancer, in particular the cancers listed in Table I (see, e.g., both its specific pattern of tissue expression as well as its overexpression in certain cancers as described for example in Example 4).

238P1B2 can be analogized to a prostate associated antigen PSA, the archetypal marker that has been used by medical practitioners for years to identify and monitor the presence of prostate cancer (see, e.g., Merrill et al., J. Urol. 163(2): 503-5120 (2000); Polascik et al., J. Urol. Aug; 162(2):293-306 (1999) and Fortier et al., J. Nat. Cancer Inst. 91(19): 1635-1640(1999)). A variety of other diagnostic markers are also used in similar contexts including p53 and K-ras (see, e.g., Tulchinsky et al., Int J Mol Med 1999 Jul 4(1):99-102 and Minimoto et al., Cancer Detect Prev 2000;24(1):1-12). Therefore, this disclosure of 238P1B2 polynucleotides and polypeptides (as well as 238P1B2 polynucleotide probes and anti-238P1B2 antibodies used to identify the presence of these molecules) and their properties allows skilled artisans to utilize these molecules in methods that are analogous to those used, for example, in a variety of diagnostic assays directed to examining conditions associated with cancer.

Typical diagnostic methods which utilize the 238P1B2 polynucleotides, polypeptides, reactive T cells and antibodies are analogous to those methods from well-established diagnostic assays which employ, e.g., PSA polynucleotides, polypeptides, reactive T cells and antibodies. For example, just as PSA polynucleotides are used as probes (for example in Northern analysis, see, e.g., Sharief et al., Biochem. Mol. Biol. Int. 33(3):567-74(1994)) and primers (for example in PCR analysis, see, e.g., Okegawa et al., J. Urol. 163(4): 1189-1190 (2000)) to observe the presence and/or the level of PSA mRNAs in methods of monitoring PSA overexpression or the metastasis of prostate cancers, the 238P1B2 polynucleotides described herein can be utilized in the same way to detect 238P1B2 overexpression or the metastasis of prostate and other cancers expressing this gene. Alternatively, just as PSA polypeptides are used to generate antibodies specific for PSA which can then be used to observe the presence and/or the level of PSA proteins in methods to monitor PSA protein overexpression (see, e.g., Stephan et al., Urology 55(4):560-3 (2000)) or the metastasis of prostate cells (see, e.g., Alanen et al., Pathol. Res. Pract. 192(3):233-7 (1996)), the 238P1B2 polypeptides described herein can be utilized to generate antibodies for use in detecting 238P1B2 overexpression or the metastasis of prostate cells and cells of other cancers expressing this gene.

Specifically, because metastases involves the movement of cancer cells from an organ of origin (such as the lung or prostate gland etc.) to a different area of the body (such as a lymph node), assays which examine a biological sample for the presence of cells expressing 238P1B2 polynucleotides and/or polypeptides can be used to provide evidence of metastasis. For example, when a biological sample from tissue that does not normally contain 238P1B2-expressing cells (lymph node) is found to contain 238P1B2- expressing cells such as the 238P1B2 expression seen in LAPC4 and LAPC9, xenografts isolated from lymph node and bone metastasis, respectively, this finding is indicative of metastasis.

Alternatively 238P1B2 polynucleotides and/or polypeptides can be used to provide evidence of cancer, for example, when cells in a biological sample that do not normally express 238P1B2 or express 238P1B2 at a different level are found to express 238P1B2 or have an increased expression of 238P1B2 (see, e.g., the 238P1B2 expression in the cancers listed in Table I and in patient samples etc. shown in the accompanying Figures). In such assays, artisans may further wish to generate supplementary evidence of metastasis by testing the biological sample for the presence of a second tissue restricted marker (in addition to 238P1B2) such as PSA, PSCA etc. (see, e.g., Alanen et al., Pathol. Res. Pract. 192(3): 233-237 (1996)).

Just as PSA polynucleotide fragments and polynucleotide variants are employed by skilled artisans for use in methods of monitoring PSA, 238P1B2 polynucleotide fragments and polynucleotide variants are used in an analogous manner. In particular, typical PSA polynucleotides used in methods of monitoring PSA are probes or primers which consist of fragments of the PSA cDNA sequence. Illustrating this, primers used to PCR amplify a PSA polynucleotide must include less than the whole PSA sequence to function in the polymerase chain reaction. In the context of such PCR reactions, skilled artisans generally create a variety of different polynucleotide fragments that can be used as primers in order to amplify different portions of a polynucleotide of interest or to optimize amplification reactions (see, e.g., Caetano-Anolles, G. Biotechniques 25(3): 472-476, 478-480 (1998); Robertson et al., Methods Mol. Biol. 98:121-154 (1998)). An additional illustration of the use of such fragments is provided in Example 4, where a 238P1B2 polynucleotide fragment is used as a probe to show the expression of 238P1B2 RNAs in cancer cells. In addition, variant polynucleotide sequences are typically used as primers and probes for the corresponding mRNAs in PCR and Northern analyses (see, e.g., Sawai et al., Fetal Diagn. Ther. 1996 Nov-Dec 11(6):407-13 and Current Protocols In Molecular Biology, Volume 2, Unit 2, Frederick M. Ausubel et al. eds., 1995)). Polynucleotide fragments and variants are useful in this context where they are capable of binding to a target polynucleotide sequence (e.g., a 238P1B2 polynucleotide shown in Figure 2 or variant thereof) under conditions of high stringency.

Furthermore, PSA polypeptides which contain an epitope that can be recognized by an antibody or T cell that specifically binds to that epitope are used in methods of monitoring PSA. 238P1B2 polypeptide fragments and polypeptide analogs or variants can also be used in an analogous manner. This practice of using polypeptide fragments or polypeptide variants to generate antibodies (such as anti-PSA antibodies or T cells) is typical in the art with a wide variety of systems such as fusion proteins being used by practitioners (see, e.g., Current Protocols In Molecular Biology, Volume 2, Unit 16, Frederick M. Ausubel et al. eds., 1995). In this context, each epitope(s) functions to provide the architecture with which an antibody or T cell is reactive. Typically, skilled artisans create a variety of different polypeptide fragments that can be used in order to generate immune responses specific for different portions of a polypeptide of interest (see, e.g., U.S. Patent No. 5,840,501 and U.S. Patent No. 5,939,533). For example it may be preferable to utilize a polypeptide comprising one of the 238P1B2 biological motifs discussed herein or a motif-bearing subsequence which is readily identified by one of skill in the art based on motifs available in the art. Polypeptide fragments, variants or analogs are typically useful in this context as long as they comprise an epitope capable of generating an antibody or T cell specific for a target polypeptide sequence (e.g. a 238P1B2 polypeptide shown in Figure 3).

As shown herein, the 238P1B2 polynucleotides and polypeptides (as well as the 238P1B2 polynucleotide probes and anti-238P1B2 antibodies or T cells used to identify the presence of these molecules) exhibit specific properties that make them useful in diagnosing cancers such as those listed in Table I. Diagnostic assays that measure the presence of 238P1B2 gene products, in order to evaluate the presence or onset of a disease condition described herein, such as prostate cancer, are used to identify patients for preventive measures or further monitoring, as has been done so successfully with PSA. Moreover, these materials satisfy a need in the art for molecules having similar or complementary characteristics to PSA in situations where, for example, a definite diagnosis of metastasis of prostatic origin cannot be made on the basis of a test for PSA alone (see, e.g., Alanen et al., Pathol. Res. Pract. 192(3): 233-237 (1996)), and consequently, materials such as 238P1B2 polynucleotides and polypeptides (as well as the 238P1B2 polynucleotide probes and anti-238P1B2 antibodies used to identify the presence of these molecules) need to be employed to confirm a metastases of prostatic origin.

Finally, in addition to their use in diagnostic assays, the 238P1B2 polynucleotides disclosed herein have a number of other utilities such as their use in the identification of oncogenetic associated chromosomal abnormalities in the chromosomal region to which the 238P1B2 gene maps (see Example 3 below). Moreover, in addition to their use in diagnostic assays, the 238P1B2-related proteins and polynucleotides disclosed herein have other utilities such as their use in the forensic analysis of tissues of unknown origin (see, e.g., Takahama K Forensic Sci Int 1996 Jun 28;80(1-2): 63-9).

Additionally, 238P1B2-related proteins or polynucleotides described herein can be used to treat a pathologic condition characterized by the over-expression of 238P1B2. For example, the amino acid or nucleic acid sequence of Figure 2 or Figure 3, or fragments of either, can be used to generate an immune response to a 238P1B2 antigen. Antibodies or other molecules that react with 238P1B2 can be used to modulate the function of this molecule, and thereby provide a therapeutic benefit.

### VIII.) Kits

For use in the diagnostic and therapeutic applications described herein, kits are also described herein. Such kits can comprise a carrier, package or container that is compartmentalized to receive one or more containers such as vials, tubes, and the like, each of the container(s) comprising one of the separate elements to be used in the method. For example, the container(s) can comprise a probe that is or can be detectably labeled. Such probe can be an antibody or polynucleotide specific for a 238P1B2-related protein or a 238P1B2 gene or message, respectively. Where the method utilizes nucleic acid hybridization to detect the target nucleic acid, the kit can also have containers containing nucleotide(s) for amplification of the target nucleic acid sequence and/or a container comprising a reporter-means, such as a biotin-binding protein, such as avidin or streptavidin, bound to a reporter molecule, such as an enzymatic, florescent, or radioisotope label. The kit can include all or part of the amino acid sequence of Figure 2 or Figure 3 or analogs thereof, or a nucleic acid molecules that encodes such amino acid sequences.

The kit will typically comprise the container described above and one or more other containers comprising materials desirable from a commercial and user standpoint, including buffers, diluents, filters, needles, syringes, and package inserts with instructions for use.

A label can be present on the container to indicate that the composition is used for a specific therapy or non-therapeutic application, and can also indicate directions for either *in vivo* or *in vitro* use, such as those described above. Directions and or other information can also be included on an insert which is included with the kit.

### EXAMPLES:

Various aspects of the invention are further described and illustrated by way of the several examples that follow, none of which are intended to limit the scope of the invention.

### Example 1: SSH-Generated Isolation of a cDNA Fragment of the 238P1B2 Gene

The Suppression Subtractive Hybridization (SSH) procedure using cDNA derived from patient cancer tissues is used to isolate genes that are over-expressed in cancer. The 238P1B2 SSH cDNA sequence was derived from a colon cancer pool minus normal tissue cDNA subtraction. Included in the driver were cDNAs derived from 10 normal tissues. The 238P1B2 cDNA was identified as highly expressed in the prostate cancer tissue pool, with restricted expression detected in normal tissues.

The SSH DNA sequence of 210 bp (Figure 1) is novel but shows homology to mouse olfactory receptor MOR14-10 mRNA. A 238P1B2 cDNA, 238P1B2-clone A, of 3754 bp was isolated from prostate cDNA library, revealing an ORF of 254 amino acids (Figure 2, Figure 3 and Figure 4A).

### Materials and Methods

### Human Tissues:

The patient cancer and normal tissues were purchased from different sources such as the NDRI (Philadelphia, PA). mRNA for some normal tissues were purchased from Clontech, Palo Alto, CA.

### RNA Isolation:

Tissues were homogenized in Trizol reagent (Life Technologies, Gibco BRL) using 10 ml/ g tissue isolate total RNA. Poly A RNA was purified from total RNA using Qiagen's Oligotex mRNA Mini and Midi kits. Total and mRNA were quantified by spectrophotometric analysis (O.D. 260/280 nm) and analyzed by gel electrophoresis.

### Oligonucleotides:

The following HPLC purified oligonucleotides were used.

### DPNCDN (cDNA synthesis primer):

5'TTTT**GATCAA**GCTT₃₀3'

### Adaptor 1:

5'CTAATACGACTCACTATAGGGCTCGAGCGGCCGCCCGGGCAG3' (SEQ ID NO: 708)

3'GGCCCGTCCTAG5'

### Adaptor 2:

5'GTAATACGACTCACTATAGGGCAGCGTGGTCGCGGCCGAG3'
3'CGGCTCCTAG5'

### PCR primer 1:

5'CTAATACGACTCACTATAGGGC3'

### Nested primer (NP) 1:

5'TCGAGCGGCCGCCCGGGCAGGA3'

### Nested primer (NP)2:

5'AGCGTGGTCGCGGCCGAGGA3'

### Suppression Subtractive Hybridization:

Suppression Subtractive Hybridization (SSH) was used to identify cDNAs corresponding to genes that are differentially expressed in cancer. The SSH reaction utilized cDNA from colon cancer and normal tissues.

The gene 238P1B2 sequence was derived from a colon cancer pool minus normal tissue cDNA subtraction. The SSH DNA sequence (Figure 1) was identified.

The cDNA derived from of a pool of normal tissues was used as the source of the "driver" cDNA, while the cDNA from a pool of patient cancer tissues was used as the source of the "tester" cDNA. Double stranded cDNAs corresponding to tester and driver cDNAs were synthesized from 2 µg of poly(A)⁺ RNA isolated from the relevant xenograft tissue, as described above, using CLONTECH's PCR-Select cDNA Subtraction Kit and 1 ng of oligonucleotide DPNCDN as primer. First- and second-strand synthesis were carried out as described in the Kit's user manual protocol (CLONTECH Protocol No. PT1117-1, Catalog No. K1804-1). The resulting cDNA was digested with Dpn II for 3 hrs at 37°C. Digested cDNA was extracted with phenol/chloroform (1:1) and ethanol precipitated.

Driver cDNA was generated by combining in a 1:1 ratio Dpn II digested cDNA from the relevant tissue source (see above) with a mix of digested cDNAs derived from the nine normal tissues: stomach, skeletal muscle, lung, brain, liver, kidney, pancreas, small intestine, and heart.

Tester cDNA was generated by diluting 1 µl of Dpn II digested cDNA from the relevant tissue source (see above) (400 ng) in 5 µl of water. The diluted cDNA (2 µl, 160 ng) was then ligated to 2 µl of Adaptor 1 and Adaptor 2 (10 µM), in separate ligation reactions, in a total volume of 10 µl at 16°C overnight, using 400 u of T4 DNA ligase (CLONTECH). Ligation was terminated with 1 µl of 0.2 M EDTA and heating at 72°C for 5 min.

The first hybridization was performed by adding 1.5 µl (600 ng) of driver cDNA to each of two tubes containing 1.5 µl (20 ng) Adaptor 1- and Adaptor 2- ligated tester cDNA. In a final volume of 4 µl, the samples were overlaid with mineral oil, denatured in an MJ Research thermal cycler at 98°C for 1.5 minutes, and then were allowed to hybridize for 8 hrs at 68°C. The two hybridizations were then mixed together with an additional 1 µl of fresh denatured driver cDNA and were allowed to hybridize overnight at 68°C. The second hybridization was then diluted in 200 µl of 20 mM Hepes, pH 8.3, 50 mM NaCl, 0.2 mM EDTA, heated at 70°C for 7 min. and stored at -20°C.

### PCR Amplification, Cloning and Sequencing of Gene Fragments Generated from SSH:

To amplify gene fragments resulting from SSH reactions, two PCR amplifications were performed. In the primary PCR reaction 1 µl of the diluted final hybridization mix was added to 1 µl of PCR primer 1 (10 uM), 0.5 µl dNTP mix (10 µM), 2.5 µl 10 x reaction buffer (CLONTECH) and 0.5 µl 50 x Advantage cDNA polymerase Mix (CLONTECH) in a final volume of 25 µl. PCR 1 was conducted using the following conditions: 75°C for 5 min., 94°C for 25 sec., then 27 cycles of 94°C for 10 sec, 66°C for 30 sec, 72°C for 1.5 min. Five separate primary PCR reactions were performed for each experiment The products were pooled and diluted 1:10 with water. For the secondary PCR reaction, 1 µl from the pooled and diluted primary PCR reaction was added to the same reaction mix as used for PCR 1, except that primers NP 1 and NP2 (10 µM) were used instead ofPCR primer 1. PCR 2 was performed using 10-12 cycles of 94°C for 10 sec, 68°C for 30 sec, and 72°C for 1.5 minutes. The PCR products were analyzed using 2% agarose gel electrophoresis.

The PCR products were inserted into pCR2.1 using the T/A vector cloning kit (Invitrogen). Transformed *E*. *coli* were subjected to blue/white and ampicillin selection. White colonies were picked and arrayed into 96 well plates and were grown in liquid culture overnight. To identify inserts, PCR amplification was performed on 1 ml of bacterial culture using the conditions of PCR1 and NP1 and NP2 as primers. PCR products were analyzed using 2% agarose gel electrophoresis.

Bacterial clones were stored in 20% glycerol in a 96 well format. Plasmid DNA was prepared, sequenced, and subjected to nucleic acid homology searches of the GenBank, dBest, and NCI-CGAP databases.

### RT-PCR Expression Analysis:

First strand cDNAs can be generated from 1 µg of mRNA with oligo (dT)12-18 priming using the Gibco-BRL Superscript Preamplification system. The manufacturer's protocol was used which included an incubation for 50 min at 42°C with reverse transcriptase followed by RNAse H treatment at 37°C for 20 min. After completing the reaction, the volume can be increased to 200 µl with water prior to normalization. First strand cDNAs from 16 different normal human tissues can be obtained from Clontech.

Normalization of the first strand cDNAs from multiple tissues was performed by using the primers 5'atatcgccgcgctcgtcgtcgacaa3' and 5'agccacacgcagctcattgtagaagg 3' to amplify β-actin. First strand cDNA (5 µl) were amplified in a total volume of 50 µl containing 0.4 µM primers, 0.2 µM each dNTPs, 1XPCR buffer (Clontech, 10 mM Tris-HCL, 1.5 mM MgCl₂, 50 mM KCl, pH8.3) and 1X Klentaq DNA polymerase (Clontech). Five µl of the PCR reaction can be removed at 18, 20, and 22 cycles and used for agarose gel electrophoresis. PCR was performed using an MJ Research thermal cycler under the following conditions: Initial denaturation can be at 94°C for 15 sec, followed by a 18, 20, and 22 cycles of 94°C for 15, 65°C for 2 min, 72°C for 5 sec. A final extension at 72°C was carried out for 2 min. After agarose gel electrophoresis, the band intensities of the 283 b.p. β-actin bands from multiple tissues were compared by visual inspection. Dilution factors for the first strand cDNAs were calculated to result in equal β-actin band intensities in all tissues after 22 cycles of PCR. Three rounds of normalization can be required to achieve equal band intensities in all tissues after 22 cycles of PCR.

To determine expression levels of the 238P1B2 gene, 5 µl of normalized first strand cDNA were analyzed by PCR using 26, and 30 cycles of amplification. Semi-quantitative expression analysis can be achieved by comparing the PCR products at cycle numbers that give light band intensities. The primers used for RT-PCR were designed using the 238P1B2 SSH sequence and are listed below:
**238P1B2.1**
   5'- TTGCAGAATATCACCTCCACTTCC -3'
**238P1B2.2**
   5'- GATCAGGCTGTTTCCCAAGAGAG -3'

A typical RT-PCR expression analysis is shown in Figure 14. RT-PCR expression analysis was performed on first strand cDNAs generated using pools of tissues from multiple samples. The cDNAs were shown to be normalized using beta-actin PCR. Results show strong expression of 238P1B2 in prostate cancer pool but not in vital pool 1 and vital pool 2.

### Example 2: Full Length Cloning of 238P1B2

The 238P1B2 SSH cDNA sequence was derived from a colon cancer pool minus normal tissue cDNA subtraction. The SSH cDNA sequence (Figure 1) was designated 238P1B2.

The SSH DNA sequence of 210 bp (Figure 1) is novel and shows 91% identity to mouse olfactory receptor MOR14-10 mRNA.

A full length cDNA (238P1B2-clone A) of 3754 bp was isolated from prostate library, revealing an ORF of 254 amino acids (Figures 2 and 3). The cDNA shows highest homology to the mouse MOR-14-1 and MOR14-10 olfactory receptors, with identities of 85% over 912 nucleotides and 83% over 906 nucleotides respectively (Figure 4).

The protein sequence reveals 7 transmembrane domains and has homology to G protein-coupled receptors (GPCRs) involved in olfaction (Raming et al., 1993, Nature 361: 353; Malnic et al., 1999, Cell 96:713). Proteins that are members of this receptor family exhibit an extracellular amino-terminus, three additional extracellular loops, three intracellular loops and an intracellular carboxyl terminus.

The most homologous sequence to 238P1B2 is mouse MOR14-1 protein. The two proteins share 83% identity over a 253 amino acid region. Alignment of the two proteins is shown in Figure 4.

238P1B2 also shows significant homology to the prostate specific GPCR, PHOR-1. The two proteins share 48% identity over a region of 250 amino acids (Figure 4).

The full length 238P1B2 cDNA (238P1B2-clone A) was deposited with the American Type Culture Collection on March 7, 2002, and assigned accession number PTA-4124.

238P1B2 v.1A protein sequence codes for a six transmembrane protein. Extension of the protein at the amino terminus adds another 62 amino acids to 238P1B2 v.1A leading to 238P1B2 v.1B. This 62 amino acid terminus of 238P1B2 v.1B encodes an additional transmembrane region, and thereby making 238P1B2 v.1B a seven transmembrane protein. Furthermore, this amino terminus shows 84% identity to the mouse MOR14-10 G-coupled protein receptor, indicating that this amino-terminal portion is encoded in nature.

The natural expression of 238P1B2 v.1B can occur if the stop site upstream of the 62 amino acid extension is modified by single nucleic acid substitution leading to conversion of the stop codon into a coding amino acid. Polymorphisms can exist within different tissues, or between different individuals that mutate the stop codon, and therefore allowing proper translation of the 62 amino acid amino-terminal region of 238P1B2 v.1B. Such a variation can either add a start methionine directly, or extend the coding region 238P1B2 v.1B for an additional 16 amino acids until reaching a start methionine at nucleic acid position 1896.

### Example 3: Chromosomal Mapping of 238P1B2 Gene

Chromosomal localization can implicate genes in disease pathogenesis. Several chromosome mapping approaches are available including fluorescent *in situ* hybridization (FISH), human/hamster radiation hybrid (RH) panels (Walter et al., 1994; Nature Genetics 7:22; Research Genetics, Huntsville Al), human-rodent somatic cell hybrid panels such as is available from the Coriell Institute (Camden, New Jersey), and genomic viewers utilizing BLAST homologies to sequenced and mapped genomic clones (NCBI, Bethesda, Maryland).

Using 238P1B2 sequence and the NCBI BLAST tool: (see World Wide Web URL www.ncbi.nlm.nih.gov/genome/seq/page.cgi?F=HsBlast.html&&ORG=Hs), placed 238P1B2 to chromosome 11p15.5, a region rich in GPCRs.

Because the human 238P1B2 gene maps to chromosome 11p15.5, polynucleotides encoding different regions of the 238P1B2 protein can be used to characterize cytogenetic abnormalities on chromosome 11, band p15.5 that have been identified as being associated with various cancers. In particular, a variety of chromosomal abnormalities in 11p15.5 have been identified as frequent cytogenetic abnormalities in a number of different cancers (see, e.g., Lai et al., 2000, Clin. Cancer Res. 6(8):3172-6; Oya and Schulz, 2000, Br. J. Cancer 83(5):626-31; Svaren et al., Sept. 12, 2000, J. Biol. Chem.). Consequently, polynucleotides encoding specific regions of the 238P1B2 protein provide new tools that can be used to delineate, with greater precision than previously possible, the specific nature of the cytogenetic abnormalities in this region of chromosome 11 that contribute to the malignant phenotype. In this context, these polynucleotides satisfy a need in the art for expanding the sensitivity of chromosomal screening in order to identify more subtle and less common chromosomal abnormalities (see, e.g., Evans et al., 1994, Am J. Obstet. Gynecol. 171(4):1055-1057).

### Example 4: Expression Analysis of 238P1B2 in Normal Tissues and Patient Specimens

Expression analysis by RT-PCR demonstrated that 238P1B2 is strongly expressed in prostate cancer patient specimens (Figure 14). First strand cDNA was prepared from vital pool 1 (liver, lung and kidney), vital pool 2 (pancreas, colon and stomach) and prostate cancer pool. Normalization was performed by PCR using primers to actin and GAPDH. Semi-quantitative PCR, using primers to 238P1B2, was performed at 26 and 30 cycles of amplification. Results show strong expression of 238P1B2 in prostate cancer pool but not in vital pool 1 and vital pool 2.

Northern blot analysis of 238P1B2 in 16 human normal tissues is shown in Figure 15. Results show absence of 238P1B2 expression in all 16 normal tissues tested. Extensive analysis of expression of 238P1B2 in 76 human tissues shows restricted expression of 238P1B2 in placenta (Figure 16).

Expression of 238P1B2 in patient cancer specimens and human normal tissues is shown in Figure 17. RNA was extracted from a pool of three prostate cancers, as well as from normal prostate (NP), normal bladder (NB), normal kidney (NK), normal colon (NC), normal lung (NL) normal breast (NBr) and normal ovary (NO). Northern blot with 10 ug of total RNA/lane was probed with 238P1B2 sequence. The results show expression of an approximately 4.5kb 238P1B2 transcript in the prostate cancer pool and ovary but not in the other normal tissues tested. Analysis of individual patient specimens shows strong expression of 238P1B2 in prostate cancer tissues (Figure 18). The expression for 238P1B2 detected in tumors of Gleason score 7 is significantly stronger than the expression detected in tumors of Gleason score 5. This result indicates that 238P1B2 can be used as a prognostic marker for prostate cancer.

The restricted expression of 238P1B2 in normal tissues and the expression detected in prostate cancer suggest that 238P1B2 is a potential therapeutic target and a diagnostic marker for human cancers.

### Example 5: Transcript Variants of 238P1B2

Transcript variants are variants of mature mRNA from the same gene by alternative transcription or alternative splicing. Alternative transcripts are transcripts from the same gene that start transcription at different points. Splice variants are mRNA variants spliced differently from the same transcript In eukaryotes, when a multi-exon gene is transcribed from genomic DNA, the initial RNA is spliced to produce functional mRNA, which has only exons and is used for translation into an amino acid sequence. Accordingly, a given gene can have zero to many alternative transcripts and each transcript can have zero to many splice variants. Each transcript variant has a unique exon makeup, and can have different coding and/or non-coding (5' or 3' end) portions, from the original transcript. Transcript variants can code for similar or different proteins with the same or a similar function or can encode proteins with different functions, and can be expressed in the same tissue at the same time, or in different tissues at the same time, or in the same tissue at different times, or in different tissues at different times. Proteins encoded by transcript variants can have similar or different cellular or extracellular localizations, e.g., secreted versus intracellular.

Transcript variants are identified by a variety of art-accepted methods. For example, alternative transcripts and splice variants are identified by full-length cloning experiments, or by use of full-length transcript and EST sequences. First, all human ESTs were grouped into clusters which show direct or indirect identity with each other. Second, ESTs in the same cluster were further grouped into sub-clusters and assembled into a consensus sequence. The original gene sequence is compared to the consensus sequence(s) or other full-length sequences. Each consensus sequence is a potential splice variant for that gene (see, e.g., World Wide Web URL www.doubletwist.com/products/c11_agentsOverview.jhtml). Even when a variant is identified that is not a full-length clone, that portion of the variant is very useful for antigen generation and for further cloning of the full-length splice variant, using techniques known in the art.

Moreover, computer programs are available in the art that identify transcript variants based on genomic sequences. Genomic-based transcript variant identification programs include FgenesH (A. Salamov and V. Solovyev, "Ab initio gene finding in Drosophila genomic DNA," Genome Research. 2000 April;10(4):516-22); Grail (World Wide Web URL compbio.ornl.gov/Grail-binlEmptyGrailForm) and GenScan (World Wide Web URL genes.mit.edu/GENSCAN.html). For a general discussion of splice variant identification protocols see., e.g., Southan, C., A genomic perspective on human proteases, FEBS Lett. 2001 Jun 8; 498(2-3):214-8; de Souza, S.J., et al., Identification of human chromosome 22 transcribed sequences with ORF expressed sequence tags, Proc. Natl Acad Sci U S A. 2000 Nov 7; 97(23):12690-3.

To further confirm the parameters of a transcript variant, a variety of techniques are available in the art, such as full-length cloning, proteomic validation, PCR-based validation, and 5' RACE validation, etc. (see e.g., Proteomic Validation: Brennan, S.O., et al., Albumin banks peninsula: a new termination variant characterized by electrospray mass spectrometry, Biochem Biophys Acta. 1999 Aug 17;1433(1-2):321-6; Ferranti P, et al., Differential splicing of pre-messenger RNA produces multiple forms of mature caprine alpha(s1)-casein, Eur J Biochem. 1997 Oct 1;249(1):1-7. For PCR-based Validation: Wellmann S, et al., Specific reverse transcription-PCR quantification of vascular endothelial growth factor (VEGF) splice variants by LightCycler technology, Clin Chem. 2001 Apr;47(4):654-60; Jia, H.P., et al., Discovery of new human beta-defensins using a genomics-based approach, Gene. 2001 Jan 24; 263(1-2):211-8. For PCR-based and 5' RACE Validation: Brigle, K.E., et al., Organization of the murine reduced folate carrier gene and identification of variant splice forms, Biochem Biophys Acta. 1997 Aug 7; 1353(2): 191-8).

It is known in the art that genomic regions are modulated in cancers. When the genomic region to which a gene maps is modulated in a particular cancer, the alternative transcripts or splice variants of the gene are modulated as well. Disclosed herein is that 238P1B2 has a particular expression profile. Alternative transcripts and splice variants of 238P1B2 can share this expression pattern, thus serving as tumor-associated markers/antigens.

The exon composition of the original transcript, designated as 238P1B2 v.1, is shown in Table XXIII. No transcript variant has been identified by the above methods.

### Example 6: Single Nucleotide Polymorphisms of 238P1B2

A Single Nucleotide Polymorphism (SNP) is a single base pair variation in a nucleotide sequence at a specific location. At any given point of the genome, there are four possible nucleotide base pairs: A/T, C/G, G/C and T/A. Genotype refers to the specific base pair sequence of one or more locations in the genome of an individual. Haplotype refers to the base pair sequence of more than one location on the same DNA molecule (or the same chromosome in higher organisms), often in the context of one gene or in the context of several tightly linked genes. SNPs that occur on a cDNA are called cSNPs. These cSNPs may change amino acids of the protein encoded by the gene and thus change the functions of the protein. Some SNPs cause inherited diseases; others contribute to quantitative variations in phenotype and reactions to environmental factors including diet and drugs among individuals. Therefore, SNPs and/or combinations of alleles (called haplotypes) have many applications, including diagnosis of inherited diseases, determination of drug reactions and dosage, identification of genes responsible for diseases, and analysis of the genetic relationship between individuals (P. Nowotny, J. M. Kwon and A. M. Goate, " SNP analysis to dissect human traits," Curr. Opin. Neurobiol. 2001 Oct; 11(5):637-641; M. Pirmohamed and B. K. Park, "Genetic susceptibility to adverse drug reactions," Trends Pharmacol. Sci. 2001 Jun; 22(6):298-305; J. H. Riley, C. J. Allan, E. Lai and A. Roses, " The use of single nucleotide polymorphisms in the isolation of common disease genes," Pharmacogenomics. 2000 Feb; 1(1):39-47; R. Judson, J. C. Stephens and A. Windemuth, "The predictive power of haplotypes in clinical response," Pharmacogenomics. 2000 feb; 1(1):15-26).

SNPs are identified by a variety of art-accepted methods (P. Bean, "The promising voyage of SNP target discovery," Am. Clin. Lab. 2001 Oct-Nov; 20(9):18-20; K. M. Weiss, "In search of human variation," Genome Res. 1998 Jul; 8(7):691-697; M. M. She, "Enabling large-scale pharmacogenetic studies by high-throughput mutation detection and genotyping technologies," Clin. Chem. 2001 Feb; 47(2):164-172). For example, SNPs are identified by sequencing DNA fragments that show polymorphism by gel-based methods such as restriction fragment length polymorphism (RFLP) and denaturing gradient gel electrophoresis (DGGE). They can also be discovered by direct sequencing of DNA samples pooled from different individuals or by comparing sequences from different DNA samples. With the rapid accumulation of sequence data in public and private databases, one can discover SNPs by comparing sequences using computer programs (Z. Gu, L. Hillier and P. Y. Kwok, "Single nucleotide polymorphism hunting in cyberspace," Hum. Mutat 1998; 12(4):221-225). SNPs can be verified and genotype or haplotype of an individual can be determined by a variety of methods including direct sequencing and high throughput microarrays (P. Y. Kwok, "Methods for genotyping single nucleotide polymorphisms," Annu. Rev. Genomics Hum. Genet. 2001; 2:235-258; M. Kokoris, K. Dix, K. Moynihan, J. Mathis, B. Erwin, P. Grass, B. Hines and A. Duesterhoeft, "High-throughput SNP genotyping with the Masscode system," Mol. Diagn. 2000 Dec; 5(4):329-340).

238P1B2 SNPs are identified by direct sequencing of the cDNA clones and by comparison of those sequences with public and proprietary sequences. By comparing the sequences with high quality proprietary or public sequences (e.g, NCBI/GenBank, accesible at World Wide Web URL www.ncbi.n1m.nih.gov), five SNPs of 238P1B2 were identified at nucleotide positions 274 (T/C), 1268 (T/G), 1299 (T/G) 2806 (T/C) and 3025 (T/C). The transcripts or proteins with alternative alleles were designated as variants 238P1B2 v.2, v.3, v.4, v.5 and v.6. Figure 10 shows the schematic alignment of the nucleotide variants. Figure 11 shows the schematic alignment of protein variants, corresponding to nucleotide variants. Nucleotide variants that code for the same amino acid sequence as variant 1 are not shown in Figure 11. These alleles of the SNPs, though shown separately here, can occur in different combinations (haplotypes).

### Example 7: Production of Recombinant 238P1B2 in Prokaryotic Systems

To express recombinant 238P1B2 in prokaryotic cells, the full or partial length 238P1B2 variant 1a, variant 1b, and variant 2 cDNA sequences are cloned into any one of a variety of expression vectors known in the art. One or more of the following regions of 238P1B2 variants are expressed in these constructs: amino acids 1 to 254 of variant 1a; amino acids 1 to 316 of variant 1b, and amino acids 1-254 of variant 2, or any 8, 9, 10, 11, 12, 13, 14, 15, 16, 17,18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 or more contiguous amino acids from 238P1B2, variants, or analogs thereof.

### A. In vitro transcription and translation constructs:

pCRII: To generate 238P1B2 sense and anti-sense RNA probes for RNA *in situ* investigations, pCRII constructs (Invitrogen, Carlsbad CA) are generated encoding either all of or fragments of the 238P1B2 cDNA. The pCRII vector has Sp6 and T7 promoters flanking the insert to drive the transcription of 238P1B2 RNA for use as probes in RNA in situ hybridization experiments. These probes are used to analyze the cell and tissue expression of 238P1B2 at the RNA level. Transcribed 238P1B2 RNA representing the DNA amino acid coding region of the 238P1B2 gene is used in *in vitro* translation systems such as the TnT^{™} Coupled Reticulolysate Sytem (Promega, Corp., Madison, WI) to synthesize 238P1B2 protein.

### B. Bacterial Constructs:

pGEX Constructs: To generate recombinant 238P1B2 proteins in bacteria that are fused to the Glutathione S-transferase (GST) protein; all of or parts of the 238P1B2 cDNA protein coding sequence are fused to the GST gene by cloning into pGEX-6P-1 or any other GST- fusion vector of the pGEX family (Amersham Pharmacia Biotech, Piscataway, NJ). These constructs allow controlled expression of recombinant 238P1B2 protein sequences with GST fused at the amino-terminus and a six histidine epitope (6X His) at the carboxyl-terminus. The GST and 6X His tags permit purification of the recombinant fusion protein from induced bacteria with the appropriate affinity matrix and allow recognition of the fusion protein with anti-GST and anti-His antibodies. The 6X His tag is generated by adding 6 histidine codons to the cloning primer at the 3' end, e.g., of the open reading frame (ORF). A proteolytic cleavage site, such as the PreScission^{™} recognition site in pGEX-6P-1, can be employed to permit cleavage of the GST tag from 238P1B2-related protein. The ampicillin resistance gene and pBR322 origin permits selection and maintenance of the pGEX plasmids in *E. coli.*

pMAL Constructs: To generate, in bacteria, recombinant 238P1B2 proteins that are fused to maltose-binding protein (MBP), all of or parts of the 238P1B2 cDNA protein coding sequence are fused to the MBP gene by cloning into the pMAL-c2X and pMAL-p2X vectors (New England Biolabs, Beverly, MA). These constructs allow controlled expression of recombinant 238P1B2 protein sequences with MBP fused at the amino-terminus and a 6X His epitope tag at the carboxyl-terminus. The MBP and 6X His tags permit purification of the recombinant protein from induced bacteria with the appropriate affinity matrix and allow recognition of the fusion protein with anti-MBP and anti-His antibodies. The 6X His epitope tag is generated by adding 6 histidine codons to the 3' cloning primer. A Factor Xa recognition site permits cleavage of the pMAL tag from 238P1B2. The pMAL-c2X and pMAL-p2X vectors are optimized to express the recombinant protein in the cytoplasm or periplasm respectively. Periplasm expression enhances folding of proteins with disulfide bonds.

pET Constructs: To express 238P1B2 in bacterial cells, all of or parts of the 238P1B2 cDNA protein coding sequence are cloned into the pET family of vectors (Novagen, Madison, WI). These vectors allow tightly controlled expression of recombinant 238P1B2 protein in bacteria with and without fusion to proteins that enhance solubility, such as NusA and thioredoxin (Trx), and epitope tags, such as 6X His and S-Tag ™ that aid purification and detection of the recombinant protein. For example, constructs are made utilizing pET NusA fusion system 43.1 such that regions of the 238P1B2 protein are expressed as amino-terminal fusions to NusA. ANusA-fusion protein encompassing amino acids 412-254 of 238P1B2 with a C-terminal 6xHis tag was expressed in *E*. *Coli,* purified by metal chelate affinity chromatography, and used as an immunogen for generation of antibodies.

### C. Yeast Constructs:

pESC Constructs: To express 238P1B2 in the yeast species *Saccharomyces cerevisiae* for generation of recombinant protein and functional studies, all of or parts of the 238P1B2 cDNA protein coding sequence are cloned into the pESC family of vectors each of which contain 1 of 4 selectable markers, HIS3, TRP1, LEU2, and URA3 (Stratagene, La Jolla, CA). These vectors allow controlled expression from the same plasmid of up to 2 different genes or cloned sequences containing either Flag^{™} or Myc epitope tags in the same yeast cell. This system is useful to confirm protein-protein interactions of 238P1B2. In addition, expression in yeast yields similar post-translational modifications, such as glycosylations and phosphorylations, that are found when expressed in eukaryotic cells.

pESP Constructs: To express 238P1B2 in the yeast species *Saccharomyces pombe,* all of or parts of the 238P1B2 cDNA protein coding sequence are cloned into the pESP family of vectors. These vectors allow controlled high level of expression of a 238P1B2 protein sequence that is fused at either the amino terminus or at the carboxyl terminus to GST which aids purification of the recombinant protein. A Flag^{™} epitope tag allows detection of the recombinant protein with anti- Flag^{™} antibody.

### Example 8: Production of Recombinant 238P1B2 in Eukaryotic Systems

### A. Mammalian Constructs:

To express recombinant 238P1B2 in eukaryotic cells, the full or partial length 238P1B2 cDNA sequences can be cloned into any one of a variety of expression vectors known in the art. One or more of the following regions of 238P1B2 are expressed in these constructs: amino acids 1 to 254 of variant 1A or variant 2, amino acids 1 to 316 of variant 1B, or any 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 or more contiguous amino acids from 238P1B2, variants, or analogs thereof. A region of a specific variant of 238P1B2 is expressed that encodes an amino acid at specific position which differs from the amino acid of any other variant found at that position. Alternatively, a region of a variant of 238P1B2 is expressed that lies partly or entirely within a sequence that is unique to that variant.

The constructs can be transfected into any one of a wide variety of mammalian cells such as 293T cells. Transfected 293T cell lysates can be probed with the anti-238P1B2 polyclonal serum, described herein.

**pcDNA4/HisMax Constructs**: To express 238P1B2 in mammalian cells, a 238P1B2 ORF, or portions thereof, of 238P1B2 are cloned into pcDNA4/HisMax Version A (Invitrogen, Carlsbad, CA). Protein expression is driven from the cytomegalovirus (CMV) promoter and the SP16 translational enhancer. The recombinant protein has Xpress^{™} and six histidine (6X His) epitopes fused to the amino-terminus. The pcDNA4/HisMax vector also contains the bovine growth hormone (BGH) polyadenylation signal and transcription termination sequence to enhance mRNA stability along with the SV40 origin for episomal replication and simple vector rescue in cell lines expressing the large T antigen. The Zeocin resistance gene allows for selection of mammalian cells expressing the protein and the ampicillin resistance gene and ColE1 origin permits selection and maintenance of the plasmid in *E*. *coli.*

**pcDNA3.1/MycHis Constructs:** To express 238P1B2 in mammalian cells, a 238P1B2 ORF, or portions thereof, of 238P1B2 with a consensus Kozak translation initiation site are cloned into pcDNA3.1/MycHis Version A (Invitrogen, Carlsbad, CA). Protein expression is driven from the cytomegalovirus (CMV promoter. The recombinant proteins have the myc epitope and 6X His epitope fused to the carboxyl-terminus. The pcDNA3.1/MycHis vector also contains the bovine growth hormone (BGH) polyadenylation signal and transcription termination sequence to enhance mRNA stability, along with the SV40 origin for episomal replication and simple vector rescue in cell lines expressing the large T antigen. The Neomycin resistance gene can be used, as it allows for selection of mammalian cells expressing the protein and the ampicillin resistance gene and ColE1 origin permits selection and maintenance of the plasmid in E. *coli.*

**pcDNA3.1/CT-GFP-TOPO Construct:** To express 238P1B2 in mammalian cells and to allow detection of the recombinant proteins using fluorescence, a 238P 1B2 ORF, or portions thereof, with a consensus Kozak translation initiation site are cloned into pcDNA3.1/CT-GFP-TOPO (Invitrogen, CA). Protein expression is driven from the cytomegalovirus (CMV) promoter. The recombinant proteins have the Green Fluorescent Protein (GFP) fused to the carboxyl-terminus facilitating non-invasive, in vivo detection and cell biology studies. The pcDNA3.1CT-GFP-TOPO vector also contains the bovine growth hormone (BGH) polyadenylation signal and transcription termination sequence to enhance mRNA stability along with the SV40 origin for episomal replication and simple vector rescue in cell lines expressing the large T antigen. The Neomycin resistance gene allows for selection of mammalian cells that express the protein, and the ampicillin resistance gene and ColE1 origin permits selection and maintenance of the plasmid in *E. coli.* Additional constructs with an amino-terminal GFP fusion are made in pcDNA3.1/NT-GFP-TOPO spanning the entire length of a 238P1B2 protein.

**PAPtag:** A 238P1B2 ORF, or portions thereof, is cloned into pAPtag-5 (GenHunter Corp. Nashville, TN). This construct generates an alkaline phosphatase fusion at the carboxyl-terminus of a 238P1B2 protein while fusing the IgGk signal sequence to the amino-terminus. Constructs are also generated in which alkaline phosphatase with an amino-terminal IgGκ signal sequence is fused to the amino-terminus of a 238P1B2 protein. The resulting recombinant 238P1B2 proteins are optimized for secretion into the media of transfected mammalian cells and can be used to identify proteins such as ligands or receptors that interact with 238P1B2 proteins. Protein expression is driven from the CMV promoter and the recombinant proteins also contain myc and 6X His epitopes fused at the carboxyl-terminus that facilitates detection and purification. The Zeocin resistance gene present in the vector allows for selection of mammalian cells expressing the recombinant protein and the ampicillin resistance gene permits selection of the plasmid in *E*. *coli.*

**ptag5: A** 238P1B2 ORF, or portions thereof, is cloned into pTag-5. This vector is similar to pAPtag but without the alkaline phosphatase fusion. This construct generates 238P1B2 protein with an amino-terminal IgGκ signal sequence and myc and 6X His epitope tags at the carboxyl-terminus that facilitate detection and affinity purification. The resulting recombinant 238P1B2 protein is optimized for secretion into the media of transfected mammalian cells, and is used as immunogen or ligand to identify proteins such as ligands or receptors that interact with the 238P1B2 proteins. Protein expression is driven from the CMV promoter. The Zeocin resistance gene present in the vector allows for selection of mammalian cells expressing the protein, and the ampicillin resistance gene permits selection of the plasmid in *E. coli.*

**PsecFc:** A 238P1B2 ORF, or portions thereof, is also cloned into psecFc. The psecFc vector was assembled by cloning the human immunoglobulin G1 (IgG) Fc (hinge, CH2, CH3 regions) into pSecTag2 (Invitrogen, California). This construct generates an IgG1 Fc fusion at the carboxyl-tenminus of the 238P1B2 proteins, while fusing the IgGK signal sequence to N-terminus. 238P1B2 fusions utilizing the murine IgG1 Fc region are also used. The resulting recombinant 238P1B2 proteins are optimized for secretion into the media of transfected mammalian cells, and can be used as immunogens or to identify proteins such as ligands or receptors that interact with 238P1B2 protein. Protein expression is driven from the CMV promoter. The hygromycin resistance gene present in the vector allows for selection of mammalian cells that express the recombinant protein, and the ampicillin resistance gene permits selection of the plasmid in *E*. *coli.*

**pSRα Constructs:** To generate mammalian cell lines that express 238P1B2 constitutively, 238P1B2 ORF, or portions thereof, of 238P1B2 are cloned into pSRa constructs. Amphotropic and ecotropic retroviruses are generated by transfection of pSRα constructs into the 293T-10A1 packaging line or co-transfection of pSRa and a helper plasmid (containing deleted packaging sequences) into the 293 cells, respectively. The retrovirus is used to infect a variety of mammalian cell lines, resulting in the integration of the cloned gene, 238P1B2, into the host cell-lines. Protein expression is driven from a long terminal repeat (LTR). The Neomycin resistance gene present in the vector allows for selection of mammalian cells that express the protein, and the ampicillin resistance gene and ColE1 origin permit selection and maintenance of the plasmid in *E. coli.* The retroviral vectors can thereafter be used for infection and generation of various cell lines using, for example, PC3, NIH 3T3, TsuPr1, 293 or rat-1 cells.

Additional pSRα constructs are made that fuse an epitope tag such as the FLAG^{™} tag to the carboxyl-terminus of 238P1B2 sequences to allow detection using anti-Flag antibodies. For example, the FLAG^{™} sequence 5' gat tac aag gat gac gac gat aag 3' is added to cloning primer at the 3' end of the ORF. Additional pSRα constructs are made to produce both amino-terminal and carboxyl-terminal GFP and myc/6X His fusion proteins of the full-length 238P1B2 proteins.

**Additional Viral Vectors:** Additional constructs are made for viral-mediated delivery and expression of 238P1B2. High virus titer leading to high level expression of 238P1B2 is achieved in viral delivery systems such as adenoviral vectors and herpes amplicon vectors. A 238P1B2 coding sequence or fragments thereof is amplified by PCR and subcloned into the AdEasy shuttle vector (Stratagene). Recombination and virus packaging are performed according to the manufacturer's instructions to generate adenoviral vectors. Alternatively, 238P1B2 coding sequences or fragments thereof are cloned into the HSV-1 vector (Imgenex) to generate herpes viral vectors. The viral vectors are thereafter used for infection of various cell lines such as PC3, NIH 3T3, 293 or rat-1 cells.

**Regulated Expression Systems:** To control expression of 238P1B2 in mammalian cells, coding sequences of 238P1B2, or portions thereof, are cloned into regulated mammalian expression systems such as the T-Rex System (Invitrogen), the GeneSwitch System (Invitrogen) and the tightly-regulated Ecdysone System (Sratagene). These systems allow the study of the temporal and concentration dependent effects of recombinant 238P1B2. These vectors are thereafter used to control expression of 238P1B2 in various cell lines such as PC3, NIH 3T3, 293 or rat-1 cells.

### B. Baculovirus Expression Systems

To generate recombinant 238P1B2 proteins in a baculovirus expression system, 238P1B2 ORF, or portions thereof, are cloned into the baculovirus transfer vector pBlueBac 4.5 (Invitrogen), which provides a His-tag at the N-terminus. Specifically, pBlueBac-238P1B2 is co-transfected with helper plasmid pBac-N-Blue (Invitrogen) into SF9 (*Spodoptera frugiperda*) insect cells to generate recombinant baculovirus (see Invitrogen instruction manual for details). Baculovirus is then collected from cell supernatant and purified by plaque assay.

Recombinant 238P1B2 protein is then generated by infection of HighFive insect cells (Invitrogen) with purified baculovirus. Recombinant 238P1B2 protein can be detected using anti-238P1B2 or anti-His-tag antibody. 238P1B2 protein can be purified and used in various cell-based assays or as immunogen to generate polyclonal and monoclonal antibodies specific for 238P1B2.

### Example 9 Antigenicity Profiles and Secondary Structure

Figure 5A,B, Figure 6 A,B, Figure 7 A,B, Figure 8 A,B, and Figure 9 A,B depict graphically five amino acid profiles of the 238P1B2 variant 1a (SA-9a) amino acid sequence and variant 1b (5B-9B), each assessment available by accessing the ProtScale website (URL www.expasy.ch/cgi-bin/protscale.pl) on the ExPasy molecular biology server.

These profiles: Figure 5, Hydrophilicity, (Hopp T.P., Woods K.R., 1981. Proc. Natl. Acad. Sci. U.S.A. 78:3824-3828); Figure 6, Hydropathicity, (Kyte J., Doolittle R.F., 1982. J. Mol. Biol. 157:105-132); Figure 7, Percentage Accessible Residues (Janin J., 1979 Nature 277:491-492); Figure 8, Average Flexibility, (Bhaskaran R., and Ponnuswamy P.K., 1988. Int. J. Pept. Protein Res. 32:242-255); Figure 9, Beta-turn (Deleage, G., Roux B. 1987 Protein Engineering 1:289-294); and optionally others available in the art, such as on the ProtScale website, were used to identify antigenic regions of the 238P1B2 protein. Each of the above amino acid profiles of 238P1B2 were generated using the following ProtScale parameters for analysis: 1) A window size of 9; 2) 100% weight of the window edges compared to the window center; and, 3) amino acid profile values normalized to lie between 0 and 1.

Hydrophilicity (Figure 5), Hydropathicity (Figure 6) and Percentage Accessible Residues (Figure 7) profiles were used to determine stretches of hydrophilic amino acids (i.e., values greater than 0.5 on the Hydrophilicity and Percentage Accessible Residues profile, and values less than 0.5 on the Hydropathicity profile). Such regions are likely to be exposed to the aqueous environment, be present on the surface of the protein, and thus be available for immune recognition, such as by antibodies.

Average Flexibility (Figure 8) and Beta-turn (Figure 9) profiles determine stretches of amino acids (i.e., values greater than 0.5 on the Beta-turn profile and the Average Flexibility profile) that are not constrained in secondary structures such as beta sheets and alpha helices. Such regions are also more likely to be exposed on the protein and thus accessible for immune recognition, such as by antibodies.

Antigenic sequences of the 238P1B2 variant la and variant 1b protein indicated, e.g., by the profiles set forth in Figure 5A,B, Figure 6 A,B, Figure 7 A,B, Figure 8 A,B, and/or Figure 9 A,B are used to prepare immunogens, either peptides or nucleic acids that encode them, to generate therapeutic and diagnostic anti-238P1B2 antibodies. The immunogen can be any 5, 6, 7, 8, 9, 10, 11, 12, 13, 14, 15, 16, 17, 18, 19, 20, 21, 22, 23, 24, 25, 30, 35, 40, 45, 50 or more than 50 contiguous amino acids, or the corresponding nucleic acids that encode them, from the 238P1B2 variant proteins. In particular, peptide immunogens for 238P1B2 variant 1a can comprise, a peptide region of at least 5 amino acids of Figure 2 in any whole number increment up to 254 that includes an amino acid position having a value greater than 0.5 in the Hydrophilicity profile of Figure 5A; a peptide region of at least 5 amino acids of Figure 2 in any whole number increment up to 254 that includes an amino acid position having a value less than 0.5 in the Hydropathicity profile of Figure 6A; a peptide region of at least 5 amino acids of Figure 2 in any whole number increment up to 254 that includes an amino acid position having a value greater than 0.5 in the Percent Accessible Residues profile of Figure 7A; a peptide region of at least 5 amino acids of Figure 2 in any whole number increment up to 254 that includes an amino acid position having a value greater than 0.5 in the Average Flexibility profile on Figure 8A; and, a peptide region of at least 5 amino acids of Figure 2 in any whole number increment up to 254 that includes an amino acid position having a value greater than 0.5 in the Beta-turn profile of Figure 9A.

In addition, peptide immunogens for 238P1B2 variant 1b can comprise, a peptide region of at least 5 amino acids of Figure 2 in any whole number increment up to 316 that includes an amino acid position having a value greater than 0.5 in the Hydrophilicity profile of Figure 5B; a peptide region of at least 5 amino acids of Figure 2 in any whole number increment up to 316 that includes an amino acid position having a value less than 0.5 in the Hydropathicity profile of Figure 6B; a peptide region of at least 5 amino acids of Figure 2 in any whole number increment up to 316 that includes an amino acid position having a value greater than 0.5 in the Percent Accessible Residues profile of Figure 7B; a peptide region of at least 5 amino acids of Figure 2 in any whole number increment up to 316 that includes an amino acid position having a value greater than 0.5 in the Average Flexibility profile on Figure 8B; and, a peptide region of at least 5 amino acids of Figure 2 in any whole number increment up to 316 that includes an amino acid position having a value greater than 0.5 in the Beta-turn profile of Figure 9B. Immunogens can also comprise nucleic acids that encode any of the foregoing peptides.

All immunogens described herein, wether peptides or nucleic acids, can be embodied in human unit dose form, or comprised by a composition that includes a pharmaceutical excipient compatible with human physiology.

The secondary structure of 238P1B2, namely the predicted presence and location of alpha helices, extended strands, and random coils, is predicted from the primary amino acid sequence using the HNN - Hierarchical Neural Network method (Guermeur, 1997, World Wide Web URL pbil.ibcp.fr/cgi-bin/npsa automat.p1?page npsa nn.html), accessed from the ExPasy molecular biology server (World Wide Web URL www.expasy.ch/tools/). The analysis indicates that 238P1B2 variant 1a is composed of 66.54% alpha helix, 6.69% extended strand, and 26.77% random coil (Figure 12A). The analysis indicates that 238P1B2 variant 1b is composed of 61.71% alpha helix, 8.86% extended strand, and 29.43% random coil (Figure 12B).

Analysis for the potential presence of transmembrane domains in 238P1B2 was carried out using a variety of transmembrane prediction algorithms accessed from the ExPasy molecular biology server (World Wide Web URL www.expasy.ch/tools/). The programs predict the presence of multiple transmembrane domains in 238P1B2 variant 1a and variant 1b. The highest probability of topology for variant 1a is that of a cell surface protein with 6 transmembrane domains. The highest probability of topology for variant 1b is that of a cell surface protein with 7 transmembrane domains, consistent with that of a G-protein coupled receptor. Shown graphically in Figure 12C and Figure 12D are the results of analysis of 238P1B2 variant 1a using the TMpred (Figure 12C) and TMHMM (Figure 12D) prediction programs depicting the location and topology of the 6 transmembrane domains. Shown in Figure 12E and Figure 12F are the results of the prediction programs for 238P1B2 variant 1b showing the location and topology of the 7 transmembrane domains. The results of each program, namely the amino acids encoding the transmembrane domain, are summarized in Table XXII.

### Example 10: Homology Comparison of 238P1B2 to Known Sequences

The 238P1B2 gene is homologous to a cloned and sequenced gene, namely the mouse olfactory receptor MOR14-1 (gi 18479244) (Zhang X, Firestein S. Nat Neurosci. 2002, 5:124), showing 83% identity and 90% homology to that gene product (Figure 4A; Figure 4C). The 238P1B2 protein shows 78% identity and 87% homology to another mouse olfactory receptor, namely MOR14-10 (gi 18480766). The closest human homolog to 238P1B2 is the human olfactory receptor 5BETA12 (51I2, gi 17456801), with 61% identity and 79% homology (Figure 4E). Comparison of 238P1B2 to another member of the human olfactory receptor family, namely 101P3A11 (reference AGS patent), the 238P1B2 protein shows 48% identity and 70% homology to 101P3A11, with the first amino acid of 238P1B2 aligning with aa 62 of 101P3A11. The 238P1B2 variant 1A protein consists of 254 amino acids, with calculated molecular weight of 28.5kDa, and pI of 9.2. 238P1B2 is a cell surface protein with some localization to the mitochondria and endoplasmic reticulum. Three forms of the 238P1B2 protein have been identified, with variant 238P1B2 V2 containing a isoleucine to threonine point mutation at amino acid 225, and variant 238P1B2 V1B containing an additional 62 aa at its amino-terminus (Figure 4F). While 238P1B2 V1A is projected to have 6 transmembrane domains, 238P1B2 V1B contains 7 transmembrane domains, with the N-terminus oriented extracellularly and the C-terminus being intracellular (Table XXII, Figure 13).

Motif analysis revealed the presence of several known motifs, including a 7 transmembrane olfactory receptor GPCR motif and a fibronectin Type III repeat Proteins that are members of the G-protein coupled receptor family exhibit an extracellular amino-terminus, three extracellular loops, three intracellular loops and an intracellular carboxyl terminus. G-protein coupled receptors are seven-transmembrane receptors that are stimulated by polypeptide hormones, neurotransmitters, chemokines and phospholipids (Civelli O et al, Trends Neurosci. 2001, 24:230; Vrecl M et al., Mol Endocrinol. 1998, 12:1818). Ligand binding traditionally occurs between the first and second extracellular loops of the GPCR. Upon ligand binding GPCRs transduce signals across the cell surface membrane by associating with trimeric G proteins. Their signals are transmitted via trimeric guanine-nucleotide binding proteins (G proteins) to cell surface receptor, effector enzymes or ion channels (Simon et al., 1991, Science 252: 802). Signal transduction and biological output mediated by GPCR can be modulated through various mechanisms including peptide mimics, small molecule inhibitors and GPCR kinases or GRK (Pitcher JA et al, J Biol Chem. 1999, 3;274:34531; Fawzi AB, et al. 2001, Mol. Pharmacol., 59:30).

Recently, GPCRs have also been shown to link to mitogenic signaling pathways of tyrosine kinases (Luttrell et al., 1999, Science 283: 655; Luttrell et al., 1999 Curr Opin Cell Biol 11: 177). GPCRs are regulated by phosphorylation mediated by GPCR kinases (GRKs), which themselves are indirectly activated by the GPCRs (Pitcher et al., 1998, Ann. Rev. Biochem. 67: 653). Olfactory GPCRs transmit their signals by activating the cAMP pathway via adenylate cyclase resulting in downstream signaling to protein kinase A, and by activating the phospholipase C pathway by generating inositol 1,4,5-trisphosphate (IP3) and diacyl-glycerol (DAG) (Breer, 1993, Ciba Found Symp 179: 97; Bruch, 1996, Comp Biochem Physiol B Biochem Mol Biol 113:451). IP3 results in an increase in intracellular calcium, while DAG activates protein kinase C.

Recent studies have associated GPCRs with cellular transformation. In particular, KSHV G protein-coupled receptor was found to transform NIH 3T3 cells *in vitro* and induces multifocal KS-like lesions in KSHV-GPCR-transgenic mice (Schwarz M, Murphy PM. J Immunol 2001, 167:505). KSHV-GPCR was capable of producing its effect on endothelial cells and fibroblasts by activating defined signaling pathways, including the AKT survival pathway (Montaner S et al, Cancer Res 2001, 61:2641). In addition, KSHV-GPCR induced the activation of mitogenic pathways such as AP-1 and NFkB, resulting in the expression of pro-inflammatory genes (Schwarz M, Murphy PM. J Immunol 2001, 167:505). Other GPCRs associated with tumor formation include G2A, and PAR-1, which has been found to induce transformation of NIH 3T3 cells (Whitehead IP et al, Oncogene 2001, 20:1547).

Fibronectin repeat regions are motifs that mediate binding to a variety of substances such as heparin, collagen, fibrin and fibronectin receptors on cell surfaces. Due to their binding capacity fibronectins are involved in cell adhesion, cell differentiation, spreading, migration, and tumor metastasis (Nykvist P et al, J Biol Chem 2001, 276:38673; Danen EH, Yamada KM. J Cell Physiol 2001, 189:1; Nabeshima K et al, Histol Histopathol 1999, 14:1183). In addition, fibronectin enhances angiogenesis and *de novo* blood vessel formation by regulating the migration of endothelial cells, which constitute essential components of blood vessels (Urbich C et al, Arterioscler Thromb Vasc Biol 2002, 22:69), thereby enhancing tumor growth and survival.

This information indicates that 238P1B2 plays a role in the transformation of mammalian cells, induces mitogenic responses including activation of various signaling pathways, and regulates gene transcription by transmitting cell surface signals to the nucleus. Accordingly, when 238P1B2 functions as a regulator of cell transformation, tumor formation, or as a modulator of transcription involved in activating genes associated with inflammation, tumorigenesis or proliferation, 238P1B2 is used for therapeutic, diagnostic, prognostic and/or preventative purposes. In addition, when a molecule, such as a variant or polymorphism of 238P1B2 is expressed in cancerous tissues, it is used for therapeutic, diagnostic, prognostic and/or preventative purposes.

### Example 11: Identification and Confirmation of Potential Signal Transduction Pathways

Many mammalian proteins have been reported to interact with signaling molecules and to participate in regulating signaling pathways. (J Neurochem 2001; 76:217-223). In particular, GPCRs have been reported to activate MAK cascades as well as G proteins, and have been associated with the EGFR pathway in epithelial cells (Naor, Z., et al, Trends Endocrinol Metab. 2000, 11:91; Vacca F et al, Cancer Res. 2000, 60:5310; Della Rocca GJ et al, J Biol Chem. 1999,274:13978). Using immunoprecipitation and Western blotting techniques, proteins are identified that associate with 238P1B2 and mediate signaling events. Several pathways known to play a role in cancer biology can be regulated by 238P1B2, including phospholipid pathways such as PI3K, AKT, etc.; adhesion and migration pathways, including FAK, Rho, Rac-1, etc.; and mitogenic/survival cascades such as ERK, p38, etc. (Cell Growth Differ. 2000,11:279; J Biol Chem 1999, 274:801; Oncogene. 2000, 19:3003, J. Cell Biol. 1997, 138:913).

Several GPCRs have been shown to transactivate receptor tyrosine kinases associated with the cell membrane, such as the EGF receptor (EGFR) (Pierce KL et al, J Biol Chem. 2001, 276:23155; Nath D et al, J Cell Sci. 2001, 114:1213). In order to determine whether 238P1B2 signaling results in the activation of EGFR, cells are grown in media alone or in the presence of the EGFR inhibitor AG1517. EGFR phosphorylation is compared in control and treated cells. Similarly, cross talk between 238P1B2 and EGFR pathways is investigated.

To confirm that 238P1B2 directly or indirectly activates known signal transduction pathways in cells, luciferase (luc) based transcriptional reporter assays are carried out in cells expressing individual genes. These transcriptional reporters contain consensus-binding sites for known transcription factors that lie downstream of well-characterized signal transduction pathways. The reporters and examples of these associated transcription factors, signal transduction pathways, and activation stimuli are listed below.
1. NFkB-luc, NFkB/Rel; Ik-kinase/SAPK; growth/apoptosis/stress
2. SRE-luc, SRF/TCF/ELK1; MAPK/SAPK; growth/differentiation
3. AP-1-luc, FOS/JUN; MAPK/SAPK/PKC; growth/apoptosis/stress
4. ARE-luc, androgen receptor; steroids/MAPK; growth/differentiation/apoptosis
5. p53-luc, p53; SAPK; growth/differentiation/apoptosis
6. CRE-luc, CREB/ATF2; PKA/p38; growth/apoptosis/stress

Gene-mediated effects can be assayed in cells showing mRNA expression. Luciferase reporter plasmids can be introduced by lipid-mediated transfection (TFX-50, Promega). Luciferase activity, an indicator of relative transcriptional activity, is measured by incubation of cell extracts with luciferin substrate and luminescence of the reaction is monitored in a luminometer.

Signaling pathways activated by 238P1B2 are mapped and used for the identification and validation of therapeutic targets. When 238P1B2 is involved in cell signaling, it is used as target for diagnostic, prognostic, preventative and/or therapeutic purposes.

### Example 12: 238P1B2 Functions as a GPCR

Sequence and homology analysis of 238P1B2 indicate that the 238P1B2 protein is a member of the olfactory receptor family of GPCR. Olfactory receptors are known to regulate biological responses by activating adenylate cyclase. In order to confirm that 238P1B2 functions as a GPCR and mediates the activation of adenylate cyclase, cAMP accumulation in PC3 and PC3-238P1B2 cells are compared in cells grown in the presence or absence of fetal bovine serum (FBS). The cells are lysed and intracellular concentration of cAMP are measured using a commercially available enzyme immunoassay (EIA). Calculations of cAMP concentrations were based on OD450 of the standard curve. Similarly, the same assay can be used to determine whether the induction of cAMP accumulation by 238P1B2 is inhibited by GPCR inhibitors such as pertussis toxin.

GPCR transmit their signal by activating trimeric G proteins. Once GPCRs are activated, the associated Ga subunit binds GTP, dissociates from the receptor and participates in downstream signaling events (Schild D and Restrepo D. Physiol Rev. 1998, 78:429-66). In order to determine that inhibition of Ga subunits has an effect on 238P1B2 mediated cell growth, the effect of Ga inhibitors on the proliferation of 3T3-238P1B2 and PC3-238P1B2 cells is investigated. Control and 238P1B2-expressing cells are grown in the presence or absence of suramin or its derivative NF 449 (Sigma). Cells are analyzed for proliferation using an MTT-like assay.

When 238P1B2 functions as a GPCR, it is used as target for diagnostic, prognostic, preventative and/or therapeutic purposes.

### Example 13: Involvement in Tumor Progression

The 238P1B2 gene can contribute to the growth of cancer cells. The role of 238P1B2 in tumor growth is confirmed in a variety of primary and transfected cell lines including prostate as well as NIH 3T3 cells engineered to stably express 238P1B2. Parental cells lacking 238P1B2 and cells expressing 238P1B2 are evaluated for cell growth using a well-documented proliferation assay (Fraser SP, Grimes JA, Djamgoz MB. Prostate. 2000;44:61, Johnson DE, Ochieng J, Evans SL. Anticancer Drugs. 1996, 7:288). To confirm that 238P1B2 mediates enhanced proliferation by way of its GPCR activity, control cells and cells expressing 238P1B2 are grown in the presence or absence of pertussis toxin, and evaluated for their proliferative capability using the same assay described above.

To confirm the role of 238P1B2 in the transformation process, its effect in colony forming assays is investigated Parental NIH-3T3 cells lacking 238P1B2 are compared to NIH-3T3 cells expressing 238P1B2, using a soft agar assay under stringent and more permissive conditions (Song Z. et al., Cancer Res. (2000) 60:6730).

To confirm the role of 238P1B2 in invasion and metastasis of cancer cells, a well-established assay is used, e.g., a Transwell Insert System assay (Becton Dickinson) (Cancer Res. 1999; 59:6010). Control cells, including prostate and fibroblast cell lines lacking 238P1B2 are compared to cells expressing 238P1B2. Cells are loaded with the fluorescent dye, calcein, and plated in the top well of the Transwell insert coated with a basement membrane analog. Invasion is determined by fluorescence of cells in the lower chamber relative to the fluorescence of the entire cell population.

238P1B2 can also play a role in cell cycle and apoptosis. Parental cells and cells expressing 238P1B2 are compared for differences in cell cycle regulation using a well-established BrdU assay (Abdel-Malek ZA. J Cell Physiol. 1988, 136:247). In short, cells grown under both optimal (full serum) and limiting (low serum) conditions are labeled with BrdU and stained with anti-BrdU Ab and propidium iodide. Cells are analyzed for entry into the G1, S, and G2M phases of the cell cycle. Alternatively, the effect of stress on apoptosis is evaluated in control parental cells and cells expressing 238P1B2, including normal and tumor prostate cells. Engineered and parental cells are treated with various chemotherapeutic agents, such as etoposide, flutamide, etc, and protein synthesis inhibitors, such as cycloheximide. Cells are stained with annexin V-FITC and cell death is measured by FACS analysis. The modulation of cell death by 238P1B2 can play a critical role in regulating tumor progression and tumor load.

When 238P1B2 plays a role in cell growth, transformation, invasion or apoptosis, it is used as a target for diagnostic, prognostic, preventative and/or therapeutic purposes.

### Example 14: Involvement in Angiogenesis

Angiogenesis or new capillary blood vessel formation is necessary for tumor growth (Hanahan D, Folkman J. Cell. 1996, 86:353; Folkman J. Endocrinology. 1998 139:441). Based on the effect of phsophodiesterase inhibitors on endothelial cells, 238P1B2 plays a role in angiogenesis (DeFouw L et al, Microvasc Res 2001, 62:263). Several assays have been developed to measure angiogenesis *in vitro* and in *vivo,* such as the tissue culture assays of endothelial cell tube formation and endothelial cell proliferation. Using these assays as well as *in vitro* neo-vascularization, the role of 238P1B2 in angiogenesis, enhancement or inhibition, is confirmed.

For example, endothelial cells engineered to express 238P1B2 are evaluated using tube formation and proliferation assays. The effect of 238P1B2 is also confirmed in animal models *in vivo*. For example, cells either expressing or lacking 238P1B2 are implanted subcutaneously in immunocompromised mice. Endothelial cell migration and angiogenesis are evaluated 5-15 days later using immunohistochemistry techniques: 238P1B2 affects angiogenesis, and it is used as a target for diagnostic, prognostic, preventative and/or therapeutic purposes

### Example 15: Regulation of Transcription

The cell surface localization of 238P1B2 and its similarity to GPCRs indicate that 238P1B2 is effectively used as a modulator of the transcriptional regulation of eukaryotic genes. Regulation of gene expression is confirmed, e.g., by studying gene expression in cells expressing or lacking 238P1B2. For this purpose, two types of experiments are performed.

In the first set of experiments, RNA from parental and 238P 1 B2-expressing cells are extracted and hybridized to commercially available gene arrays (Clontech) (Smid-Koopman E et al. Br J Cancer. 2000. 83:246). Resting cells as well as cells treated with FBS or androgen are compared. Differentially expressed genes are identified in accordance with procedures known in the art. The differentially expressed genes are then mapped to biological pathways (Chen K et al. Thyroid. 2001. 11:41.).

In the second set of experiments, specific transcriptional pathway activation is evaluated using commercially available (Stratagene) luciferase reporter constructs including: NTkB-luc, SRE-luc, ELK1-luc, ARE-luc, p53-luc, and CRE-luc. These transcriptional reporters contain consensus binding sites for known transcription factors that lie downstream of well-characterized signal transduction pathways, and represent a good tool to ascertain pathway activation and screen for positive and negative modulators of pathway activation.

Thus, 238P1B2 plays a role in gene regulation, and it is used as a target for diagnostic, prognostic, preventative and/or therapeutic purposes.

### Example 16 Involvement in Cell Adhesion

Cell adhesion plays a critical role in tissue colonization and metastasis. Based on the presence of a fibronectin repeat in its C-terminus, 238P1B2 can participate in cellular organization, and as a consequence affects cell adhesion and motility. To confirm that 238P1B2 regulates cell adhesion, control cells lacking 238P1B2 are compared to cells expressing 238P1B2, using techniques previously described (see, e.g., Haier et al, Br. J. Cancer. 1999, 80:1867; Lehr and Pienta, J. Natl. Cancer Inst. 1998, 90:118). Briefly, cells labeled with a fluorescent indicator, such as calcein, are incubated on tissue culture wells coated with media alone or with matrix proteins. Adherent cells are detected by fluorimetric analysis and percent adhesion is calculated. Alternatively, cells lacking or expressing 238P1B2 are analyzed for their ability to mediate cell-cell adhesion using similar experimental techniques as described above. Both of these experimental systems are used to identify proteins, antibodies and/or small molecules that modulate cell adhesion to extracellular matrix and cell-cell interaction. Cell adhesion plays a critical role in tumor growth, progression, and colonization, and 238P1B2 is involved in these processes. Thus, it serves as a diagnostic, prognostic, preventative and/or therapeutic modality.

### Example 17: Protein-Protein Association

Several GPCRs have been shown to interact with other proteins, thereby regulating signal transduction, gene transcription, transformation and cell adhesion (Sexton PM et al, Cell Signal. 2001, 13:73; Turner CE, J Cell Sci. 2000, 23:4139). Using immunoprecipitation techniques as well as two yeast hybrid systems, proteins are identified that associate with 238P1B2. Immunoprecipitates from cells expressing 238P1B2 and cells lacking 238P1B2 are compared for specific protein-protein associations.

Studies are performed to confirm the extent of association of 238P 1B2 with effector molecules, such as nuclear proteins, transcription factors, kinases, phsophates etc. Studies comparing 238P1B2 positive and 238P1B2 negative cells as well as studies comparing unstimulated/resting cells and cells treated with epithelial cell activators, such as cytokines, growth factors, androgen and anti-integrin Ab reveal unique interactions.

In addition, protein-protein interactions are confirmed using two yeast hybrid methodology (Curr Opin Chem Biol. 1999, 3:64). A vector carrying a library of proteins fused to the activation domain of a transcription factor is introduced into yeast expressing a 238P1B2-DNA-binding domain fusion protein and a reporter construct. Protein-protein interaction is detected by colorimetric reporter activity. Specific association with effector molecules and transcription factors directs one of skill to the mode of action of 238P1B2, and thus identifies therapeutic, prognostic, preventative and/or diagnostic targets for cancer. This and similar assays are also used to identify and screen for small molecules that interact with 238P1B2.

Thus it is found that 238P1B2 associates with protein and small molecules. Accordingly, 238P1B2and these proteins and small molecules are used for diagnostic, prognostic, preventative and/or therapeutic purposes.

### TABLES

**TABLE I: Tissues that Express 238P1B2 When Malignant**
Prostate

**TABLE II: AMINO ACID ABBREVIATIONS**

| **SINGLE LETTER** | **THREE LETTER** | **FULL NAME** |
|---|---|---|
| F | Phe | phenylalanine |
| L | Leu | leucine |
| s | Ser | serine |
| Y | Tyr | tyrosine |
| C | Cys | cysteine |
| w | Trp | tryptophan |
| P | Pro | proline |
| H | His | histidine |
| Q | Gln | glutamine |
| R | Arg | arginine |
| I | Ile | isoleucine |
| M | Met | methionine |
| T | Thr | threonine |
| N | Asn | asparagine |
| K | Lys | lysine |
| V | Val | valine |
| A | Ala | alanine |
| D | Asp | aspartic acid |
| E | Glu | glutamic acid |
| G | Gly | glycine |

**TABLE III: AMINO ACID SUBSTITUTION MATRIX**

| Adapted from the GCG Software 9.0 BLOSUM62 amino acid substitution matrix (block substitution matrix). The higher the value, the more likely a substitution is found in related, natural proteins. (See URL www.ikp.unibe.ch/manual/blosum62.html) | | | | | | | | | | | | | | | | | | | | |
|---|---|---|---|---|---|---|---|---|---|---|---|---|---|---|---|---|---|---|---|---|
| A | C | D | E | F | G | H | I | K | L | M | N | P | Q | R | S | T | V | W | Y | . |
| 4 | 0 | -2 | -1 | -2 | 0 | -2 | -1 | -1 | -1 | -1 | -2 | -1 | -1 | -1 | 1 | 0 | 0 | -3 | -2 | A |
| | 9 | -3 | -4 | -2 | -3 | -3 | -1 | -3 | -1 | -1 | -3 | -3 | -3 | -3 | -1 | -1 | -1 | -2 | -2 | C |
| | | 6 | 2 | -3 | -1 | -1 | -3 | -1 | -4 | -3 | 1 | -1 | 0 | -2 | 0 | -1 | -3 | -4 | -3 | D |
| | | | 5 | -3 | -2 | 0 | -3 | 1 | -3 | -2 | 0 | -1 | 2 | 0 | 0 | -1 | -2 | -3 | -2 | E |
| | | | | 6 | -3 | -1 | 0 | -3 | 0 | 0 | -3 | -4 | -3 | -3 | -2 | -2 | -1 | 1 | 3 | F |
| | | | | | 6 | -2 | -4 | -2 | -4 | -3 | 0 | -2 | -2 | -2 | 0 | -2 | -3 | -2 | -3 | G |
| | | | | | | 8 | -3 | -1 | -3 | -2 | 1 | -2 | 0 | 0 | -1 | -2 | -3 | -2 | 2 | H |
| | | | | | | | 4 | -3 | 2 | 1 | -3 | -3 | -3 | -3 | -2 | -1 | 3 | -3 | -1 | I |
| | | | | | | | | 5 | -2 | -1 | 0 | -1 | 1 | 2 | 0 | -1 | -2 | -3 | -2 | K |
| | | | | | | | | | 4 | 2 | -3 | -3 | -2 | -2 | -2 | -1 | 1 | -2 | -1 | L |
| | | | | | | | | | | 5 | -2 | -2 | 0 | -1 | -1 | -1 | 1 | -1 | -1 | M |
| | | | | | | | | | | | 6 | -2 | 0 | 0 | 1 | 0 | -3 | -4 | -2 | N |
| | | | | | | | | | | | | 7 | -1 | -2 | -1 | -1 | -2 | -4 | -3 | P |
| | | | | | | | | | | | | | 5 | 1 | 0 | -1 | -2 | -2 | -1 | Q |
| | | | | | | | | | | | | | | 5 | -1 | -1 | -3 | -3 | -2 | R |
| | | | | | | | | | | | | | | | 4 | 1 | -2 | -3 | -2 | S |
| | | | | | | | | | | | | | | | | 5 | 0 | -2 | -2 | T |
| | | | | | | | | | | | | | | | | | 4 | -3 | -1 | V |
| | | | | | | | | | | | | | | | | | | 11 | 2 | W |
| | | | | | | | | | | | | | | | | | | | 7 | Y |

**Table XXI: Motifs and Post-translational Modifications of 238P1B2**

| |
|---|
| cAMP- and cGMP-dependent protein kinase phosphorylation site. 176 - 179 RKeT |
| Protein kinase C phosphorylation site. 235 - 237 SvK |
| Casein kinase II phosphorylation site. 9 - 12 SatD 50 - 53 TvmE 130 - 133 SctD 172 - 175 SpeE |
| N-myristoylation site. 14 - 19 GLsiST |
| G-protein coupled receptors family 1 signature. 52 - 68 MESsvLIaMAFDRFvaV |
| G-protein coupled receptors family 1. 1-234 |

**TABLE XXII: Physical Features of 238P1B2**

| 238P1B2 Variant 1A | Bioinformatic Program | URL | Outcome |
|---|---|---|---|
| ORF | ORF finder | | 3758 bp |
| Protein length | | | 254 aa |
| Transmembrane region | TM Pred | World Wide Web URL www.ch.embnet.org/ | N terminus intracellular, 6 TM TM helices at 3-29, 44-62, 86-110, 144-161, 182-203, 217-236aa |
| | HMMTop | World Wide Web URL www.enzim.hu/hmmtop/ | N terminus intracellular, 6TM TM helices at 6-28, 43-62, 86-105, 136-158, 180-203, 216-235aa |
| | Sosui | World Wide Web URL www.genome.ad.jp/SOSui/ | Membrane protein, 6TM TM helices at 6-28, 42-64, 87-109, 144-166, 187-209, 217-237aa |
| | TMHMM | World Wide Web URL www.cbs.dtu.dk/services/TMH MM | N terminus intracellular, 6TM TM helices at 7-29, 44-66, 86-108, 142-164, 185-207, 212-234aa |
| Signal Peptide | Signal P | World Wide Web URL www.cbs.dtu.dk/services/Signal P/ | cleavage site between 169-170aa |
| pI | pI/MW tool | World Wide Web URL www.expasy.ch/tools/ | pI 9.24 |
| Molecular weight | pI/MW tool | World Wide Web URL www.expasy.ch/tools/ | 28.59 kDa |
| Localization | PSORT | World Wide Web URL psort.nibb.ac.jp/ | 60% plasma membrane, 42.9% mitochondrial inner membrane, 40% Golgi, 30% endoplasmic reticulum membrane |
| | PSORT II | World Wide Web URL psort.nibb.ac.jp/ | 44.4% endoplasmic reticulum, 22.2% plasma membrane |
| Motifs | Pfam | World Wide Web URL www.sanger.ac.uk/Pfam/ | 7TM receptor (rhodopsin family) |
| | Prints | World Wide Web URL www.biochemucl.ac.uk/ | Olfactory receptor signature, Type III secretion system inner membrane R protein, fibronectin Type III repeat signature |
| | Blocks | World Wide Web URL www.blocks.thcrc.org/ | no significant motif |
| | | | |
| 238P1B2 Variant 1B | Bioinformatic Program | URL | Outcome |
| ORF | ORF finder | | 3758 bp |
| Protein length | | | 316 aa |
| Transmembrane | TM Pred | World Wide Web URL | N terminus extracellular, 8 |
| region | | www.ch.embnet.org/ | TM TM helices at 9-27,33-51, 75-91, 98-125, 145-171, 206-226, 249-265, 276-296aa |
| | HMMTop | World Wide Web URL www.enzim.hu/hmmtop/ | N terminus extracellular, 7TM TM helices at 29-53, 66-90, 105-124, 148-171, 202-226, 241-265, 278-297aa |
| | Sosui | World Wide Web URL www.genome.ad.jp/SOSui/ | Membrane protein, 8TM TM helices at 2-24, 33-55, 67-89, 104-126, 149-171, 206-228,245-267,279-299aa |
| | TMHMM | World Wide Web URL www.cbs.dtu.dk/services/TMH MM | N terminus intracellular, 7TM TM helices at 30-52, 65-87, 102-124,145-167,204-226, 247-269, 274-296aa |
| Signal Peptide | Signal P | World Wide Web URL www.cbs.dtu.dk/services/Signal P/ | cleavage site between aa 26 and 27 |
| pI | pI/MW tool | World Wide Web URL www.expasy.ch/tools/ | pI 9.03 |
| Molecular weight | pI/MW tool | World Wide Web URL www.expasy.ch/tools/ | 35.34 kDa |
| Localization | PSORT | World Wide Web URL psort.nibb.ac.jp/ | 64% plasma membrane, 46% Golgi, 37% endoplasmic reticulum membrane |
| | PSORT II | World Wide Web URL psort.nibb.ac.jp/ | 44.4% endoplasmic reticulmn, 33.3% plasma membrane |
| Motifs | Pfam | World Wide Web URL www.sanger.ac.uk/Pfam/ | 7TM receptor (rhodopsin family), polysaccharide biosynthesis protein |
| | Prints | World Wide Web URL www.biochem.ucl.ac.uk/ | Olfactory receptor signature, Type III secretion system inner membrane R protein, fibronectin Type III repeat signature |
| | Blocks | World Wide Web URL www.blocks.fhcrc.org/ | no significant motif |

**Table XXIII. Exon compositions of 238P1B2 v.1**

| Exon Number | Start | End |
|---|---|---|
| Exon 1 | 1 | 3758 |

## Claims

1. A method for detecting the presence of prostate cancer in a test sample, the method comprising:
contacting the sample with a probe that specifically binds to a polynucleotide of Figure 2B ; and
detecting binding of the polynucleotide in the sample thereto.

## Patentansprüche

1. Verfahren zum Nachweisen der Anweseneheit von Prostatakrebs in einer Testprobe, das Verfahren umfassend:
Kontaktieren der Probe mit einer Sonde, die spezifisch ein Polynukleotid der Abbildung 2B bindet, und
Nachweisen der Bindung des Polynukleotids in der Probe daran.

## Revendications

1. Procédé pour détecter la présence d'un cancer de la prostate dans un échantillon de test, le procédé comprenant:
la mise en contact de l'échantillon avec une sonde qui se lie spécifiquement à un polynucléotide de la figure 2B, et
la détection de la liaison du polynucléotide dans l'échantillon à celle-ci.
